# EUROPEAN PATENT APPLICATION

(11) **EP 1 724 264 A1**
(43) Date of publication of application: **22.11.2006**
(21) Application number: 05720835.7
(22) Date of filing: 09.03.2005
(51) Int. Cl.: C07D 239/42

(54) **NITRILES AND MEDICINAL COMPOSITIONS CONTAINING THE SAME AS THE ACTIVE INGREDIENT**

(30) Priority: 10.03.2004 JP 2004068212
(71) Applicant: ONO PHARMACEUTICAL CO., LTD., Osaka-shi, Osaka 541-8526 (JP)
(72) Inventor: OHMOTO, Kazuyuki, c/o Ono Pharmaceutical Co., Ltd., Mishima-gun, Osaka 618-8585 (JP); HISAICHI, Katsuya, c/o Ono Pharmaceutical Co. Ltd., Mishima-gun, Osaka 618-8585 (JP); OKUMA, Motohiro, c/o Ono Pharmaceutical Co., Ltd., Mishima-gun, Osaka 618-8585 (JP); TANAKA, Makoto, c/o Ono Pharmaceutical Co., Ltd., Mishima-gun, Osaka 618-8585 (JP); KAWADA, Naoki, c/o Ono Pharmaceutical Co., Ltd., Mishima-gun, Osaka 618-8585 (JP)
(74) Representative: Henkel, Feiler & Hänzel
(86) International application number: PCT/JP2005/004580
(87) International publication number: WO 2005/085210

(57) **Abstract**

The present invention relates to a compound of formula (I) 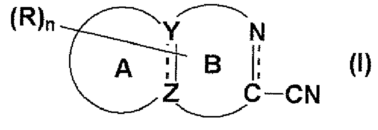 wherein Y and Z each is independently N or C; ring A is a carbocyclic group or a heterocyclic group; ring B is a heterocyclic group containing at least one nitrogen atom; R is a hydrogen atom, a substituent, *etc,*; n is 0, or an integer of from 1 to 10, a salt thereof, a solvate thereof, or an N-oxide thereof, or a prodrug thereof. The compound of formula (I), a salt thereof, a solvate thereof, or an N-oxide thereof, or a prodrug thereof has an inhibitory activity against cysteine protease, and it is useful for the prophylaxis and/or treatment of a cysteine protease-related disease such as osteoporosis, *etc.*

## Description

### TECHNICAL FIELD

The present invention relates to nitrile derivatives which are useful for the treatment of bone diseases such as osteoporosis, a method for the preparation thereof and use thereof

### BACKGROUND OF ART

Cysteine protease is a generic name of proteases which have a cysteine residue in the activity center and catalyze protein degradation thereat. In animal cells, many cysteine proteases are known; for example, cathepsin family, calpain, caspase, *etc*. Cysteine protease exists in various kinds of cells extensively and plays a basic and essential role in the homeostasis, such as conversion of precursor protein into its active form (processing) and degradation of proteins which have become out of use, *etc.* Until now, its physiological effects are being vigorously studied, and as the studies progress and characters of the enzymes are revealed, cysteine proteases came to be taken as a cause of really various kinds of diseases.

It is revealed that cathepsin S (see J. Immunol., 161, 2731 (1998)), cathepsin L (see J. Exp. Med., 183, 1331 (1996)) and cathepsin F (J. Exp. Med., 191, 1177 (2000)) play a role in processing of major histocompatibility complex class-II in antigen presenting cells which play an important role in the early stage of immune responses. In an experimental inflammatory response model induced by antigens, a specific inhibitor of cathepsin S showed an inhibitory effect (see J. Clin. Invest., 101, 2351 (1998)). It is also reported that in a leishmania-infected immune response model a cathepsin B inhibitor controlled an immune response and by means of this effect it inhibited the proliferation of protozoans (see J. Immunol., 161, 2120 (1998)). In vitro, a result is given that a calpain inhibitor and a cysteine protease inhibitor E-64 inhibited apoptosis which is induced by stimuli on T cell receptors (see J. Exp. Med., 178, 1693 (1993)). And cathepsin W, which is expressed in CD8 T cells and NK cells specifically, is known to increase its expression by stimuli of IL-2 by 7 times and so it is conceived that it is concerned with immune responses (see J. Immunol., 167, 2172 (2001)). It is also reported that in leukemia patients, gene expression of cathepsin C and cathepsin W increase and cytotoxic T cells are activated (see Int. J. Oncol., 22, 33 (2003) ). Therefore, it is conceivable that cysteine proteases are much concerned with the progress of immune responses.

It is speculated that caspase or a similar cysteine protease thereto occupies an important position in the mechanism of cell death including apoptosis. Therefore it is expected for a cysteine protease inhibitor to be used as an agent for the prophylaxis and/or treatment of those diseases concerning apoptosis, such as infectious diseases, deterioration or sthenia of immune function and brain function, or tumors *etc.* Diseases concerning apoptosis include, acquired immune deficiency syndrome (AIDS), AIDS-related complex (ARC), adult T cell leukemia, hairy cell leukemia, spondylopathy, respiratory apparatus disorder, arthritis, virus-related diseases (HIV, HTLV-1 related diseases (uveitis *etc.*) and hepatitis C *etc.),* cancer, collagenosis (systemic lupus erythematosus, rheumatoid arthritis, *etc.*), autoimmune diseases (inflammatory bowel diseases, Sjoegren syndrome, primary biliary cirrhosis, spontaneous thrombocytopenic purpura, autoimmune hemolytic anemia, myasthenia gravis, insulin dependent (type-I) diabetes, *etc.*), diseases accompanied by thrombocytopenia (osteomyelodysplasia syndrome, periodic thrombocytopenia, aplastic anemia, spontaneous thrombocytopenia, disseminated intravascular coagulation (DIC), *etc*.), hepatic diseases such as viral hepatitis (C, A, B, F, *etc.)* or hepatitis medicamentosa and cirrhosis, dementia (Alzheimer's disease, Alzheimer's senile dementia, *etc*.), cerebrovascular injury, nerve degeneration diseases, adult acute respiratory distress syndrome, infectious diseases, prostate hypertrophy, hysteromyoma, bronchial asthma, arteriosclerosis, all kinds of lusus naturae, nephropathy, senile cataract, chronic fatigue syndrome, myodystrophy, peripheral neuropathy, *etc.*

Moreover, caspase-1 is concerned with various inflammatory diseases and those diseases caused by immune disorders, by means of interleukin-1β (IL-Iβ) production. A lot of diseases are shown to be involved with caspase-1; for example, inflammatory bowel diseases such as ulcerative colitis, inflammatory diseases and autoimmune disease (insulin-dependent (type-I) diabetes, autoimmune thyroid diseases, infectious diseases, rejection of an organ transplant, graft versus host diseases, psoriasis, periodontitis (above, see N. Eng. J. Med., 328, 106 (1993)), pancreatitis (see J. Interferon Cytokine Res., 17, 113 (1997)), hepatitis (see J. Leuko. Biol., 58, 90 (1995)), glomerulonephritis (see Kidney Int., 47, 1303 (1995)), endocarditis (see Infect. Immun., 64, 1638 (1996)), myocarditis (see Br. Hearat J., 72, 561 (1995)), systemic lupus erythematosus (see Br. J. Rheumatol., 34, 107 (1995)), Hashimoto's diseases (see Autoimmunity, 16, 141 (1993)), *etc.*), *etc.* Experimentally, it is reported that in liver injury model induced by lipopolysaccharide and D-galactosamine, a caspase-1 inhibitor improved the symptoms, and it is expected that a caspase inhibitor shows an effect in sepsis, ischemic reperfusion and hepatitis gravis (see Am. J. Respir. Crit. Care Med., 159, 1308 (1999)).

It is also shown that cysteine protease is concerned with rheumatoid arthritis. IL-1β is shown to be concerned with this disease (see Arthritis Rheum., 39, 1092 (1996)), and in addition, as autoantibody toward calpastatin (endogenous calpain inhibitor) was found in the serum of the patients (see Proc. Natl. Acad. Sci. USA, 92, 7267 (1995)), it is thought that increase of calpain activity leads to the cause of diseases. Also, it is reported that cathepsin B and cathepsin C activity is increased in leukocyte of patients suffering from rheumatoid arthritis (see Biol. Chem., 383, 865 (2002)). It is reported that in experimental arthritis model the production of inflammatory cytokine is suppressed and affection of arthritis completely in cathepsin C knock-out mice, so it is expected that cathepsin C inhibition leads to treatment of rheumatoid arthritis (see J. Clin. Invest., 109, 357 (2002)).

It is reported that cathepsin B plays a role in decomposing muscular protein in the chronic phase of sepsis (see J, Clin. Invest., 97, 1610 (1996)), and in decomposing muscular protein in myodystrophy model (see Biochem. J., 288, 643 (1992)). At the same time it is reported that calpain decomposes the myocyte cell proteins of myodystrophy patients (see J. Biol. Chem., 270, 10909 (1995)).

In ischemic reperfusion model, a result is given that calpain causes degeneration of brain tissues by means of degradation of protein kinase C-β (see J. Neurochem., 72, 2556 (1999)) and that a cathepsin B inhibitor inhibits nerve injury (see Eur. J. Neurosci., 10, 1723 (1998)).

In the brain ischemic model, it is known that the degradation of spectrin by calpain causes a damage and its function disorder in the neurocyte (see Brain Res., 790, 1(1998)) and it is reported that an IL-1β receptor antagonist relieved the symptoms (see Brain Res. Bull., 29, 243 (1992)).

In myocardial ischemic model it is confirmed that cathepsin B activity increases in the lesion (see Biochem. Med. Metab. Biol., 45, 6 (1991)).

In the experiment utilizing ischemic liver injury model, it proved that necrosis and apoptosis of hepatocyte were induced by means of protein-decomposing activity of calpain (see Gastroenterology, 116, 168 (1999)).

Otherwise, it is known that calpain causes cornea turbid by means of degradation of crystalline (see Biol. Chem., 268, 137 (1993)) and that in the lesion of contracted gut mucosa model it was confirmed that the activity of cathepsin B, H and L increased (see J. Parenter. Enteral. Nutr., 19, 187 (1995)) and it is shown that cysteine protease is a cause of the diseases resulting from these protein degradation. It has been revealed that cysteine protease is concerned with systemic disorders of organs and tissues by shock.

It is shown that IL-1β is concerned with septic shock and systemic inflammatory response syndrome (see Igakuno ayumi, 169, 850 (1994)) and besides, it is reported that in endotoxin shock model induced by lipopolysaccharide, a calpain inhibitor prevented circulatory system disorder, disorders of liver and pancreas and acidosis by means of inhibitory effect of activation of nuclear factor κB (see Br. J. Pharmacol., 121, 695 (1997)).

Since it is reported that calpain is concerned with platelet coagulation process and a calpain inhibitor prevented platelet coagulation (see Am. J. Physiol., 259, C862 (1990)), it is conceivable that a cysteine protease inhibitor is useful for the disorder of blood coagulation. From the fact that calpain activity increased in the serum of the patients of purpura (thrombocytopenia) resulting from marrow transplantation, so it is conceivable that calpain is concerned with the actual disease symptoms (see Bone Marrow Transplant., 24, 641 (1999)).

Caspase-1 inhibitor suppressed apoptosis of blood vessel endothelial cells, which is seen in the early phase of purpura (thrombocytopenia) and is thought to be important for the progression of the pathology afterwards (see Am. J. Hematol., 59, 279 (1998)), so it is expected that a cysteine protease inhibitor makes effect on purpura and hemolytic uremic syndrome.

The effect of cysteine protease and its inhibitor is being investigated in the area of cancer and metastasis of cancer.

Since the proliferations of pancreas cancer cells (see Cancer Res., 59, 4551 (1999)) and acute myeloid leukemia cells (see Clin. Lab. Haematol., 21, 173 (1999)) were inhibited by a caspase-1 inhibitor or its receptor antagonist, it is expected that caspase-1 activity is essential for the process of proliferation of tumor cells, and that an inhibitor thereof is effective for these cancers. Also, from the facts that cathepsin B activity increased in colon cancer metastasis model (see Clin. Exp. Metastasis, 16, 159 (1998)), that cathepsin L activity increased in urine of bladder cancer patients (see Urology, 59, 308 (2002)), that cathepsin Z expression was recognized in tumor cells (see J. Biol. Chem., 273, 16816 (1998)), that cathepsin K protein expression recognized in human breast cancer cells proved the relationship of cathepsin K and bone metastasis (see Cancer Res., 57, 5386 (1997)), and that a calpain inhibitor suppressed migration of the cells, which implies that calpain inhibition might be able to inhibit metastasis of cancer (see J. Biochem., 272, 32719 (1997)), a cysteine protease inhibitor is expected to exhibit an inhibitory effect on the metastasis of various malignant tumors.

As to AIDS (see AIDS, 10, 1349 (1996)) and AIDS-related complex (ARC) (see Arch. Immunol. Ther. Exp. (Warsz), 41, 147 (1993)), it is implied that IL-1 is concerned with the progress of symptoms, and so it is conceivable that cysteine protease inhibition leads to an effective therapy of AIDS and its complication.

Some parasites have cysteine protease activity in their bodies. Cysteine protease in the phagosome of malaria protozoan is an essential enzyme for supplying nutrition of the parasites. Its inhibitor show an inhibitory effect of the proliferation of the protozoan (see Blood, 87, 4448 (1996)). Cystein protease inhibitor is thought of as drug for treatment of malaria.

In Alzheimer-type dementia, it is said that adhesion of non-physiological protein called amyloid to brain is deeply involved with nervous function disorders. Cysteine protease has an activity of generating amyloid by decomposing its precursor protein. Clinically, it is shown that cathepsin B possesses a processing activity of amyloid proteins in the brains of Alzheimer-type dementia patients (see Biochem. Biophys. Res. Commun., 177, 377 (1991)). And expressions of cathepsin B protein (see Virchows Arch. A. Pathol. Anat. Histpathol., 423, 185 (1993)), cathepsin S protein (see Am. J. Pathol., 146, 848 (1995)) and calpain protein (see Proc. Natl. Acad. Sci. USA, 90, 2628 (1993)) and increase of caspase-1 activity (see J. Neuropathol. Exp. Neurol., 58, 582 (1999)) were confirmed in the brain lesions. And it is implied that cysteine protease is concerned with the disease symptoms, by the fact that calpain is concerned with the formation of paired helical filaments which accumulate in Alzheimer dementia patients and production of protein kinase C which stabilizes the protein (see J. Neurochem., 66, 1539 (1996)) and by the knowledge that caspase is concerned with neurocyte death by β amyloid protein adhesion (see Exp. Cell Res., 234, 507 (1997)).

As to Huntington's chorea, cathepsin H activity increased in the patient's brain (see J. Neurol. Sci., 131, 65 (1995)), and the ratio of activated form of calpain (see J. Neurosci., 48, 181 (1997)) increased. In Parkinson's disease, the increase of expression of m-calpain was recognized in the mesencephalon in the patients (see Neuroscience, 73, 979 (1996)) and IL-1β protein was expressed in brain (see Neurosci. Let., 202, 17 (1995)). Therefore, it is speculated that cysteine protease is concerned with the genesis and progress of these diseases.

Otherwise, in the central nervous system, spectrin degradation by calpain is found in the process of injury on neurocyte observed in the traumatic brain injury model (see J. Neuropathol. Exp. Neurol., 58, 365 (1999)).

In spinal cord injured model it was recognized that in glia cells calpain messenger RNA increased and its activity increased in the lesion and the possibility was shown that calpain had much to do with the degeneration of myelin and actin (see Brain Res., 816, 375 (1999)). And IL-1β was shown to be concerned with the genesis of multiple sclerosis (see Immunol. Today, 14, 260 (1993)). Therefore, it is conceivable that a cysteine protease inhibitor is hopeful as an agent for the treatment of these nerve-injured diseases.

Normally, cathepsin S and cathepsin K do not exist in human arterial walls, but it was confirmed that they expressed in arteriosclerosis lesion and they had an decomposing activity of alveolus elastica (see J. Clin. Invest., 102, 576 (1998)) and a calpain inhibitor and antisense of m-calpain inhibited the proliferation of human blood vessel smooth muscle cells and it is shown that m-calpain is concerned with the proliferation of smooth muscle (see Arteioscler. Thromb. Vssc. Biol., 18, 493 (1998)), so it is conceivable that a cysteine protease inhibitor is hopeful for the treatment of blood vessel lesion such as arteriosclerosis, restenosis after percutaneous transluminal coronary angioplasty (PTCA) *etc.* And it is also reported that LDL induces cathepsin H expression in human monocyte and cathepsin H is concerned with LDL transformation and it is implied that LDL is concerned with circulatory disorder (arteriosclerosis) (see Arterioscler. Thromb. Vasc. Biol., 27 (2003)).

It is reported that in liver, cathepsin B is activated in the process of injuring hepatocyte by bile acid (see J. Clin. Invest., 103, 137 (1999)) and so it is expected that a cysteine protease inhibitor is useful for cholestatic cirrhosis.

It is reported that in spleen, cathepsin Y is concerned with production of bradykinin potentiating peptide (BPP) which plays some role in converting kinin into bradykinin (see Immunopharmacology, 45, 207 (1999)). Therefore, it is expected that cathepsin Y inhibitor has anti-allergy effect.

In lungs and respiratory system, it is shown that cathepsin S is an enzyme that plays a role in elastin degradation by alveolus macrophages (see J. Biol. Chem., 269, 11530 (1994)), so it is probable that cysteine protease is a cause of pulmonary emphysema. In IL-13 transgenic mice in which COPD-like pathology is recognized, increase of cathepsin B, S, L, H and K expression is recognized and it is also reported that administration of a cysteine protease inhibitor suppresses lung inflammation and lung emphysema (see J. Clin. Invest., 106, 1081 (2000)). And it is also shown that lung injury (see J. Clin. Invest., 97, 963 (1996)), lung fibrosis (see Cytokine, 5, 57 (193)) and bronchial asthma (see J. Immunol., 149, 3078 (1992)) are caused by way of production of IL-1β by caspase-1. It is also shown that blood cathepsin H concentration is increased in asthma patients, so antiasthma effect by cathepsin H inhibitor is expected (see Clin. Chim. Acta, 310, 113 (2001)). It is known that cathepsin H functions in the excision of surfactant protein C which is synthesized by type-2 pneumonia cells (see Am. J. Respir. Cell Mol. Biol., 26, 659 (2002)).

It is pointed out that cysteine protease is also concerned with diseases concerning bones and cartilages. Cathepsin K is specifically recognized in osteoclast and it has a decomposing activity against bone matrix (see J. Biol. Chem., 271, 12517 (1996)), so cathepsin K inhibitor is expected to show an effect in bone deseases where pathologic bone resorption is recognized, such as osteoporosis, bone fracture, arthritis, rheumatoid arthritis, osteoarthritis, hypercalcaemia, osteometastasis of cancer, periodontitis, bone Paget's disease, *etc.* Also, since IL-1β is shown to be concerned with bone resorption and cartilage degradation, and a caspase-1 inhibitor and IL-1β receptor antagonist inhibit the symptoms of bone resorption and arthritis, so it is expected that it is effective for arthritis (see Cytokine, 8, 377 (1996)) and osteoporosis (see J. Clin. Invest., 93, 1959 (1994)). And it is also reported that IL-1β is concerned with osteoarthritis (see Life Sci., 41, 1187 (1987)).

Cysteine protease is involved with production of various hormones. Since increase of messenger RNA of cathepsin S was recognized by stimuli of thytropin on thyroid epitheliocyte strains (see J. Biol. Chem., 267, 26038 (1992)), it is conceivable that a cysteine protease inhibitor is effective for hyperthyrodism.

Since quantity and activity of cathepsin B protein increased in the gingival sulcus liquid of periodontitis patients (see J, Clin. Periodontol., 25, 34 (1998)), it is pointed out that cysteine protease is concerned with periodontitis. Cathepsin G is concerned with abnormal connective tissue decomposition.

Therefore, those compounds which have an inhibitory activity against cysteine proteases, are useful as agents for the prophylaxis and/or treatment of inflammatory diseases (periodontitis, arthritis, inflammatory bowel diseases, infectious diseases, pancreatitis, hepatitis, glomerulonephritis, endocarditis, myocarditis, ulcerative colitis, *etc.),* diseases induced by apoptosis (graft versus host diseases, rejection in transplantation, acquired immunodeficiency syndrome (AIDS), AIDS-related complex (ARC), adult T cell leukemia, hairy cells leukemia, spondylopathy, disorders of respiratory apparatus, arthritis, HIV or HTLV-1 related diseases (uveitis *etc.*), virus related diseases (hepatitis C *etc.),* cancer, collagenosis (systemic lupus erythematosus, rheumatoid arthritis, *etc.*), ulcerative colitis, Sjoegren syndrome, primary biliary cirrhosis, spontaneous thrombocytopenic purpura, autoimmune hemolytic anemia, myasthenia gravis, autoimmune diseases (insulin dependent (type I) diabetes, *etc.*), diseases accompanied by thrombocytopenia (myelodysplastic syndrome, cyclic thrombocytopenia, aplastic anemia, spontaneous thrombocytopenia, disseminated intravascular coagulation (DIC), *etc.*), viral hepatitis (A, B, C, F, *etc.*) or hepatitis medicamentosa and cirrhosis, *etc*., dementia such as Alzheimer's disease, Alzheimer-type senile dementia, cerebrovascular injury, neurodegenerative disease, adult acute respiratory distress syndrome, infectious diseases, prostatomegaly, hysteromyoma, bronchial asthma, arteriosclerosis, hyperlipidemia, all kinds of lusus naturae, nephritis , senile cataract, chronic fatigue syndrome, myodystrophy, peripheral nerve injury, *etc.*), diseases induced by immune response disorder (graft versus host diseases, rejection in transplantation, allergic diseases (asthmatic bronchitis, atopic dermatitis, allergic rhinitis, hay fever, diseases by house dust, hypersensitive pneumonia, food allergy, *etc.*), psoriasis, rheumatoid arthritis, *etc.*), autoimmune diseases (insulin dependent (type I) diabetes, systemic lupus erythematosus, Hashimoto's diseases, multiple sclerosis, *etc.*), diseases induced by decomposition of proteins which compose a body (myodystrophy, cataract, periodontitis, hepatocyte injury by bile acid (cholestatic cirrhosis *etc.*), *etc.,* decomposition of alveolus elastica (emphysema *etc.*), ischemic diseases (brain ischemia, brain disorder by ischemic reperfusion, cardiac infarction, ischemic liver damage, *etc.), etc.),* shock (septic shock, systemic inflammatory responsive syndrome, endotoxin shock, acidosis, *etc*.), circulatory disorder (arteriosclerosis, restenosis after PTCA (percutaneous transluminal coronary angioplasty), *etc.*), disorder of blood coagulation system (thrombocytopenic purpura, hemolytic uremic syndrome, *etc*.), malignant tumor, acquired immune deficiency syndrome (AIDS, AIDS-related complex (ARC), *etc.),* parasitic diseases (malaria *etc.*), neurodegenerative disease (Alzheimer-type senile dementia, Huntington's chorea, Parkinson's disease, multiple sclerosis, traumatic encephalopathy, traumatic spondylopathy, *etc*.), lung disorder (fibroid lungs, *etc*.), bone diseases (osteoporosis, bone fracture, rheumatoid arthritis, arthritis, osteoarthritis, hypercalcaemia, osteometastasis of cancer, periodontitis, bone Paget's disease, *etc*.), endocrinesthenia (hyperthyroidism *etc.), etc.*

On the other hand, what is the most important for inhibitors in inhibiting the activity of proteases is, the special reaction site which interacts with the amino acid residues the activity center of proteases. The surrounding structure of the reaction sites are represented by ---P3P2P1-P1'P2'P3'---, centering peptide binding (P1-P1') of the reaction site, and at P1 site there exist amino acid residues which fit the substance specificity of proteases which the inhibitors aim. Some reaction sites against cysteine proteases are known, for example, in the specification of WO99/54317, the followings are described; P1 position against calpain I, II such as norvaline, phenylalanine, *etc.;* P1 position against calpain I such as arginine, lysine, tyrosine, valine, *etc.*; P1 position against papain such as homophenylalanine, arginine, *etc.;* P1 position against cathepsin B -- homophenylalanine, phenylalanine, tyrosine, *etc.;* P1 position against cathepsin S such as valine, norleucine, phenylalanine, *etc.*; P1 position against cathepsin L such as homophenylalanine, lysine, *etc.*; P1 position against cathepsin K such as arginine, homophenylalanine, leucine, *etc.;* P1 position against caspase such as aspartic acid, *etc.*

The compound of formula (W) 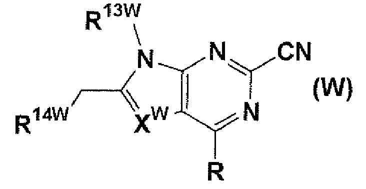 wherein all symbols are as defined in the description,
is known as a compound having an inhibitory activity against cathepsin K (see WO03/020721).

The compound of formula (Y) 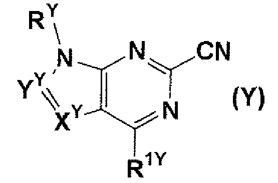 wherein all symbols are as defined in the description,
is known as a compound having an inhibitory activity against cathepsin S (see WO04/000843).

### DISCLOSURE OF THE INVENTION

A task of the present invention is to provide medicaments which are useful for the prevention and/or treatment of bone diseases such as osteoporosis.

The present inventors have energetically investigated to find out such compounds having cysteine protease inhibitory activity, to find out that the nitrile derivative of formula (I) accomplishes the purpose.

The compound in the present invention has an inhibitory activity against cystein protease, for example, cathepsin K, so it is useful for the treatment and/or prevention of bone diseases.

That is, the present invention relates to
1. A compound of formula (I) 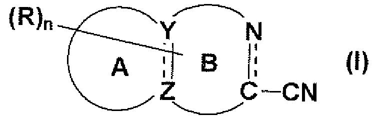 wherein ring A is a carbocyclic group or a heterocyclic group, ring B is a heterocyclic group containing at least one nitrogen atom, 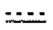 is a single bond or a double bond, Y and Z each is independently a carbon atom or a nitrogen atom, n is 0, or an integer of from 1 to 10, R is a hydrogen atom or a substituent, if R is plural, each of R are the same or different, with the proviso that two of R may form a ring which may have a substituent(s) together with an atom to which they bind,
   a salt thereof, a solvate thereof, or an N-oxide thereof, or a prodrug thereof;
2. The compound according to the above item 1, wherein ring A is a C5-7 monocyclic carbocyclic group, or a five- to seven-membered monocyclic heterocyclic group, ring B is a five- to seven-membered monocyclic heterocyclic group which contains one nitrogen atom and contains 1 to 2 hetero atom(s) optionally selected from an oxygen atom(s), a nitrogen atom(s) and a sulfur atom(s) which may be oxidized;
3. The compound according to the above item 1, wherein ring A is a ring selected from benzene, cyclopentene, cyclohexene, cycloheptene, pyridine, pyrimidine, pyrazine, tetrahydropyrimidine, dihydropyridazine, pyridazine, dihydropyrimidine, dihydropyrazine, dihydrotriazine, pyrazole, dihydropyrazole, pyrrole, imidazole, triazole, thiophene, furan, dihydrofuran, oxadiazine, tetrahydrodiazepine and diazepane;
4. The compound according to the above item 1, wherein ring B is a ring selected from pyrrole, imidazole, pyrazole, thiazole, oxazole, pyridine, pyrimidine, dihydrotriazine, pyrazine and dihydropyrimidine;
5. The compound according to the above item 1, wherein the compound of formula (I) is 5,6,7,8-tetrahydropyrimido[4,5-d]pyrimidine-2-carbonitrile, pyrazolo[1,5-a]pyrimidine-5-carbonitrile, 2,3-dihydro-1H-pyrazolo[3,4-d]pyrimidine-6-carbonitrile, pyrazolo[1,5-a][1,3,5]triazine-2-carbonitrile, 1H-pyrimido[4,5-e][1,3,4]oxadiazine-7-carbonitrile, 1H-pyrazolo[3,4-d]pyrimidine-6-carbonitrile, imidazo[1,2-a]pyrimidine-2-carbonitrile, 1,3-benzothiazol-2-carbonitrile, 6,7,8,9-tetrahydro-5H-pyrimido[4,5-e][1,4]diazepine-2-earbonitrile, 5H-pyrrolo[3,2-d]pyrimidine-2-carbonitrile, pyrido[2,3-d]pyrimidine-2-carbonitrile, 5,7-dihydrofuro[3,4-d]pyrimidine-2-carbonitrile, 6,7-dihydro-5H-cydopenta[d]pyrimidine-2-carbonitrile, 9H-purine-2-carbonitrile, or 1,3-benzoxazole-2-carbonitrile;
6. The compound according to the above item 1, which is a compound of formula (I-A) 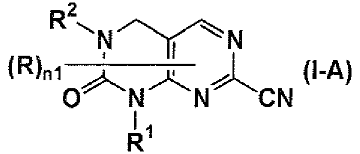 or formula(I-B) 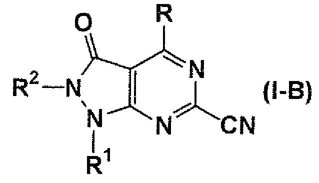 wherein R¹ is a hydrogen atom or a substituent, n1 is 0, or an integer of from 1 to 3, R² is 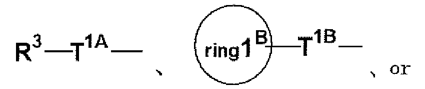 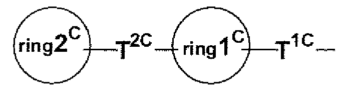 wherein T^{1A}, T^{1B}, T^{1C} and T^{2C} each is independently a bond or a spacer having from 1 to 10 atoms of the principle chain, ring 1^{B}, ring1^{C} and ring2^{C} each is independently a cyclic group which may have a substituent(s), R³ is a hydrogen atom or a substituent,
   and other symbols have the same meanings as described in the above item 1;
7. The compound according to the above item 6, wherein R¹ is a branched C1-8 alkyl;
8. The compound according to the above item 6, wherein -T^{1A}- is -E³-E²-E¹⁻(wherein E¹ and E³ each is independently a bond or a divalent C1-5 hydrocarbon group which may have a substituent(s), E² is -O-, -NR¹⁰-, -S-, -C(=O)-, -C(=O)NR¹⁰-, - NR¹⁰C(=O)-, -SO₂NR¹⁰-, -NR¹⁰SO₂-, -C(=O)O-, -OC(=O)-, or -NR¹⁰C(=O)NR¹¹⁻(wherein R¹⁰ and R¹¹ each is independently a hydrogen atom or a hydrocarbon group which may have a substituent(s)), R³ is a hydrogen atom or a substituent containing a nitrogen atom which are basic;
9. The compound according to the above item 6, wherein -T^{1B}- is -E³-E²-E¹⁻(wherein all symbols have the same meanings as described in the above item 8), ring 1^{B} is (1) C3-10 mono- or bicyclic carbocyclic group which may have a substituent(s), or (2) three- to ten-membered mono- or bicyclic heterocyclic group which may have a substituent(s);
10. The compound according to the above item 6, wherein -T^{1C}- is -E³-E²-E¹⁻(wherein all symbols have the same meanings as described in the above item 8), -T^{2C}- is a bond or a divalent C1-5 hydrocarbon group which may have a substituent(s), ring 1^{C} and/or ring 2^{C} is (1) C3-10 mono- or bicyclic carbocyclic group which may have a substituent(s), or (2) three- to ten-membered mono- or bicyclic heterocyclic group which may have a substituent(s);
11. The compound according to the above item 1, which is selected from a group that consists of
   (1) 8-(2,2-dimethylpropyl)-7-oxo-5,6,7,8-tetrahydropyrimido[4,5-d]pyrimidine-2-carbonitrile,
   (2) 1-(2,2-dimethyapropyl)-2-(4-methoxybenzyl)-3-oxo-2,3-dihydro-1H-pyrazolo[3,4-d]pyrimidine-6-carbonitrile,
   (3) 1-(2,2-dimethylpropyl)-3-[(4-methoxybenzyl)oxy]-1H-pyrazolo[3,4-d]pyrimidine-6-carbonitrile,
   (4) 4-(2,2-dimethylpropoxy)-1,3-benzothiazol-2-carbonitrile,
   (5) 3-(4-biphenylylmethoxy)-1-(2,2-dimethylpropyl)-1H-pyrazolo[3,4-d]pyrimidine-6-carbonitrile,
   (6) 2-(4-biphenylylmethyl)-1-(2,2-dimethylpropyl)-3-oxo-2,3-dihydro-1H-pyrazolo[3,4-d]pyrimidine-6-carbonitrile,
   (7) 1-(2,2-dimethylpropyl)-3-oxo-2-(2-thienylmethyl)-2,3-dihydro-1H-pyrazolo[3,4-d]pyrimidine-6-carbonitrile,
   (8) 1-(2,2-dimethylpropyl)-3-[2-(4-morpholinyl)ethoxy]-1H-pyrazolo[3,4-d]pyrimidine-6-carbonitrile,
   (9) 9-(2,2-dimethylpropyl)-6-(4-methoxybenzyl)-7-oxo-6,7,8,9-tetrahydro-5H-pyrimido[4,5-e][1,4]diazepine-2-carbonitrile,
   (10) 8-(2,2-dimethylpropyl)-6-(4-methoxybenzyl)-7-oxo-5,6,7,8-tetrahydropyrimido[4,5-d]pyrimidine-2-carbonitrile,
   (11) 4-[(2,2-dimethylpropyl)amino]pyrido[2,3-d]pyrimidine-2-carbonitrile,
   (12) 1-(2,2-dimethylpropyl)-3-{4-[(4-methyl-1-piperazinyl)methyl]phenyl}-1H-pyrimido[4,5-e][1,3,4]oxadiazine-7-carbonitrile,
   (13) 2-[6-cyano-1-(2,2-dimethylpropyl)-3-oxo-1,3-dihydro-2H-pyrazolo[3,4-d]pyrimidin-2-yl]-N-[2-(dimethylamino)ethyl]acetamide,
   (14) 4-[(2,2-dimethylpropyl)amino]-5,7-dihydrofuro[3,4-d]pyrimidine-2-carbonitrile, and
   (15) 4-[(2,2-dimethylpropyl)amino]-6,7-dihydro-5H-cyclopenta[d]pyrimidine-2-carbonitrile;
12. A pharmaceutical composition comprising the compound of formula (I) which is described in the above item 1, a salt thereof, a solvate thereof, or an N-oxide thereof, or a prodrug thereof as an active ingredient;
13. The pharmaceutical composition according to the above item 12, which is an agent for prevention and/or treatment for a cysteine protease-related disease;
14. The pharmaceutical composition according to the above item 13, wherein a cysteine protease-related disease is a cathepsin K-related disease;
15. The pharmaceutical composition according to the above item 14, wherein a cathepsin K-related disease is a bone disease;
16. The pharmaceutical composition according to the above item 15, wherein a bone disease is at least one selected from osteoporosis, bone fracture, arthritis, rheumatoid arthritis, osteoarthritis, hypercalcaemia, osteometastasis of cancer, osteosarcoma, periodontitis, and bone Paget's disease;
17. The pharmaceutical composition according to the above item 12, which is a bone resorption inhibitor;
18. A drug comprising the compound of formula (I) described in the above item 1, a salt thereof, a solvate thereof, or an N-oxide thereof, or a prodrug thereof, combined with at least one selected from bisphosphonate formulations, Vitamin D and its derivatives, Vitamin K and its derivatives, calcitonin formulations, α-calcitonin gene-related peptide formulations, female hormone formulations, selective estrogen receptor modulators, ipriflavone formulations, calcium formulations, anabolic steroid formulations, parathyroid hormone formulations, PTHrP derivatives, caspase-1 inhibitors, farnesoid X receptor agonists, Bone Morphogenetic Protein formulations, anti-RANKL antibody, metalloprotease inhibitors, prostaglandin derivatives, strontium formulations, anti TNF-α antibody, anti-IL-6 antibody, HMG-CoA reductase inhibitors, steroidal drugs, and antiinflammatory drugs;
19. A method for the prophylaxis and/or treatment of a cysteine protease-related disease in a mammal characterized by administering to a mammal an effective amount of the compound of formula (I) described in the above item 1, a salt thereof a solvate thereof, or an N-oxide thereof, or a prodrug thereof; and
20. Use of the compound of formula (I) described in the above item 1, a salt thereof, a solvate thereof, or an N-oxide thereof, or a prodrug thereof, for the manufacture of a pharmaceutical preparation for the prophylaxis and/or treatment of a cysteine protease-related disease.

Definition of the term in the present invention is described below.

Examples of a carbocyclic group represented by ring A include C3-15 mono-, polycyclic (bi- or tricyclic) carbocyclic group, more concretely, for example, C3-15 mono-, polycyclic aromatic carbocyclic group, and partially saturated or fully saturated carbocyclic group, polycyclic carbocyclic group having spiro bond, and polycyclic bridged carbocyclic group, *etc.* C3-15 mono-, polycyclic aromatic carbocyclic group, partially saturated or fully saturated carbocyclic group includes, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, as-indacene, s-indacene, acenaphthylene, acenaphthene, fluorene, phenalene, phenanthrene, anthracene ring, *etc.* Polycyclic carbocyclic group having spiro bond, and polycyclic bridged carbocyclic group include, for example, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, bicyclo[3.3.0]octane, bicyclo[4.3.0]nonane, bicyclo[4.4.0]decane, adamantane and noradamantane ring, *etc*.

Example of a heterocyclic group represented by ring A includes three- to fifteen-membered mono-, polycyclic (bi- or tricyclic) heterocyclic group, more concretely, for example, three- to fifteen-membered mono-, polycyclic aromatic heterocyclic group, and polycyclic heterocyclic group having spiro bond, and polycyclic bridged heterocyclic group which contain 1 to 5 hetero atoms selected from an oxygen atom(s), a nitrogen atom(s) and a sulfur atom(s) which may be oxidized (S, SO, SO₂), and which may be partially saturated or fully saturated, *etc*. Three- to fifteen-membered mono-, polycyclic aromatic heterocyclic group, and polycyclic heterocyclic group having spiro bond, and polycyclic bridged heterocyclic group which contain 1 to 5 hetero atoms selected from an oxygen atom(s), a nitrogen atom(s) and a sulfur atom(s) which may be oxidized, and which may be partially saturated or fully saturated include, for example, pyrrole, imidazole, triazole, tetrazole, pyrazole, pyridine, pyrazine, pyrimidine, pyridazine, triazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, pyrrolopyridine, benzoxazole, benzothiazole, thieno[3,2-c]pyridine, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, pyridonaphthyridine, pyrazoloisoquinoline, pyrazolonaphthyridine, pyrimidoindole, aziridine, azetidine, pyrroline, pyrrolidine, imidazoline, imidazolidine, triazoline, triazolidine, tetrazoline, tetrazolidine, dihydropyrazole (pyrazoline), tetrahydropyrazole (pyrazolidine), dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrotriazine, tetrahydrotriazine, perhydrotriazine, dihydroazepine, tetrahydroazepine, azepane (perhydroazepine), dihydrodiazepine, tetrahydrodiazepine, diazepane (perhydrodiazepine), oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, octahydroquinoline, perhydroquinoline, dihydroisoquinoline, tetrahydroisoquinoline, octahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, 4,5,6,7-tetrahydrothieno[3.2-c]pyridine, dihydrobenzimidazole, perhydrobenzimidazole, dihydrobenzazepine, tetrahydrobenzazepine, dihydrobenzodiazepine, tetrahydrabenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, tetrahydropyridonaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydrapyrazolopyridoazepine, hexahydropyrazolopyridoazepine, tetrahydropyrimidoindole, dihydrothiazinoindole, tetrahydrothiazinoindole, dihydrooxazinoindole, tetrahydrooxazinoindole, 2,3-dihydro-1H-benzo[de]isoquinoline, dioxolane, dioxane, dithiolane, dithiane, benzodioxole, for example, 1,3-benzodioxole, *etc.,* benzodioxane, chroman, benzodithiolane benzodithiane, azaspiro[4.4]nonane, oxaazaspiro[4.4]nonane, oxaazaspiro[2.5]actane, dioxaspiro[4.4]nonane, azaspiro[4.5]decane, thiaspiro[4.5]decane, dithiaspiro[4.5]decane, dioxaspiro[4.5]decane, oxaazaspiro[4.5]decane, azaspiro[5.5]undecane, oxaspiro[5.5]undecane, dioxaspiro[5.5]undecane, 1,4-dioxa-8-azaspiro[4.5]undecane, 1,3,8-triazaspiro[4.5]decane, azabicycio[2.2.1]heptane, oxabicyclo[2.2.1]heptane, azabicyclo[3.1.1]heptane, azabicyclo[3.2.1]octane, axabicyclo[3.2.1]octane, azabicyclo[2.2.2]octane, diazabicyclo[2.2.2]octane, 2,5-diazabicyclo[2.2.1]heptane, 1,3,8-triazaspiro[4.5]decane, 2,7-diazaspiro[4.5]decane, 1,4,9-triazaspiro[5.5]undecane, 2,4,6-trioxaspiro[bicyclo[3.3.0]octane-3,1'-cyclohexane], 1,3-dioxalana[4,5-g]chromene, 2-oxabicyclo[2.2.1]heptane, 2,3,4,9-tetrahydrospiro[β-carboline-1,1'-cyclopentane] ring, *etc.*

Example of "a heterocyclic group containing at least one nitrogen atom" represented by ring B include three- to fifteen-membered mono-, polycyclic (bi- or tricyclic) heterocyclic group which contains one nitrogen atom and contains 1 to 4 hetero atoms optionally selected from an oxygen atom(s), a nitrogen atom(s) and a sulfur atom(s) which may be oxidized, more concretely, for example, three- to fifteen-membered mono-, polycyclic aromatic heterocyclic group, and polycyclic heterocyclic group having spiro bond, and polycyclic bridged heterocyclic group which contain one nitrogen atom and contain 1 to 4 hetero atoms optionally selected from an oxygen atom(s), a nitrogen atom(s) and a sulfur atom(s) which may be oxidized and may be partially saturated or fully saturated. Three- to fifteen-membered mono-, polycyclic aromatic heterocyclic group, and polycyclic heterocyclic group having spiro bond, and polycyclic bridged heterocyclic group which contain one nitrogen atom and contain 1 to 4 hetero atoms optionally selected from an oxygen atom(s), a nitrogen atom(s) and a sulfur atom(s) which may be oxidized and may be partially saturated or fully saturated include, for example, pyrrole, imidazole, triazole, tetrazole, pyrazole, pyridine, pyrazine, pyrimidine, triazine, pyridazine, azepine, diazepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indazole, quinoline, isoquinoline, purine, phthalazine, pteridine, naphthyridine, quinoxaline, quinazoline, cinnoline, pyrrolopyridine, benzoxazole, benzothiazole, benzimidazole, benzoxazepine, benzoxadiazepine, benzothiazepine, benzothiadiazepine, benzazepine, benzodiazepine, β-earboline, phenanthridine, phenanthroline, pyridonaphthyridine, pyrazololsoquinoline, pyrazolanaphthyridine, pyrimidoindole, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrotriazine, tetrahydrotriazine, perhydrotriazine, dihydroazepine, tetrahydroazepine, azepane (perhydroazepine), dihydrodiazepine, tetrahydrodiazepine, diazepane (perhydrodiazepine), dihydroisoxazole, tetrahydroisoxazole (isoxazolidine), dihydrothiazole, tetrahydrothiazole (thiazolidine), dihydroisothiazole, tetrahydroisothiazole (isathiazolidine), dihydrofurazan, tetrahydrofurazan, dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnaline, perhydrocinnoline, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, dihydrobenzoxazepine, tetrahydrobenzoxazepine, tetrahydropyridonaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, azaspiro[4.4]nonane, oxaazaspiro[4.4]nonane, oxaazaspiro[2,5]octane, dioxaspiro[4.4]nonane, azaspiro[4.5]decane, thiaspiro[4.5]decane, dithiaspiro[4.5]decane, dioxaspiro[4.5]decane, oxaazaspiro[4.5]decane, azaspira[5.5]undecane, oxaspiro[5.5]undecane, dioxaspiro[5.5]undecane, 1,4-dioxa-8-azaspiro[4.5]undecane, 1,3,8-triazaspiro[4.5]decane, azabicyclo[2.2.1]heptane, axabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane, 2,5-diazabicyclo[2.2.1]heptane, 1,3,8-triazaspiro[4.5]decane, 2,7-diazaspiro[4.5]decane, 1,4,9-triazaspiro[5.5]undecane, 2,4,6-trioxaspiro[bicyclo[3.3.0]octane-3,1'-cyclohexane], 1,3-dioxolano[4,5-g]chromene, 2-oxabicyclo[2.2.1]heptane, 2,3,4,9-tetrahydrospiro[β-carboline-1,1'-cyclopentane] ring, *etc*.

A substituent represented by R includes, for example, (1) hydrocarbon group which may have a substituent(s), (2) carbocyclic group which may have a substituent(s), (3) heterocyclic group which may have a substituent(s), (4) hydroxy which may have a substituent(s), (5) mercapto which may have a substituent(s), (6) amino which may have a substituent(s), (7) carbamoyl which may have a substituent(s), (8) sulfamoyl which may have a substituent(s), (9) carboxy, (10) alkoxycarbonyl (e.g. C1-6 alkoxycarbonyl such as methoxycarbonyl, ethoxycarbonyl, tert-butoxycarbonyl, *etc.),* (11) sulfo, (12) sulfino, (13) phosphono, (14) nitro, (15) cyano, (16) amidino, (17) imino, (18) dihydroxyboryl, (19) halogen (fluoro, chloro, bromo, iodo), (20) alkylsulfinyl (e.g. C1-6 alkylsulfinyl such as methylsulfinyl, ethylsulfinyl, *etc.*), (21) aromatic ring sulfinyl (e.g. C6-10 aromatic ring sulfinyl such as phenylsulfinyl, *etc*.), (22) alkylsulfinyl (e.g. C1-6 alkylsulfinyl such as methylsulfonyl, ethylsulfonyl, *etc*.), (23) aromatic ring sulfonyl (e.g. C6-10 aromatic ring sulfonyl such as phenylsulfonyl, *etc.),* (24) oxo, (25) thioxo, (26) (C1-6 alkoxyimino)methyl (e.g. (methoxyimino)methyl, *etc.),* (27) acyl, (28) formyl, (29) alkyl substituted by hydroxy which may have a substituent(s), (30) alkyl substituted by mercapto which may have a substituent(s), (31) alkyl substituted by amino which may have a substituent(s), (32) (alkyl which may have a substituent(s))oxycarbonyl, (33) HO-(CO-amino acid residue-NH)ₘ-CO-T⁰- which may have a substituent(s), (34) H-(NH-amino acid residue-CO)ₘ₋O-T⁰- which may have a substituent(s), *etc.*

If R is plural, each ofR are the same or different.

Hydrocarbon group in "(1) hydrocarbon group which may have a substituent(s)" includes, for example, alkyl, alkenyl, alkynyl, alkylidene, alkenylidene, *etc.* Alkyl group includes, for example, straight chain or branched C1-8 alkyl such as methyl, ethyl, propyl, isopropyl, butyl, isobutyl, *tert*-butyl, pentyl, neopentyl, hexyl, heptyl, octyl, *etc*. Alkenyl group includes, for example, straight chain or branched C 2-8 alkenyl such as ethenyl, propenyl, butenyl, pentenyl, hexenyl, heptenyl, octenyl, *etc.* Alkynyl group includes, for example, straight chain or branched C2-8 alkynyl such as ethynyl, propynyl, butynyl, pentynyl, hexynyl heptynyl, octynyl, *etc.* Alkylidene includes, for example, straight chain or branched C1-8 alkylidene such as methylidene, ethylidene, propylidene, butylidene, pentylidene, hexylidene, heptylidene, octylidene, *etc.*

Alkenylidene includes, for example, straight chain or branched C2-8 alkenylidene such as ethenylidene, propenylidene, butenylidene, pentenylidene, hexenylide, heptenylidene, octenylidene, *etc.*

Substituent in "(1) hydrocarbon group which may have a substituent(s)" includes, for example, hydroxy, mercapto, amino, carboxy, nitro, cyano, oxo, mono- or di-C1-6 alkylamino (e.g. methylamino, ethylamino, propylamino, dimethylamino, diethylamino, *etc.*), N-aromatic ring amino (e.g. N-phenylamino, *etc.*), N-aromatic ring-N-alkylamino (e.g. N-phenyl-N-methylamino, N-phenyl-N-ehtylamino, N-phenyl-N-propylamino, N-phenyl-N-butylamina, N-phenyl-N-penntylamino, N-phenyl-N-hexylamino, *etc.*), acylamino, N-acyl-N-alkylamino, C1-6 alkoxy (e.g. methoxy, ethoxy, propoxy, isopropoxy, butoxy, tert-butoxy, hexyloxy, *etc.*), C3-7 cycloalkyl-Cl-6 alkoxy (e.g. cyclohexylmethyloxy, cyclopentylethyloxy, *etc.),* C3-7 cycloalkyloxy (e.g. cyclohexyloxy, *etc.*), C7-15 aralkyloxy (e.g. benzyloxy, phenethyloxy, phenylpropyloxy, naphthylmethyloxy, naphthylethyloxy, *etc.*), phenoxy, C1-6 alkoxycarbonyl (e.g. methoxycarbonyl, ethoxycarbonyl, tert-butoxycarbonyl, *etc.*), C1-6 alkylcarbonyloxy (e.g. acetoxy, ethylcarbonyloxy, *etc.*), C1-6 alkylthio (e.g. methylthio, ethylthio, propylthio, butylthio, hexylthio, *etc.*), halogen (fluoro, chloro, bromo, iodo), C1-6 alkylsulfonyl (e.g. methylsulfonyl, ethylsulfonyl, propylsulfonyl, butylthio, hexylsulfonyl, *etc.*), aromatic ring sulfonyl (e.g. C6-10 aromatic ring sulfonyl such as phenylsulfonyl, *etc.*), carbamoyl which may have a substituent(s) (e.g. non-substituted carbamoyl, N-mono-Cl-8 alkylcarbamoyl (N-methylcarbamoyl, N-ethylcarbamoyl, N-propylcarbamoyl, N-isopropylcarbamoyl, N-butylcarbamoyl, *etc*.), N,N-di(Cl-8 alkyl)carbamoyl (e.g. N,N-dimethylcarbamoyl, N,N-diethylcarbamoyl, N,N-dipropylcarbamoyl, N,N-dibutylcarbamoyl, *etc.*), piperldin-1-ylcarbonyl, piperazin-1-ylcarbonyl, morpholin-4-ylcarbonyl, *etc.),* acyl, carbocyclic group which may have a substituent(s), heterocyclic group which may have a substituent(s), -O-(carbocyclic group which may have a substituent(s)), -O-(heterocyclic group which may have a substituent(s)), *etc.* The 1 to 4 substituent(s) may exist wherever possible. Alkyl in the N-acyl-N-alkylamino includes, for example, straight chain or branched C1-6 alkyl such as methyl, ethyl, propyl, isopropyl, butyl, isobutyl, *sec*-butyl, *tert*-butyl, pentyl, hexyl, *etc*. Acyl, acyl in the acylamino and N-acyl-N-alkylamino have the same meanings as the below-described "(27) acyl". Carbocyclic group which may have a substituent(s) in the "carbocyclic group which may have a substituent(s)", "heterocyclic group which may have a substituent(s)", "-O- (carbocyclic group which may have a substituent(s)), and heterocyclic group which may have a substituent(s) in the -O-(heterocyclic group which may have a substituent(s)) have the same meanings as the below-described "(2) carbocyclic group which may have a substituent(s)", and "(3) heterocyclic group which may have a substituent(s)", respectively.

Carbocyclic group in the "(2) carbocyclic group which may have a substituent(s)" has the same meaning as the above-described carbocyclic group represented by ring A.

Substituent in the "(2) carbocyclic group which may have a substituent(s)" includes, for example, straight chain or branched C1-6 alkyl which may be substituted by hydroxy (e.g. methyl, ethyl, propyl, isopropyl, butyl, isobutyl, *sec*-butyl, *tert*-butyl, pentyl, hexyl, *etc.*), straight chain or branched C2-6 alkenyl (e.g. ethenyl, propenyl, butenyl, pentenyl, hexenyl, *etc.*), straight chain or branched C2-6 alkynyl (e.g. ethynyl, propynyl, butynyl, pentynyl, hexynyl, *etc.*), hydroxy, straight chain or branched C1-6 alkoxy (e.g. methoxy, ethoxy, propoxy, isopropoxy, butoxy, isobutyloxy, tert-butoxy, pentyloxy, hexyloxy, *etc.*), mercapto, straight chain or branched C1-6 alkylthio (e.g. methylthio, ethylthio, propylthio, isopropylthio, butylthio, isobutylthio, tert-butylthio, pentylthio, hexylthio, *etc.*), amino, mono- or di-C1-6 alkylamino (e.g. methylamino, ethylamino, propylamino, isopropylamino, butylamino, isobutylaznino, tert-butylamino, pentylamino, hexylamino, dimethylamino, diethylamino, dipropylamino, N-methyl-N-ethylamino, *etc.*), halogen (fluoro, chloro, bromo, iodo), cyano, nitro, carboxy, straight chain or branched C1-6 alkoxycarbonyl (e.g. methoxycarbonyl, ethoxycarbonyl, tert-butoxycarbonyl, *etc*.), trihalomethyl (e.g. trifluoromethyl, *etc.*), trihalomethoxy (e.g. trifluoromethoxy, *etc.*), trihalomethylthio (trifluoromethylthio, *etc.*), dihalomethylthio (difluoromethylthio, *etc.),* oxo, carbocyclic group (which has the same meaning as the above-described carbocyclic group represented by ring A.), heterocyclic group (which has the same meaning as the above-described heterocyclic group represented by ring A), *etc.* And the 1 to 4 substituent(s) may exist wherever possible.

Heterocyclic group in the "(3) heterocyclic group which may have a substituent(s)" has the same meaning as the above-described heterocyclic group represented by ring A.

Substituent in the "(3) heterocyclic group which may have a substituent(s)" has the same meaning as the above-described substituent(s) in the "(2) carbocyclic group which may have a substituent(s)".

Substituent in the "(4) hydroxy which may have a substituent(s)", "(5) mercapto which may have a substituent(s)", "(6) amino which may have a substituent(s)", "(33) HO-(CO-amino acid residue-NH)ₘ-CO-T⁰- which may have a substituent(s)", and "(34) H-(NH-amino acid residue-CO)ₘ-O-T⁰- which may have a substituent(s)" includes, for example, hydrocarbon group which may have a substituent(s) (which has the same meaning as the above-described "(1) hydrocarbon group which may have a substituent(s)"), carbocyclic group which may have a substituent(s) (which has the same meaning as the above-described "(2) carbocyclic group which may have a substituent(s)"), heterocyclic group which may have a substituent(s) (which has the same meaning as the above-described "(3) heterocyclic group which may have a substituent(s)"), alkylsulfonyl (e.g. C1-4 alkylsulfonyl such as methylsulfonyl, ethylsulfonyl, *etc.*), aromatic ring sulfonyl (e.g. C6-10 aromatic ring sulfonyl such as phenylsulfonyl, *etc*.), acyl (which has the same meaning as the below-described "(27) acyl"), (alkyl which may have a substituent(s))oxycarbonyl (which has the same meaning as the below-described "(32) (alkyl which may have a sub stituent(s))oxycarbonyl"), *etc.* A hydrogen atom of amino in the "(6) amino which may have a substituent(s)" may be replaced by one or two substituent(s).

"(7) Carbamoyl which may have a substituent(s)" includes, for example, non-substituted carbamoyl, N-mono-Cl-6 alkylcarbamoyl (e.g. N-methylcarbamoyl, N-ethylcarbamoyl, N-propylcarbamoyl, N-isopropylcarbamoyl, N-butylcarbamoyl, *etc.*), N,N-di(C1-6 alkyl)carbamoyl (e.g. N,N-dimethylcarbamayl, N,N-diethylcarbamoyl, N,N-dipropylcarbamoyl, N,N-dibutylcarbamoyl, *etc.*), piperldin-1-ylcarbonyl, piperazin-1-ylcarbonyl, morpholin-4-ylcarbonyl, *etc.*

"(8) Sulfamoyl which may have a substituent(s)" includes, for example, non-substituted sulfamoyl, N-mono-C1-6 alkylsulfamoyl (e.g. N-methylsulfamoyl, N-ethylsulfamoyl, N-propylsulfamoyl, N-isopropylsulfamoyl, N-butylsulfamoyl, *etc*.), N,N-di(C1-6 alkyl)sulfamoyl (e.g. N,N-dimethylsulfamoyl, N,N-diethylsulfamoyl, N,N-dipropylsulfamoyl, N,N-dibutylsulfamoyl, *etc.), etc.*

"(27) Acyl" includes, for example, alkylcarbonyl which may have a substituent(s) (wherein alkyl which may have a substituent(s) has the same meaning as the above-described "alkyl which may have a substituent(s)" in the "(1) hydrocarbon group which may have a substituent(s)".), (alkenyl which may have a substituent(s))carbonyl (wherein alkenyl which may have a substituent(s) has the same meaning as the above-described "alkenyl which may have a substituent(s)" in the "(1) hydrocarbon group which may have a substituent(s)".), (alkynyl which may have a substituent(s))carbonyl (wherein alkynyl which may has a substituent(s) have the same meaning as the above-described "alkynyl which may have a substituent(s)" in the "(1) hydrocarbon group which may have a substituent(s)".), (carbocyclic group which may have a substituent(s))carbonyl (wherein carbocyclic group which may have a substituent(s) has the same meaning as the above-described "(2) carbocyclic group which may have a substituent(s)".), (heterocyclic group which may have a substituent(s))carbonyl (wherein heterocyclic group which may have a substituent(s) has the same meaning as the above-described "(3) heterocyclic group which may have a substituent(s)".), *etc*.

Hydroxy which may have a substituent(s) in the "(29) alkyl substituted by hydroxy which may have a substituent(s)" has the same meaning as the above-described "(4) hydroxy which may have a substituent(s)", mercapto which may have a substituent(s) in the "(30) alkyl substituted by mercapto which may have a substituent(s)" has the same meaning as the above-described "(5) mercapto which may have a substituent(s)", amino which may have a substituent(s) in the "(31) alkyl substituted by amino which may have a substituent(s)" has the same meaning as the above-described "(6) amino which may have a substituent(s)". Alkyl in the "(29) alkyl substituted by hydroxy which may have a substituent(s)", "(30) alkyl substituted by mercapto which may have a substituent(s)" and "(31) alkyl substituted by amino which may have a substituent(s) includes, for example, straight chain or branched C1-6 alkyl such as methyl, ethyl, propyl, isopropyl, butyl, isobutyl, *sec*-butyl, *tert*-butyl, pentyl, hexyl, *etc.*

Alkyl which may have a substituent(s) in the "(32) (alkyl which may have a substituent(s))oxycarbonyl" has the same meaning as the above-described "alkyl which may have a substituent(s)" in the "(1) hydrocarbon group which may have a substituent(s)".

"m" in the "(33) HO-(CO-amino acid residue-NH)ₘ-CO-T⁰- which may have a substituent(s)", and "(34) H-(NH-amino acid residue-CO)ₘ-O-T⁰- which may have a substituent(s)" is an integer of 1 to 3. "Amino acid" means natural amino acid or unusual amino acid, and includes, for example, glycine, alanine, valine, leucine, isoleucine, serine, threonine, cystein, methionine, proline, asparagine, glutamine, phenylalanine, tyrosine, tryotophan, aspartic acid, glutamic acid, lysine, arginine, histidine, β-alanine, cystathionine, cystine, homoserine, isoleucine, lanthionine, norleucine, norvaline, ornithine, sarcosine, thyronine, *etc.* T° in the group has the same meaning as the below-described T^{1A}.

A substituent represented by R also includes group described hereinafter; 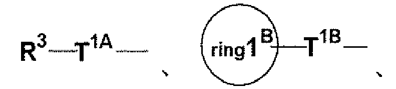 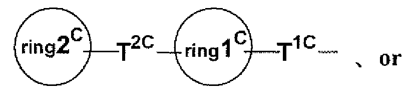 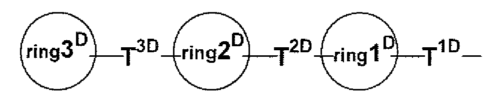 wherein T^{1A}, T^{1B}, T^{1C}, T^{1D}, T^{2C}, T^{2D} and T^{3D} each is independently a bond or a spacer having from 1 to 10 atoms of the principle chain, ring^{1B}, ring^{1C}, rimg^{1D}, ring^{2C}, ring^{2D}, and ring^{3D} each is independently a cyclic group which may have a substituent(s).

A spacer having from 1 to 10 atoms of the principle chain represented by T^{1A}, T^{1B}, T^{1C}, T^{1D}, T^{2C}, T^{2D} and T^{3D} means a space formed by 1 to 10 continued atoms of a main chain. In this case, the "number of atoms as a principle chain" should be counted such that atoms as a main chain become minimum. "A spacer having from 1 to 10 atoms of the principle chain" includes, for example, a bivalent group formed by 1 to 10 continued atoms of a main chain comprising 1 to 10 selected from -O-, -S-, - S(O)-, -SO₂-, -CO-, a nitrogen atom which may have a substituent(s), and a bivalent C1-10 aliphatic hydrocarbon group which may have a substituent(s), *etc*. "A nitrogen atom which may have a substituent(s)" includes, an -NH- in which hydrogen atom is replaced by hydrocarbon group which may have a substituent(s) (which has the same meaning as the above-described "(1) hydrocarbon group which may have a substituent(s)".) besides -NH- and =N-.

"A divalent Cl-10 aliphatic hydrocarbon group" in the "a divalent Cl-10 aliphatic hydrocarbon group which may have a substituent(s)" includes, for example, C1-10 alkylene (e.g. methylene, ethylene, propylene, butylene, pentylene, hexylene, heptylene, octylene, nonylene, decylene, *etc.*), C2-10 alkenylene (e.g. ethenylene, propenylene, butenylene, butadienylene, penntenylene, pentadienylene, hexenylene, hexadienylene, heptenylene, heptadienylene, octenylene, ocadienylene, nonenylene, nonadienylene, decenylene, decadienylene, *etc.*), C2-10 alkynylene, (e.g. ethynylene, propynylene, butynylene, butadiynylene, pentynylene, pentadiynylene, hexynylene, hexadiynylene, heptynylene, heptadiynylene, octynylene, octadiynylene, nonynylene, nonadiynylene, decynylene, decadiynylene), *etc.* "Substituent"in the "a divalent C1-10 aliphatic hydrocarbon group which may have a substituent(s)" has the same meaning as the above-described " substituent"in the "(1) hydrocarbon group which may have a substituent(s)", and the 1 to 4 substituent(s) may exist wherever possible.

Ring in the "ring which may have a substituent(s)" represented by ring^{1B}, ring^{1C}, ring^{1D}, ring^{2C}, ring^{2D} and ring^{3D} includes, for example, carbocyclic group and heterocyclic group, *etc*. This carbocyclic group and heterocyclic group have the same meanings as the above-described carbocyclic group and heterocyclic group represented by ring A.

Substituent in the "ring which may have a substituent(s)" represented by ring^{1B}, ring^{1C}, ring^{1D}, ring^{2C}, ring^{2D} and ring^{3D} includes, for example, hydrocarbon group which may have a substituent(s) (which has the same meaning as above-described "(1) hydrocarbon group which may have a substituent(s)".), carbocyclic group which may have a substituent(s) (which has the same meaning as the above-described "(2) carbocyclic group which may have a substituent(s)".), heterocyclic group which may have a substituent(s) (which has the same meaning as the above-described "(3) heterocyclic group which may have a substituent(s)".), hydroxy which may have a substituent(s) (which has the same meaning as the above-described "(4) hydroxy which may have a substituent(s)".), mercapto which may have a substituent(s) (which has the same meaning as the above-described "(5) mercapto which may have a substituent(s)".), amino which may have a substituent(s) (which has the same meaning as the above-described "(6) amino which may have a substituent(s)".), carbamoyl which may have a substituent(s) (which has the same meaning as the above-described "(7) carbamoyl which may have a substituent(s)".), sulfamoyl which may have a substituent(s) (which has the same meaning as the above-described "(8) sulfamoyl which may have a substituent(s)".), carboxy, alkoxycarbonyl (e.g. C1-6 alkoxycarbonyl such as methoxycarbonyl, ethoxycarbonyl, tert-butoxycarbonyl, *etc.),* sulfo, sulfino, phosphono, nitro, cyano, amidino, imino, dihydroborono, halogen (fluoro, chloro, bromo, iodo), alkylsulfinyl (e.g. C1-6 alkylsulfinyl such as methylsulfinyl, ethylsulfinyl, *etc.),* aromatic ring sulfinyl (e.g. C6-10 aromatic ring sulfinyl such as phenylsulfinyl, *etc.*), alkylsulfonyl (e.g. C1-6 alkylsulfonyl such as methylsulfonyl, ethylsulfonyl, *etc.),* aromatic ring sulfonyl (e.g. C6-10 aromatic ring sulfonyl such as phenylsulfonyl, *etc.*), oxo, thioxo, (C1-6 alkoxyimino)methyl (e.g. (methoxyimino)methyl, *etc.),* acyl (which has the same meaning as the above-described "(27) acyl".), formyl, alkyl substituted by hydroxy which may have a substituent(s) (which has the same meaning as the above-described "(29) alkyl substituted by hydroxy which may have a substituent(s)".), alkyl substituted by mercapto which may have a substituent(s) (which has the same meaning as the above-described "(30) alkyl substituted by mercapto which may have a substituent(s)".), alkyl substituted by amino which may have a substituent(s) (which has the same meaning as the above-described "(31) alkyl substituted by amino which may have a substituent(s)".), (alkyl which may have a sub stituent(s))oxycarbonyl (which has the same meaning as the above-described "(32) (alkyl which may have a subslituent(s))oxycarbonyl".), and the 1 to 5 substituent(s) may exist wherever possible.

"A ring which may have a substituent(s)" formed by two of Rs together with an atom to which they bind has the same meaning as the above-described "a ring which may have a substituent(s)" represented by ring^{1B}.

"Substituent" represented by R¹ or R³ has the same meaning as the above-described substituent(s) represented by R.

When 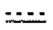 in ring B is single bond, a compound represented by formula (I) includes a compound represented by formula described below 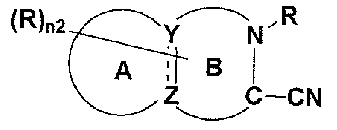 wherein n2 is 0 or an integer of 1 to 9, other symbols have the same meaning as described above.

Preferred as carbocyclic group represented by ring A is, for example, C3-10 mono-, or bicyclic carbocyclic group (e.g. C3-10 mono-, or bicyclic aromatic carbocyclic group and partially saturated or fully saturated carbocyclic group, *etc.*). More preferably is, for example, C3-10 monocyclic carbocyclic group (e.g. C3-10 monocyclic aromatic carbocyclic group and partially saturated or fully saturated carbocyclic group, *etc.*), or C5-10 bicyclic carbocyclic group (e.g. C5-10 bicyclic aromatic carbocyclic group and partially saturated or fully saturated carbocyclic group, *etc.*). More preferred is, for example, a C5-7 monocyclic carbocyclic group (e.g. C5-7 monocyclic aromatic carbocyclic group and partially saturated or fully saturated carbocyclic group, *etc.).* Most preferred is, for example, cyclopropane, cyclobutane, cyclopentane, cyclohexane, cycloheptane, cyclopentene, cyclohexene, cycloheptene, cyclopentadiene, cyclohexadiene, cycloheptadiene, benzene ring.

Preferred as heterocyclic group represented by ring A is, for example, three-to ten-membered mono-, or bicyclic heterocyclic group (e.g. three- to ten-membered mono-, or bicyclic aromatic heterocyclic group which contains 1 to 5 hetero atoms selected from an oxygen atom(s), a nitrogen atom(s) and a sulfur atom(s) which may be oxidized, and which may be partially saturated or fully saturated, *etc.*). More preferably is, for example, three- to ten-membered monocyclic heterocyclic group (e.g. three- to ten-membered monocyclic aromatic heterocyclic group which contains 1 to 5 hetero atoms selected from an oxygen atom(s), a nitrogen atom(s) and a sulfur atom(s) which may be oxidized, and which may be partially saturated or fully saturated, *etc.*), or five- to ten-membered bicyclic heterocyclic group (e.g. five- to ten-membered bicyclic aromatic heterocyclic group which contains 1 to 5 hetero atoms selected from an oxygen atom(s), a nitrogen atom(s) and a sulfur atom(s) which may be oxidized, and which may be partially saturated or fully saturated, *etc.*). More preferred is, for example, a five- to seven-membered monocyclic heterocyclic group (e.g. five- to seven-membered monocyclic aromatic heterocyclic group which contains 1 to 3 hetero atoms selected from an oxygen atom(s), a nitrogen atom(s) and a sulfur atom(s) which may be oxidized, and which may be partially saturated or fully saturated, *etc.*). Most preferred is, for example, pyridine, pyrimidine, pyrazine, tetrahydropyrimidine, dihydropyridazine, pyridazine, dihydropyrimidine, dihydropyrazine, dihydrotriazine, pyrazole, dihydropyrazole, pyrrole, imidazole, triazole, thiophene, furan, dihydrofuran, oxadiazine, tetrahydrodiazepine or diazepane ring.

Preferred as 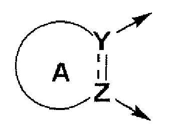 wherein the rightward arrow represents a binding position to ring B, is 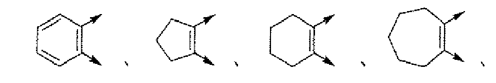 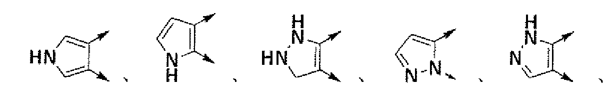 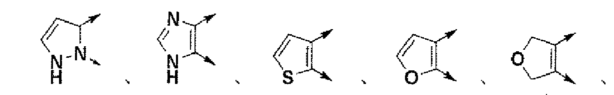 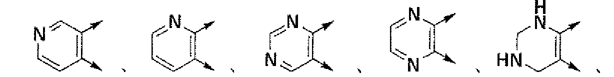 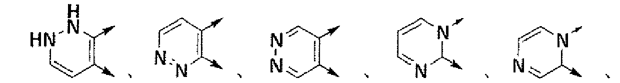 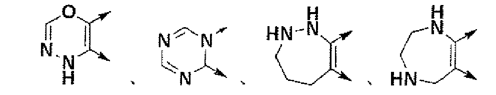 wherein a hydrogen atom bound to a nitrogen atom may be replaced by R.

Preferred as a heterocyclic group containing at least one nitrogen atom represented by ring B is, for example, a three- to ten-membered monocyclic or bicyclic heterocyclic group which contains one nitrogen atom and contains 1 to 2 hetero atoms optionally selected from an oxygen atom(s), a nitrogen atom(s) and a sulfur atom(s) which may be oxidized (e.g. three- to ten-membered mono-, or bicyclic aromatic heterocyclic group which contains one nitrogen atom and contains 1 to 2 hetero atoms optionally selected from an oxygen atom(s), a nitrogen atom(s) and a sulfur atom(s) which may be oxidized, and may be partially saturated or fully saturated, *etc.*). More preferably is, for example, a three- to ten-membered monocyclic heterocyclic group which contains one nitrogen atom and contains 1 to 2 hetero atoms optionally selected from an oxygen atom(s), a nitrogen atom(s) and a sulfur atom(s) which may be oxidized (e.g. three- to ten-membered monocyclic aromatic heterocyclic group which contains one nitrogen atom and contains 1 to 2 hetero atoms optionally selected from an oxygen atom(s), a nitrogen atom(s) and a sulfur atom(s) which may be oxidized, and may be partially saturated or fully saturated, *etc.*), or a five- to ten-membered monocyclic heterocyclic group which contains one nitrogen atom and contains 1 to 2 hetero atoms optionally selected from an oxygen atom(s), a nitrogen atom(s) and a sulfur atom(s) which may be oxidized (e.g. five- to ten-membered bicyclic aromatic heterocyclic group which contains one nitrogen atom and contains 1 to 2 hetero atoms optionally selected from an oxygen atom(s), a nitrogen atom(s) and a sulfur atom(s) which may be oxidized, and may be partially saturated or fully saturated, *etc.*). More preferred is, for example, a five- to seven-membered monocyclic heterocyclic group which contains one nitrogen atom and contains 1 to 2 hetero atoms optionally selected from an oxygen atom(s), a nitrogen atom(s) and a sulfur atom(s) which may be oxidized (e.g. five- to seven-membered monocyclic aromatic heterocyclic group which contains one nitrogen atom and contains 1 to 2 hetero atoms optionally selected from an oxygen atom(s), a nitrogen atom(s) and a sulfur atom(s) which may be oxidized, and may be partially saturated or fully saturated, *etc*.). Most preferred is, for example, pyrrole, imidazole, triazole, pyrazole, thiazole, oxazole, pyridine, pyrimidine, dihydrotriazine, pyrazine or dihydropyrimidine ring.

Preferred as 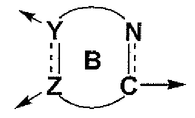 wherein the leftward arrow represents a binding position to ring A, the rightward arrow represents a binding position to cyano, is

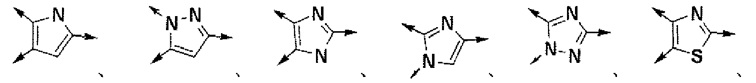

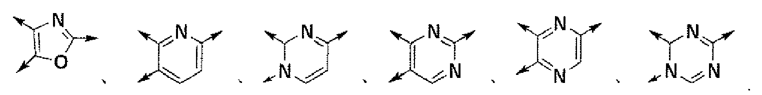

Preferred as 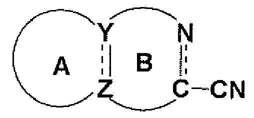 is concretely, for example, 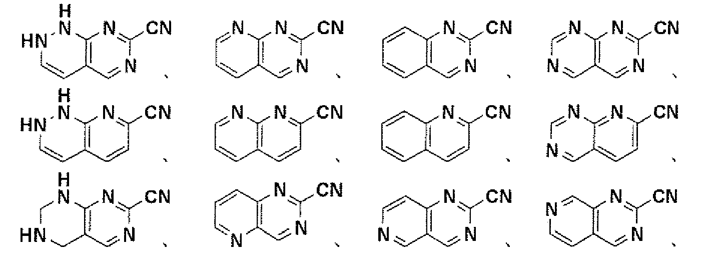 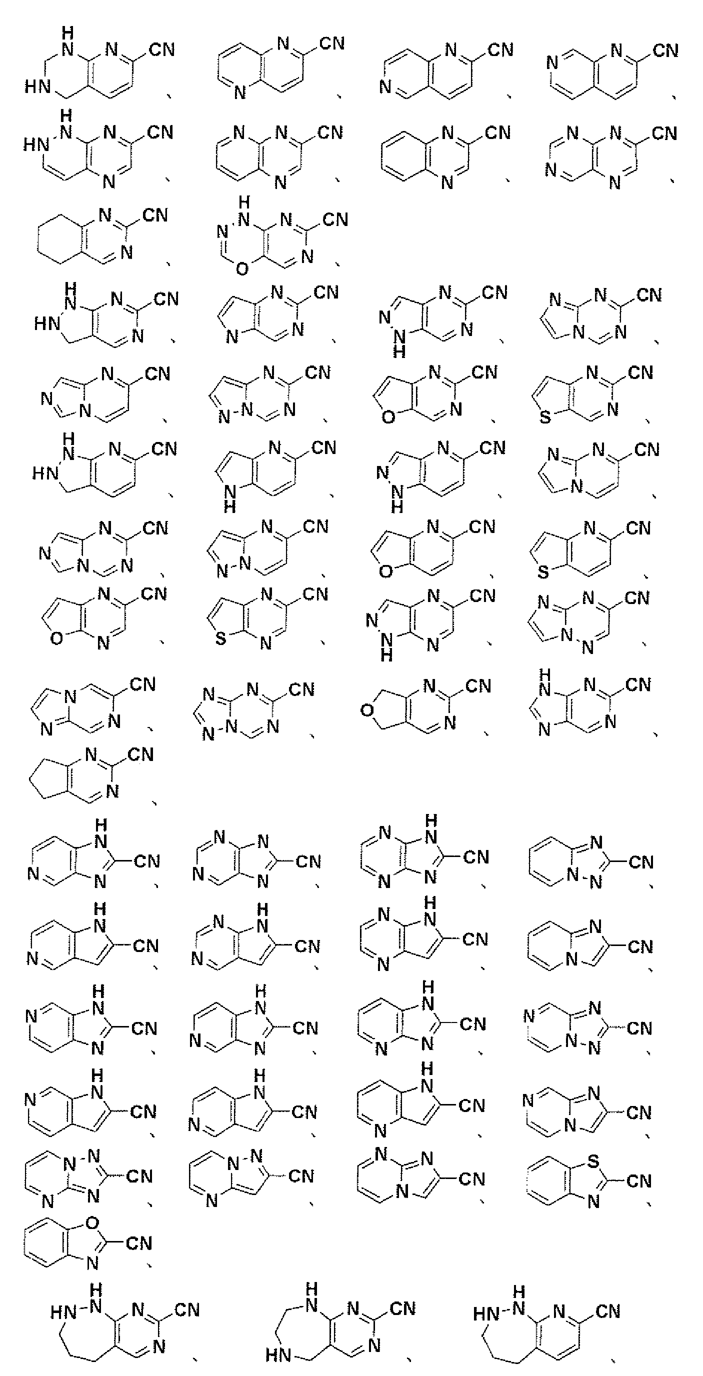 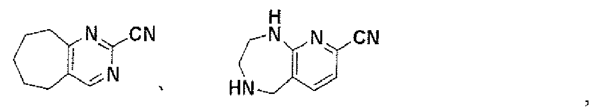 *etc.*

More preferred is 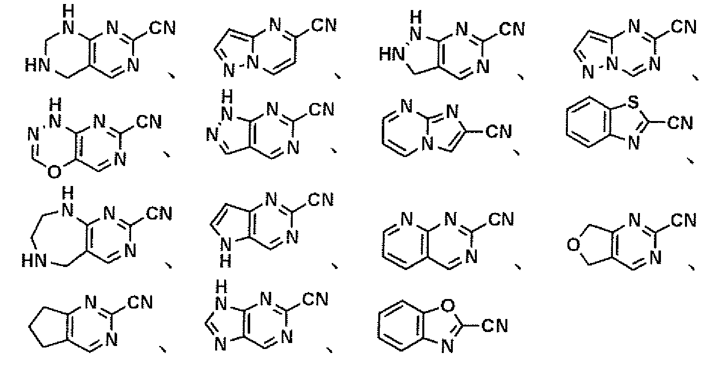 wherein, a hydrogen atom bound to a nitrogen atom may be replaced by ).

They can also be named as 5,6,7,8-tetrahydropyrimido[4,5-d]pyrimidine-2-carbonitrile, pyrazolo[1,5-a]pyrimidine-5-carbonitrile, 2,3-dihydro-1H-pyrazolo[3,4-d]pyrimidine-6-carbonitrile, pyrazolo[1,5-a][1,3,5]triazine-2-carbonitrile, 1H-pyrizxaido[4,5-e][1,3,4]oxadiazine-7-carbonitrile, 1H-pyrazolo[3,4-d]pyrimidine-6-carbonitrile, imidazo[1,2-a]pyrimidine-2-carbonitrile, 1,3-benzothiazole-2-carbonitrile, 6,7,8,9-tetrahydro-5H-pyrimido[4,5-e][1,4]diazepine-2-carbonitrile, 5H-pyrrolo[3,2-d]pyrimidine-2-carbonitrile, pyrido[2,3-d]pyrimidine-2-carbonitrile, 5,7-dihydrofuro[3,4-d]pyrimidine-2-carbonitrile, 6,7-dihydro-5H-cyclopenta[d]pyrimidine-2-carbonitrile, 9H-purine-2-carbonitrile, or 1,3-benzoxazole-2-carbonitrile.

Another preferred as 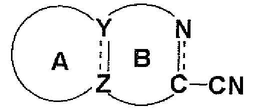 includes, for example, 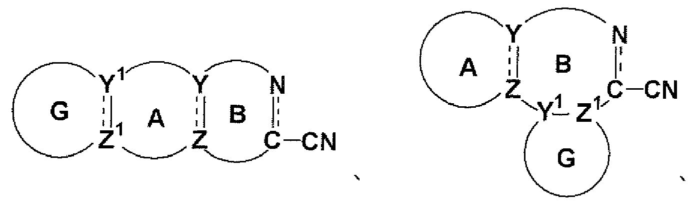 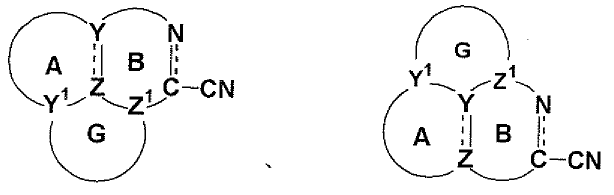 wherein Y¹ and Z¹ each is independently a carbon atom or a nitrogen atom, ringG is a ring formed by two Rs,
*etc.*

Preferred as 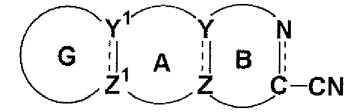 is concretely, for example, 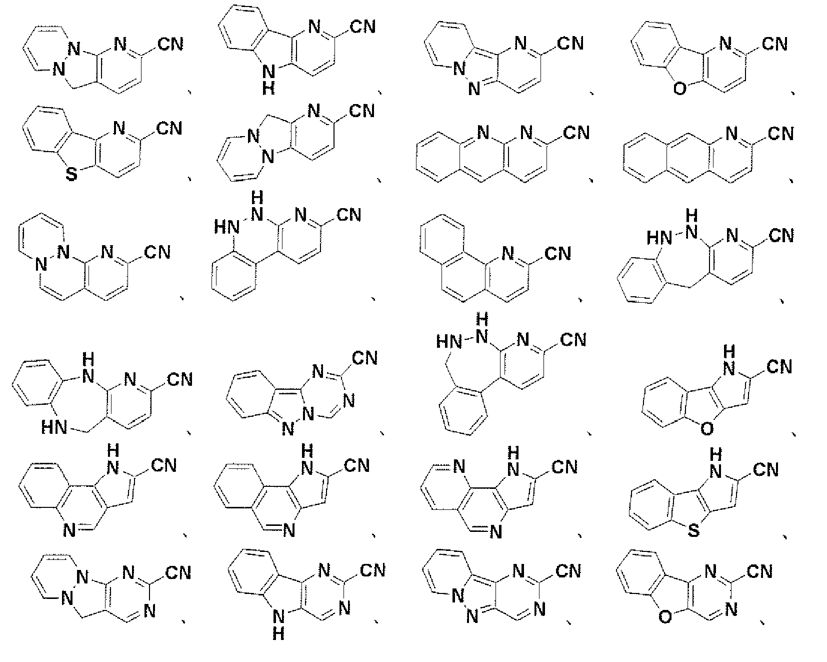 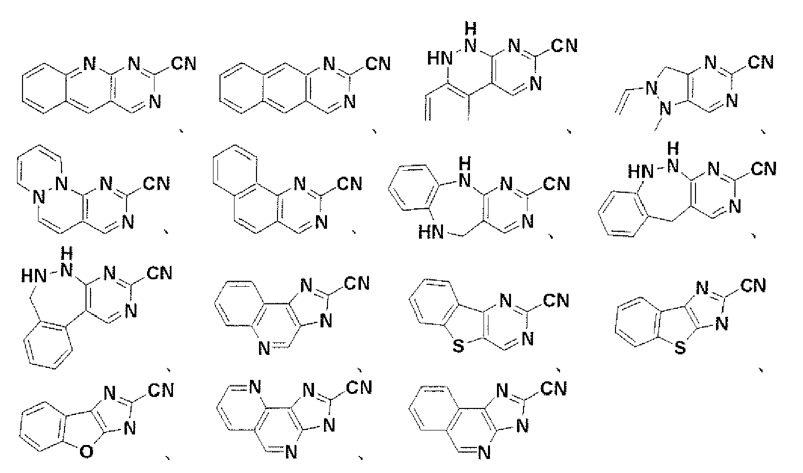 wherein, a hydrogen atom bound to a nitrogen atom may be replaced by R,
*etc.*

Preferred as 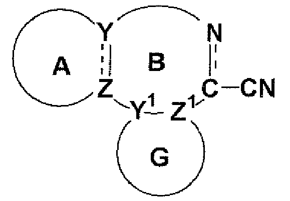 is concretely, for example, 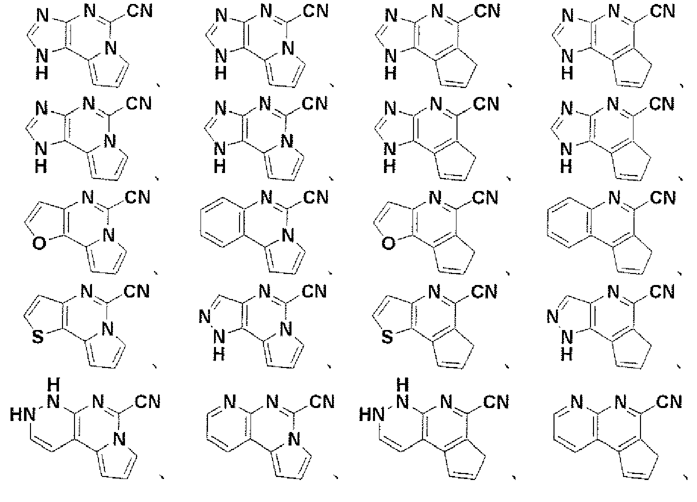 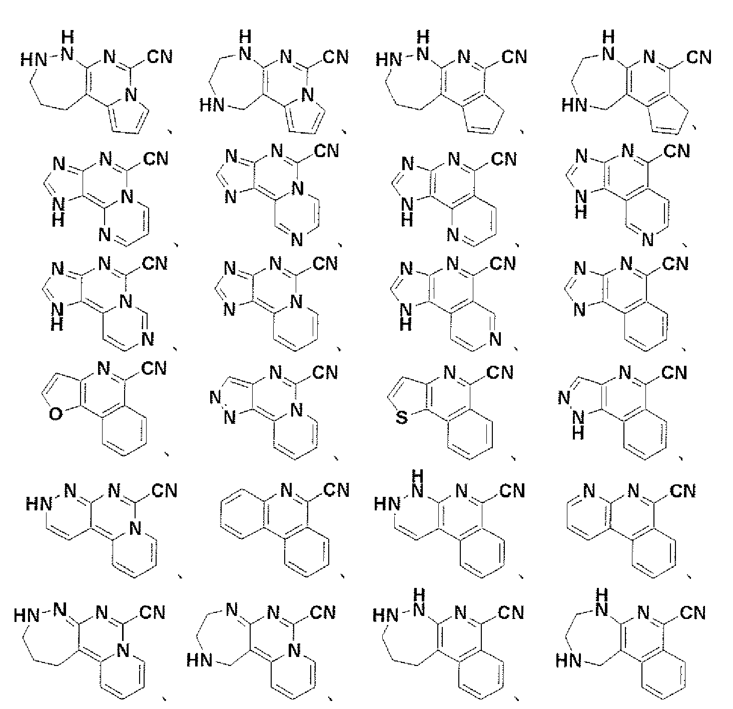 wherein, a hydrogen atom bound to a nitrogen atom may be replaced by R,
*etc.*

Preferred as 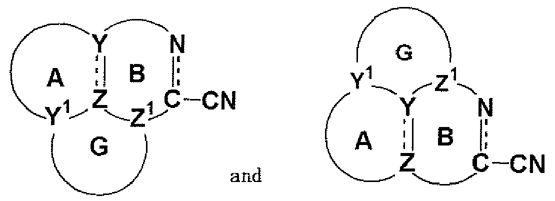 is concretely, for example, 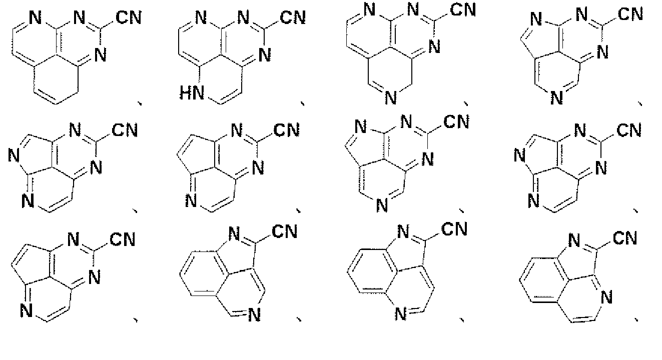 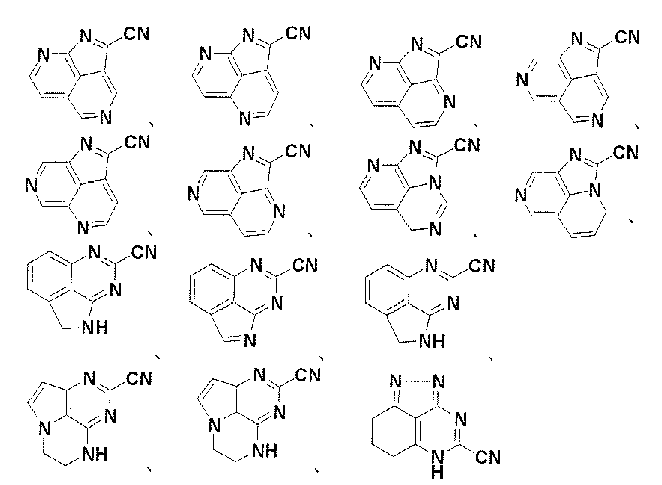 wherein, a hydrogen atom bound to a nitrogen atom may be replaced by R,
*etc.*

Preferred as a substituent(s) represented by R¹ is, for example, branched C1-8 alkyl. More preferably is, for example, isopropyl, 2-methylpropyl, tert-butyl, or 2,2-dimethylpropyl (neopentyl).

Preferred as a substituent represented by R is, for example, 1 to 5 substituent(s) selected from C1-8 alkyl which may be substituted by 1 to 5 R²⁰(s), C2-8 alkenyl which may be substituted by 1 to 5 R²⁰(s), C2-8 alkynyl which may be substituted by 1 to 5 R²⁰(s), C1-8 alkoxy which may be substituted by 1 to 5 R²⁰(s) (wherein C1-8 alkoxy includes, for example, methoxy, ethoxy, n-propoxy, isopropoxy, n-butoxy, isobutoxy, sec-butoxy, tert-butoxy, pentyloxy, hexyloxy, heptyloxy, octyloxy, *etc.*), C1-8 alkylthio which may be substituted by 1 to 5 R²⁰(s) (wherein C1-8 alkylthio includes, for example, methylthio, ethylthio, n-propylthio, isopropylthio, n-butylthio, isobutylthio, sec-butylthio, tert-butylthio, pentylthio, hexylthio, heptylthio, octylthio, *etc.*), carbocyclic group which may have a substituent(s) (phenyl, naphthyl, *etc.*), heterocyclic group which may have a substituent(s) (pyridyl, thienyl, furyl, *etc.*), -O-(carbocyclic group (phenyl, naphthyl, *etc.*)), -O-(heterocyclic group (pyridyl, thienyl, furyl, *etc.*)), hydroxy, mercapto, amino, NR²¹R²², carboxy, C1-6 alkoxycarbonyl, nitro, cyano, halogen, C1-6 acyl (e.g. formyl, ethanoyl, propanoyl, butanoyl, pentanoyl, hexanoyl, *etc.*), oxo,

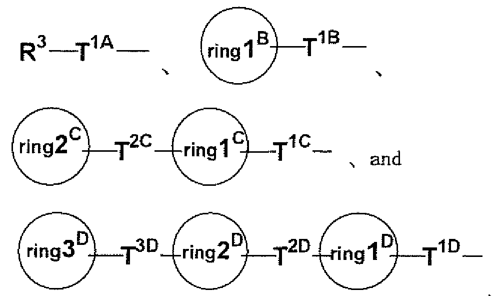

More preferably is, for example, 1 to 5 substituent(s) selected from methyl, 2-methylpropyl, isopropyl, 2,2-dimethylpropyl, ethenyl, methoxy, ethoxy, 2,2-dimethylpropoxy, oxo,

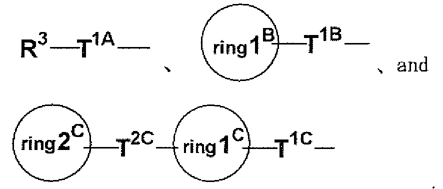

R²⁰ includes, for example, hydroxy, halogen, carbocyclic group which may have a substituent(s) (phenyl, naphthyl, *etc.*), heterocyclic group which may have a substituent(s) (pyridyl, thienyl, furyl, *etc.*), C1-6 alkoxy, -O-(carbocyclic group (phenyl, naphthyl, *etc.*)), carboxy, C1-6 alkoxycarbonyl, -CONR²¹R²², *etc.*

R²¹ or R²² includes, for example, a hydrogen atom, C1-6 alkyl, *etc.*

Preferred as T^{1A}, T^{1B}, T^{1C} or T^{1D} is, for example, a bond or a divalent C1-5 hydrocarbon group which may have a substituent(s), -O-(a divalent C1-5 hydrocarbon group which may have a substituent(s))-O-, -E³-E²-E¹- (wherein E¹ and E³ are each independently, a bond or a divalent C1-5 hydrocarbon group which may have a substituent(s), E² is -O-, -NR¹⁰-, -S-, -C(=O)-, -C(=O)NR¹⁰-, -NR¹⁰C(=O)-, -SO₂NR¹⁰-, -NR¹⁰SO₂-, -C(=O)O-, -OC(=O)-, or -NR¹⁰C(=O)NR¹¹- (wherein R¹⁰ and R¹¹ are each independently a hydrogen atom or hydrocarbon group which may have a substituent(s).). Preferred as E² is -C(=O)NR¹⁰-, or -NR¹⁰C(=O)-. Preferred as a divalent C1-5 hydrocarbon group represented by E¹ or E³ is C1-5 alkylene. Preferred as hydrocarbon group which may have a substituent(s) represented by R¹⁰ or R¹¹ is C1-8 alkyl.

Preferred as R³ is, for example, a hydrogen atom, C1-5 hydrocarbon group, a substituent containing a nitrogen atom which is basic. A substituent containing a nitrogen atom which is basic represented by R³ includes, for example, amino which may have a substituent(s) or nitrogen-containing heterocyclic group which may have a substituent(s). The "amino which may have a substituent(s)" has the same meaning as the above-described "(6) amino which may have a substituent(s)". "Nitrogen-containing heterocyclic group" has the same meaning as the above-described a heterocyclic group containing at least one nitrogen atom" represented by ring B. The "nitrogen-containing heterocyclic group" may have 1 to 5 substituent(s), and the substituent(s) of nitrogen-containing heterocyclic group has the same meaning as the above-described the substituent(s) in the "(2) carbocyclic group which may have a substituent(s)". Preferred as R³ is, for example, C1-8 alkyl, amino, dimethylamino, morpholin-4-yl, piperidin-4-yl, 4-methylpiperazin-1-yl.

Preferred as T^{2C}, T^{2D} or T^{3D} is, for example, a bond, or a C1-5 hydrocarbon group which may have a substituent(s).

Preferred as ring in the "ring which may have a substituent(s)" represented by ring^{1B}, ring^{1C}, ring ^{2C}, ring^{1D}, ring^{2D} or ring^{3D} is, for example, C3-10 mono-, or bicyclic carbocyclic group (e.g. C3-10 mono-, or bicyclic aromatic carbocyclic group, and partially saturated or fully saturated carbocyclic group, *etc.*), or three- to ten-membered mono-, or bicyclic heterocyclic group (e.g. three- to ten-membered mono-, or bicyclic aromatic heterocyclic group which contains 1 to 5 hetero atoms selected from an oxygen atom(s), a nitrogen atom(s) and a sulfur atom(s) which may be oxidized, and which may be partially saturated or fully saturated). More preferably is, for example, C3-10 monocyclic carbocyclic group (e.g. C3-10 monocyclic aromatic carbocyclic group, and partially saturated or fully saturated carbocyclic group, *etc.),* three- to ten-membered monocyclic heterocyclic group (e.g. three- to ten-membered monocyclic aromatic heterocyclic group which contains 1 to 5 hetero atoms selected from an oxygen atom(s), a nitrogen atom(s) and a sulfur atom(s) which may be oxidized, and which may be partially saturated or fully saturated, *etc.*), C5-10 bicyclic carbocyclic group (e.g. C5-10 bicyclic aromatic carbocyclic group, and partially saturated or fully saturated carbocyclic group, *etc.),* or five- to ten-membered bicyclic heterocyclic group (e.g. five- to ten-membered bicyclic aromatic heterocyclic group which contains 1 to 5 hetero atoms selected from an oxygen atom(s), a nitrogen atom(s) and a sulfur atom(s) which may be oxidized, and which may be partially saturated or fully saturated, *etc.*), More preferably is, for example, C5-7 monocyclic carbocyclic group (e.g. C5-7 monacyclic aromatic carbocyclic group, and partially saturated or fully saturated carbocyclic group, *etc.),* or five- to seven-membered monocyclic heterocyclic group (e.g. five- to seven-membered monocyclic aromatic heterocyclic group which contains 1 to 3 hetero atoms selected from an oxygen atom(s), a nitrogen atom(s) and a sulfur atom(s) which may be oxidized, and which may be partially saturated or fully saturated, *etc.).* Most preferably is, for example, cyclopropane, cyclobutane, cyclopentane, cyclohexane, cycloheptane, cyclopentene, cyclohexene, cycloheptene, cyclopentadiene, cyclohexadiene, cycloheptadiene, benzene, piperidine, piperazine, morpholine, pyridine, pyrimidine, pyrazine, tetrahydropyrimidine, dihydropyridazine, pyridazine, dihydropyrimidine, dihydropyrazine, dihydrotriazine, pyrazole, dihydropyrazole, pyrrole, imidazole, triazole, thiophene, furan, dihydrofuran, oxadiazine, tetrahydrodiazepine or diazepane ring.

Preferred as substituent in the "ring which may have a substituent(s)" represented by ring^{1B}, ring^{1C}, ring^{2C}, ring^{1D}, ring^{2D} or ring^{3D} is, for example, 1 to 3 substituent(s) selected from C1-8 alkyl which may be substituted by 1 to 5 R²⁰(s), C2-8 alkenyl which may be substituted by 1 to 5 R²⁰(s), C2-8 alkynyl which may be substituted by 1 to 5 R²⁰(s), C1-8 alkoxy which may be substituted by 1 to 5 R²⁰(s), C1-8 alkylthio which may be substituted by 1 to 5 R²⁰(s) carbocyclic group which may have a substituent(s) (phenyl, naphthyl, *etc.*), heterocyclic group which may have a substituent(s) (pyridyl, thienyl, furyl, *etc*.), -O-(carbocyclic group (phenyl, naphthyl, *etc.*)), -O-(heterocyclic group (pyridyl, thienyl, furyl, *etc.*)), hydroxy, mercapto, amino, NR²¹R²², carboxy, C1-6 alkoxycarbonyl, nitro, cyano, halogen, C1-6 acyl (e.g. formyl, ethanoyl, propanoyl, butanoyl, pentanoyl, hexanoyl, *etc*.) and oxo.

Among compounds of formula (I), a preferred is a compound of formula (I-1) 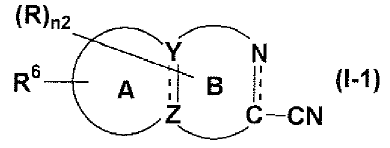 wherein R⁶ is 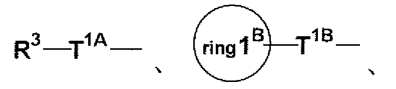 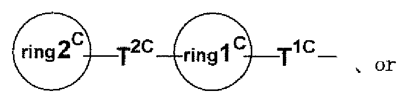 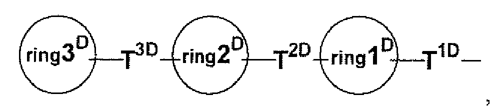 n2 is 0 or an integer of 1 to 9, other symbols have the same meanings as described above,
a salt thereof, a solvate thereof, or an N-oxide thereof, or a prodrug thereof.

Among compounds of formula (I-1), a preferred is a compound of formula (I-1-1) 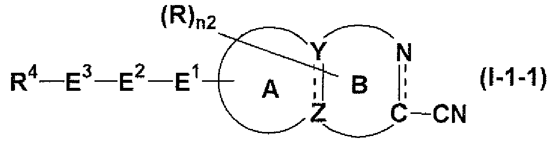 wherein R⁴ is 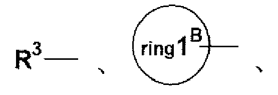 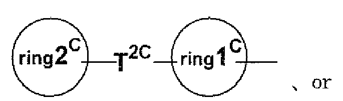 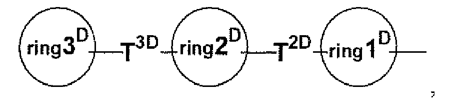 other symbols have the same meanings as described above, formula (I-1-2) 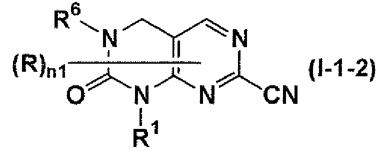 wherein all symbols have the same meanings as described above, or formula(I-1-3) 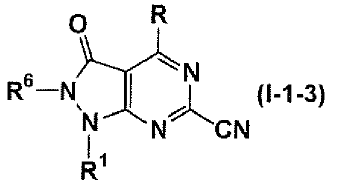 wherein all symbols have the same meanings as described above,
a salt thereof, a solvate thereof, or an N-oxide thereof, or a prodrug thereof.

Among compounds of formula (I-1-2), a preferred is a compound of formula (I-1-2a) 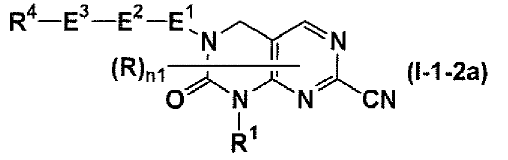 wherein all symbols have the same meanings as described above,
a salt thereof, a solvate thereof, or an N-oxide thereof, or a prodrug thereof.

Among compounds of formula (I-1-3), a preferred is a compound of formula (I-1-3a) 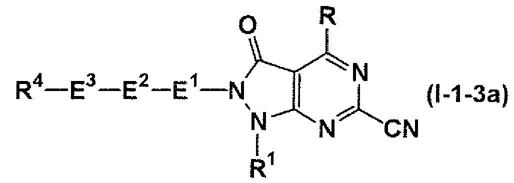 wherein all symbols have the same meanings as described above,
a salt thereof, a solvate thereof, or an N-oxide thereof, or a prodrug thereof

As a specific aspect, compounds listed hereinafter or described in Examples, *etc.* are included in this invention.
(1) 7-[(2,4-dioxo-1,3,8-triazaspiro[4.5]dec-8-yl)methyl]-8-neopentyl-5..oxo-4,5-dihydropyrrolo[2,3,4-de]quinazoline-2-carbonitrile,
(2) 7-[(2,4-dioxo-1,3,8-triazaspiro[4.5]dec-8-yl)methyl]-8-neopentylpyrrolo[2,3,4-de]quinazoline-2-carbonitrile,
(3) 7-[(2,4-dioxo-1,3,8-triazaspiro[4.5]dec-8-yl)methyl]-4-methyl-8-neopentyl-4,5-dihydropyrrolo[2,3,4-de]quinazoline-2-carbonitrile,
(4) 7-[(3-methyl-2,5-dioxo-1,4,9-triazaspiro[5.5]undec-9-yl)methyl]-8-neopentyl-5-oxo-4,5-dihydropyrrolo[2,3,4-de]quinazoline-2-carbonitrile,
(5) 7-[(3-methyl-2,5-dioxo-1,4,9-triazaspiro[5.5]undec-9-yl)methyl]-8-neopentylpyrrolo[2,3,4-de]quinazoline-2-carbonitrile,
(6) 4-methyl-7-[(3-methyl-2,5-dioxo-1,4,9-triazaspiro[5.5]undec-9-yl)methyl]-8-neopentyl-4,5-dihydropyrrolo[2,3,4-de]quinazoline-2-carbonitrile,
(7) 8-neopentyl-5-oxo-7-{[1-oxo-2-(pyridin-4-ylmethyl)-2,7-diazaspiro[4.5]dec-7-yl]methyl}-4,5-dihydropyrrolo[2,3,4-de]quinazoline-2-carbonitrile,
(8) 8-neopentyl-7-{[1-oxo-2-(pyridin-4-ylmethyl)-2,7-diazaspiro[4.5]dec-7-yl]methyl}pyrrolo[2,3,4-de]quinazoline-2-carbonitrile,
(9) 4-methyl-8-neopentyl-7-{[1-oxo-2-(pyridin-4-ylmethyl)-2,7-diazaspiro[4.5]dec-7-yl]methyl}-4,5-dihydropyrrolo[2,3,4-de]quinazoline-2-carbonitrile,
(10) 8-[(2,4-dioxo-1,3,8-triazaspiro[4.5]dec-8-yl)methyl]-4-methyl-9-neopentyl-5,6-dihydro-4H-pyrrolo[3,2,1-de]pteridine-2-carbonitrile,
(11) 4-methyl-8-[(3-methyl-2,5-dioxo-1,4,9-triazaspiro[5.5]undec-9-yl)methyl]-9-neopentyl-5,6-dihydro-4H-pyrrolo[3,2,1-de]pteridine-2-carbonitrile,
(12) 4-methyl-9-neopentyl-8-{[1-oxo-2-(pyridin-4-ylmethyl)-2,7-diazaspiro[4.5]dec-7-yl]methyl}-5,6-dihydro-4H-pyrrolo[3,2,1-de]pteridine-2-carbonitrile,
(13) N-[(2-cyano-4-methyl-9-neapentyl-5,6-dihydro-4H-pyrrolo[3,2,1-de]ptendin-8-yl)methyl]-4-[1-(2-methoxyethyl)piperidin-4-yl]benzamide,
(14) N-[(2-cyano-4-methyl-9-neopentyl-5,6-dihydro-4H-pyrralo[3,2,1-de]pteridin-8-yl)methyl]-4-[(4-methylpiperazin-1-yl)methyl]benzamide,
(15) N-[(2-cyano-4-methyl-9-neopentyl-5,6-dihydro-4H-pyrrola[3,2,1-de]pteridin-8-yl)methyl]-4-[2-(4-methylpiperazin-1-yl)-1,3-thiazol-4-yl]benzamide,
(16) 8-[(2,4-dioxo-1,3,8-triazaspiro[4.5]dec-8-yl)methyl]-4-methyl-9-neopentyl-6-oxo-5,6-dihydro-4H-pyrrolo[3,2,1-de]pteridine-2-carbonitrile,
(17) 4-methyl-8-[(3-methyl-2,5-dioxo-1,4,9-triazaspiro[5.5]undec-9-yl)methyl]-9-neopentyl-6-oxo-5,6-dihydro-4H-pyrrolo[3,2,1-de]pteridine-2-carbonitrile,
(18) 4-methyl-9-nenpentyl-6-oxo-8-{[1-oxo-2-(pyridin-4-ylmethyl)-2,7-diazaspiro[4.5]dec-7-yl]methyl}-5,6-dihydro-4H-pyrrolo[3,2,1-de]pteridine-2-carbonitrile,
(19) 1-{4-[1-(2-methoxyethyl)piperidin-4-yl]benzoyl}-2-neopentyl-1,2-dihydropyrazolo[3,4,5-de]qninazoiine-4-carbonitrile,
(20) 1-{4-[(4-tnethylpiperazin-1-yl)methyl]benzoyl}-2-neopentyl-1,2-dihydropyrazolo [3,4,5-de] quinazoline-4-carbonitrile,
(21) 1-{4-[2-(4-methylpiperazin-1-yl)-1,3-thiazol-4-yl]berazoyl}-2-neopentyl-1,2-dihydropyrazolo[3,4,5-de]quinazoline-4-carbonitrile,
(22) 1-{4-[1-(2-methoxyethyl)piperidin-4-yl]benzoyl}-2-neopentyl-1,2-dihydro-1,2,3,5,6-pentaazaacenaphthylene-4-carbonitrtle,
(23) 1-{4-[(4-methylpiperazin-1-yl)methyl]benzoyl}-2-neopentyl-1,2-dihydro-1,2,3,5,6-pentaazaacenaphthylene-4-carbonitrile,
(24) 1-{4-[2-(4-methylpiperazin-1-yl)-1,3-thiazol-4-yl]benzoyl}-2-neopentyl-1,2-dihydro-1,2,3,5,6-pentaazaacenaphthylene-4-carbonitrile,
(25) 2-[(2,4-dioxo-1,3,8-triazaspiro[4.5]dec-8-yl)methyl]-3-neopentyl-7,8-dihydro-6H-cyclopenta[e]pyrazolo[1,5-a]pyrimidine-5-carbanitrile,
(26) 2-[(3 -methyl-2,5-dioxo-1,4,9-triazaspiro[5.5]undec-9-yl)methyl]-3-neopentyl-7,8-dihydro-6H-cyclopenta[e]pyrazolo[1,5-a]pyrimidine-5-carbonitrile,
(27) 3-neopentyl-2-{[1-oxo-2-(pyridin-4-ylmethyl)-2,7-diazaspiro[4.5]dec-7-yl]methyl}-7,8-dihydro-6H-cyclopenta[e]pyrazolo[1,5-a]pyrimidine-5-carbonitrile,
(28) 2-[(2,4-dioxo-1,3,8-triazaspiro[4.5]dec-8-yl)methyl]-3-neopentyl-6,7,8,9-tetrahydropyrazolo[1,5-a]quinazoline-5-carbonitrile,
(29) 2-[(3-methyl-2,5-dioxo-1,4,9-triazaspiro[5.5]undec-9-yl)methyl]-3-neapentyl-6,7,8,9-tetrahydropyrazolo[1, 5-a]quinazoline-5-carbonitrile,
(30) 3-neopentyl-2-{[1-oxo-2-(pyridin-4-ylmethyl)-2,7-diazaspiro[4.5]dec-7-yl]methyl}-6,7,8,9-tetrahydropyrazolo[1,5-a]quinazaline-5-carbonitrile,
(31) 7-{4-[1-(2-methoxyethyl)piperidin-4-yl]benzoyl}-4-neopentyl-4,5,6,7-tetrahydro[1,4]diazepino[5,6,7-de]quinazaline-2-carbonitriie,
(32) 7-{4-[(4-methylpiperazin-1-yl)methyl]benzoyl}-4-neopentyl-4,5,6,7-tetrahydro[1,4]diazepino[5,6,7-de]quinazoline-2-carbonitrile,
(33) 7-{4-[2-(4-methylpiperazin-1-yl)-1,3-thiazol-4-yl]benzoyl}-4-neopentyl-4,5,6,7-tetrahydro[1,4]diazepino[5,6,7-de]quinazoline-2-carbonitrile,
(34) 4,10,10-trimethyl-7-{4-[(4-methylpiperazin-1-yl)methyl]benzyl}-8,9,10,11-tetrahydro-7H-[1,3,5]triazino[1',2' : 1,5]pyrazolo[3,4-b]azepine-2-carbonitrile,
(35) 2-({4-[(4-methylpiperazin-1-yl)carbonyl]piperidin-1-yl}methyl)-4-neopentylpyrazolo[1,5-a][1,3,5]triazine-7-carbonitrile,
(36) 2-[(2,4-dioxo-1,3,8-triazaspiro[4.5]dec-8-yl)methyl]-4-neopentylpyrazolo[1,5-a][1,3,5]triazine-7-carbonitrile,
(37) 2-[(3-methyl-2,5-dioxo-1,4,9-triazaspiro[5.5]undec-9-yl)methyl]-4-neopentylpyrazolo[1,5-a][1,3,5]triazine-7-carbonitrile,
(38) 4-neopentyl-2-{[1-oxo-2-(pyridin-4-ylmethyl)-2,7-diazaspiro[4.5]dec-7-yl]methyl}pyrazolo[1,5-a][1,3,5]triazine-7-carbonitrile,
(39) 2-[(2,4-dioxo-1,3,8-triazaspiro[4.5]dec-8-yl)carbonyl]-4-neopentylpyrazolo[1,5-a][1,3,5]triazine-7-carbonitrile,
(40) 2-[(3-methyl-2,5-dioxo-1,4,9-triazaspiro[5.5]undec-9-yl)carbonyl]-4-neopentylpyrazolo[1,5-a][ 1,3,5]triazine-7-carbonitrile,
(41) 4-neopentyl-2-{[1-oxo-2-(pyridin-4-ylmethyl)-2,7-diazaspiro[4.5]dec-7-yl]carbonyl}pyrazolo[1,5-a][1,3,5]triazine-7-carbonitrile,
(42) 6-{4-[(4-methylpiperazin-1-yl)methyl]benzyl}-8-neopentyl-7-oxo-5,6,7,8-tetrahydropyrimido[4,5-d]pyrimidine-2-carbonitrile,
(43) 6-{4-[1-(2-methoxyethyl)piperidin-4-yl]benzyl}-8-neopentyl-7-oxo-5,6,7,8-tetrahydropyrimido [4,5 -d]pyrimidine-2-carbonitrile,
(44) 6-{4-[2-(4-methylpiperazin-1-yl)-1,3-thiazol-4-yl]benzyl}-8-neopentyl-7-oxo-5,6,7,8-tetrahydropyrimido[4,5-d]pyrimidine-2-carbonitrile,
(45) 8-neopentyl-7-{[1-oxo-2-(pyridin-4-ylmethyl)-2,7-diazaspiro[4.5]dec-7-yl]methyl}quinazoline-2-carbonitrile,
(46) 7-[(3-methyl-2,5-dioxo-1,4,9-triazaspiro[5.5]undec-9-yl)methyl]-8-neopentylquinazoline-2-carbonitrile,
(47) 7-[(2,4-dioxo-1,3,8-triazaspiro[4.5]dec-8-yl)metllyl]-8-neopentylquinazoline-2-carbonitrile,
(48) 8-neopentyl-7-{[1-oxo-2-(pyridin-4-ylmethyl)-2,7-diazaspiro[4.5]dec-7-yl]mcthyl}pyrido[3,2-d]pyrimidine-2-carbonitrile,
(49) 7- [(3 -methyl-2,5-dioxo-1,4,9-triazaspiro[5.5]undec-9-yl)methyl]-8-neopentylpyrido[3,2-d]pyrimidine-2-carbonitrile,
(50) 7-[(2,4-dioxo-1,3,8-triazaspiro[4.5]dec-8-yl)methyl]-8-neopentylquinazoline-2-carbonitrile,
(51) 5-({3-[(4-methylpiperazin-1-yl)methyl]-2,5-dioxo-1,4,9-triazaspiro[5.5]nndec-9-yl}carbonyl)-7-neopentyl-5H-pyrrolo[3,2-d]pyrimidine-2-carbonitrile,
(52) 5-[(3-methyl-2,5-dioxo-1,4,9-triazaspiro[5.5]undec-9-yl)carbonyl]-7-neopentyl-5H-pyrrolo[3,2-d]pyrimldine-2-carbonitrile,
(53) 5-[(2,4-dioxo-1,3,8-triazaspiro[4.5]dec-8-yl)carbonyl]-7-neopentyl-5H-pyrrolo[3,2-d]pyrimidine-2-carbonitrile,
(54) 7-neopentyl-5-{[1-oxo-2-(pyridin-4-ylmethyl)-2,7-diazaspiro[4.5]dec-7-yl]carbonyl}-5H-pyrrolo[3,2-d]pyrimidine-2-carbonitrile,
(55) 5-{4-[1-(2-methoxyethyl)piperidin-4-yl]benzyl}-7-neopentyl-5H-pyrrolo[3,2-d]pyrimidine-2-carbonitrile,
(56) 5-{4-[(4-methylpiperazin-1-yl)methyl]benzyl}-7-neopentyl-5H-pyrrolo[3,2-d]pyrimidine-2-carbonitrile,
(57) 5-{4-[2-(4-methylpiperazin-1-yl)-1,3-thiazol-4-yl]benzyl}-7-neopentyl-5H-pyrrolo[3,2-d]pyrimidine-2-carbonitrile,
(58) N'-(2-cyanopyrazolo[1,5-a][1,3,5]triazin-4-yl)-4-{[1-(2-methoxyethyl)piperidin-4-yl]methyl}-N'-neopentylbenzohydrazide,
(59) N'-(2-cyanopyrazolo[1,5-a][1,3,5]triazin-4-yl)-4-[(4-methylpiperazin-1-yl)methyl]-N'-neopentylbenzohydrazide,
(60) N'-(2-cyanopyrazolo[1,5-a][1,3,5]triazin-4-yl)-4-[2-(4-methylpiperazin-1-yl)-1,3-thiazol-4-yl]-N'-neopentylbenxohydrazide,
(61) N'-(2-cyanopyrazolo[1,5-a][1,3,5]triazin-4-yl)-2-(2,4-dioxo-1,3,8-triazaspiro[4.5]dec-8-yl)-N'-neopentylacetohydrazide,
(62) N'-(2-cyanopyrazolo[1,5-a][1,3,5]triazin-4-yl)-2-(3-methyl-2,5-dioxo-1,4,9-triazaspiro[5.5]undec-9-yl)-N'-neopentylacetohydrazide,
(63) N'-(2-cyanopyrazolo[1,5-a][1,3,5]triazin-4-yl)-N'-neopentyl-2-[1-oxo-2-(pyridin-4-ylmethyl)-2,7-diazaspiro[4.5]dec-7-yl]acetohydrazide,
(64) 6-[(2,4-dioxo-1,3,8-triazaspiro[4,5]dec-8-yl)methyl]-7-neopentyl-6,7-dihydro-5H-cyclopenta[d]pyrimidine-2-carbonitrile,
(65) 6-[(2,4-dioxo-1,3,8-triazaspiro[4.5]dec-8-yl)methyl]-7-neopentyl-5H-cyclopenta[d]pyrimidine-2-carbonitrile,
(66) 7-[(2,4-dioxo-1,3,8-triazaspiro[4.5]dec-8-yl)methyl]-8-ncopentyl-5,6,7,8-tetrahydroquinazoline-2-carbonitrile,
(67) 7-[(2,4-dioxo-1,3,8-triazaspiro[4.5]dec-8-yl)methyl]-8-neopentyl-5,6-dihydroquinazoline-2-carbonitrile,
(68) 7,7-dimethyl-6-{4-[(4-methylpiperazin-1-yl)methyl]benzoyl}-6,7,8,9-tetrahydro-5H-pyrimido[5,4-c]azepine-2-carbonitrile,
(69) 3-neopentyl-7-(neopentylamino)pyrazolo[1,5-a]pyrimidine-5-carbonitrile,
(70) 7-[(2,4-dioxo-1,3,8-triazaspiro[4.5]dec-8-yl)methyl]-8-neopentylpyrazolo[1,5-a][1,3,5]triazine-2-carbonitrile,
(71) 7-[(3-methyl-2,5-dioxo-1,4,9-triazaspiro[5.5]undec-9-yl)methyl]-8-neopentylpyrazolo[1,5-a][1,3,5]triazine-2-carbonitrile,
(72) 7-({4-[(4-methylpiperazin-1-yl)carbonyl]piperidin-1-yl}methyl)-8-neopentylpyrazolo[1, 5-a][1,3,5]triazine-2-carbonitrile,
(73) 7-{4-[(4-methylpiperazin-1-yl)methyl]benzoyl}-8-neopentylpyrazolo[1,5-a][1,3,5]triazine-2-carbonitrile,
(74) 2-{4-[(4-methylpiperazin-1-yl)methyl]benzoyl}-3-neopentylpyrazolo[1,5-a]pyrimidine-5-carbonitrile,
(75) 2-({4-[(4-methylpiperazin-1-yl)carbonyl]piperidin-1-yl}methyl)-3-neopentylpyrazolo[1,5-a]pyrimidine-5-carbonitrile,
(76) 2-[(3-methyl-2,5-dioxo-1,4,9-triazaspiro[5.5]undec-9-yl)methyl]-3-neopentylpyraznlo[1,5-a]pyrimidine-5-carbonitrile,
(77) 2-{[2,4-dioxo-3-(pyridin-4-ylmethyl)-1,3,8-triazaspiro[4.5]dec-8-yl]methyl}-3-neopentylpyrazolo[1,5-a]pyrimidine-5-carbonitrile,
(78) 7-(tert-butylamino)-6-[4-(2-piperazin-1-yl-1,3-thiazol-4-yl)benzoyl]pyrazolo[1,5-a]pyrimidine-2-carbonitrile,
(79) 7-(tert-butylamino)-6-{4-[(4-methylpiperazin-1-yl)methyl]benzoyl}pyrazolo[1,5-a]pyrimidine-2-carbonitrile,
(80) 7-[2-(3-methyl-2,5-dioxo-1,4,9-triazaspiro[5.5]undec-9-yl)-2-oxoethyl]-6-(neopentylamino)-7H-purine-2-carbonitrile,
(81) 7-[2-(2,4-dioxo-1,3,8-triazaspiro[4.5]dec-8-yl)-2-oxoethyl]-6-(neopentylamino)-7H-purine-2-carbonitrile,
(82) 7-[[2-(3-methyl-2,5-dioxo-1,4,9-triazaspiro[5.5]undec-9-yl)ethyl](neopentyl)amino]pyrazolo[1,5-a]pyrimidine-5-carbonitrile,
(83) 7-[{2-[2,4-dioxo-3-(pyridin-4-ylmethyl)-1,3,8-triazaspiro[4.5]dec-8-yl]ethyl}(neopentyl)amino]pyrazolo[1,5-a]pyrimidine-5-carbonitrile,
(84) 7-{(2,2-dimethylpropyl)[2-(2,4-dioxo-1,3,8-triazaspiro[4.5]dec-8-yl)-2-oxoethyl]amino}pyrazolo[1,5-a]pyrimidine-5-carbonitrile,
(85) 2-methyl-3-[(3-methyl-2,5-dioxo-1,4,9-triazaspiro[5.5]undec-9-yl)methyl]-1-neopentyl-1,2-dihydropyrimido[4,5-c]pyridazine-7-carbonitrile,
(86) 2-[2-(2,4-dioxo-1,3,8-triazaspiro[4.5]dec-8-yl)-2-oxoethyl]-1-neopentyl-1,2-dihydrapyrimido[4,5-c]pyridazine-7-carbonitrile,
(87) 2-[2-(3-methyl-2,5-dioxo-1,4,9-triazaspiro[5,5]undec-9-yl)-2-oxoethyl]-1-neopentyl-1,2-dihydropyrimido[4,5-c]pyridazine-7-carbonitrile,
(88) 6-{4-[(4-methylpiperazin-1-yl)methyl]benzoyl}-9-neopentyl-6,7,8,9-tetrahydro-5H-pyrimido[4,5-e][1,4]diazepine-2-carbonitrile,
(89) 6-{4-[1-(2-methoxyethyl)piperidin-4-yl]benzoyl}-9-neopentyl-6,7,8,9-tetrahydro-5H-pyrimido [4, 5-e] [1,4] diazep ine-2-carbonitrile,
(90) 6-[2-(2,4-dioxo-1,3,8-triazaspiro[4,5]dec-8-yl)-2-oxoethyl]-9-neopentyl-6,7,8,9-tetrahydro-5H-pyrimido[4,5-e] [1,4]diazepine-2-carbonitrile,
(91) 6-{4-[2-(4-methylpiperazin-1-yl)-1,3-thiazol-4-yl]benzoyl}-9-neopentyl-6,7,8,9-tetrahydro-5H-pyrimido[4,5-e][1,4]diazepine-2-carbonitrile,
(92) 2-methyl-1-neopentyl-3-{[1-oxo-2-(pyridin-4-ylmethyl)-2,7-diazaspiro[4.5]dec-7-yl]methyl}-1,2-dihydropyrimido[4,5-c]pyridazine-7-carbonitrile,
(93) 6-[2-(3-methyl-2,5-dioxo-1,4,9-triazaspiro[5.5]ondec-9-yl)-2-axoethyl]-5-neopentyl-6,7-dihydro-5H-pyrimido[4,5-d][2,3]benzodiazepine-3-carbonitrile,
(94) 6-[2-(2,4-dioxo-1,3,8-triazaspiro[4.5]dec-8-yl)ethyl]-5-neopentyl-6,7-dihydra-5H-pyrimida[4,5-d][2,3]benzodiazepine-3-carbanitrile,
(95) 10-{2-[1-methyl-2,5-dioxo-3-(pyridin-4-ylmethyl)-1,4,9-triazaspiro[5.5]undec-9-yl]-2-oxoethyl}-11-neopentyl-10,11-dihydro-5H-pyrimido[4,5-c][1,2]benzodiazepine-2-carbonitrile,
(96) 10-[2-(2,4-dioxo-1,3,8-triazaspiro[4.5]dec-8-yl)-2-oxoethyl]-11-neopentyl-10,11-dihydro-5H-pyrimido[4,5-c][1,2]benzodiazepine-2-carbonitrile,
(97) 2-[2-(3-methyl-2,5-dioxo-1,4,9-triazaspiro[5.5]undec-9-yl)-2-oxoethyl]-1-neopentyl-2,3-dihydro-1H-1,2,6,7,9-pentaazaphenalene-8-carbonitrile,
(98) 1-neopentyl-2-{2-oxo-2-[1-oxo-2-(pyridin-4-ylmethyl)-2,8-diazaspiro[4.5]dec-8-yl]ethyl}-2,3-dihydro-1H-1,2,6,7,9-pentaazaphenalene-8-carbonitrile,
(99) 1-neopentyl-2-{2-oxo-2-[1-oxo-2-(pyridin-4-ylmethyl)-2,7-diazaspiro[4.5]dec-7-yl]ethyl}-2,3-dihydro-1H-1,2,6,7,9-pentaazaphenalene-8-carbonitrile,
(100) 1-neopentyl-2-{2-oxo-2-[1-oxo-2-(pyridin-4-ylmethyl)-2,7-diazaspiro[4.5]dec-7-yl]ethyl}-2,3-dihydro-1H-pyridazino[3,4,5-de]quinazoline-8-carbonitrile,
(101) 2-[2-(1,3-dimethyl-2,5-dioxo-1,4,9-trazaspiro[5.5]undec-9-yl)-2-oxoethyl]-1-neopentyl-2,3-dihydro-1H-pyridazino[3,4,5-de]quinazoline-8-carbonitrile,
(102) 3-methyl-2-[(3-methyl-2,5-dioxo-1,4,9-triazaspiro[5.5]undec-9-yl)methyl]-4-neopentyl-4,7,8,9-tetrahydro-3H-cyclopenta[4,5]pyrido[2,3-c]pyridazine-6-carbonitrile,
(103) 2-[(2,4-dioxo-1,3,8-triazaspiro[4.5]dec-8-yl)methyl]-3-methyl-4-neopentyl-4,7,8,9-tetrahydro-3H-cyc1openta[4,5]pyrido[2,3-c]pyndazine-6-carbonitrile,
(104) 3-{2-[2,4-dioxo-3-(pyridin-4-ylmethyl)-1,3,7-triazaspiro[4.5]dec-7-yl]-2-oxoethyl}-4-neopentyl-4,7,8,9-tetrahydro-3H-cyclopenta[4,5]pyrido[2,3-c]pyridazine-6-carbonitrile,
(105) 3-[2-(3-methyl-2,4-dioxo-1,3,8-triazaspiro[4.5]dec-8-yl)-2-oxoethyl]-4-neopentyl-4,7,8,9-tetrahydro-3H-cyclopenta[4,5]pyrido[2,3-c]pyridazine-6-carbonitrile,
(106) 2-{4-[1-(2-methoxyethyl)piperidin-4-yl]benzoyl}-5-neopentyl-2,3,4,5-tetrahydro-1H-[1,4]diazepino[5,b-c]isoquinoline-7-carbonitrile,
(107) 2-{4-[2-(4-methylpiperazin-1-yl)-1,3-thiazol-4-yl]benzoyl}-5-neopentyl-2,3,4,5-tetrahydro-1H-[1,4]diazepino[5,6-c]isoquinoline-7-carbonitrile,
(108) 2-[(4-methylphenyl)sulfonyl]-5-neopentyl-2,3,4,5-tetrahydro-1H-[1,4]diazepino[5,6-c]isoquinoline-7-carbonitrile,
(109) 2-({3-[(4-methylpiperazin-1-yl)methyl]-2,5-dioxo-1,4,9-triazaspiro[5.5]undec-9-yl}carbonyl)-5-neopentyl-2,3,4,5-tetrahydro-1H-[1,4]diazepino[5,6-c]isoquinaline-7-carbonitrile,
(110) 2-{[2,4-dioxo-3-(pyridin-4-ylmethyl)-1,3,8-triazaspiro[4.5]dec-5-yl]carbonyl}-5-neopentyl-2,3,4,5-tetrahydro-1H-[1,4]diazepino[5,6-c]isoquinoline-7-carbonitrile,
(111) 2-[(3-methyl-2,5-dioxo-1,4,9-triazaspiro[5.5]undec-9-yl)acetyl]-5-neopentyl-2,3,4,5-tetrahydro-1H-[1,4]diazepino[5,6-c]isoquinoline-7-carbonitrile,
(112) 2-[(2-methyl-1-oxo-2,7-diazaspiro[4.5]dec-7-yl)acetyl]-5-neopentyl-2,3,4,5-tetrahydro-1H-[1,4]diazepino[5,6-c]isoquinoline-7-carbonitrile,
(113) 2-[2-(1,3-dimethyl-2,5-dioxo-1,4,9-triazaspiro[5.5]undec-9-yl)-2-oxoethyl]-5-neopentyl-2,3,4,5-tetrahydro-1H-[1,4]diazepino[5,6-c]isoquinoline-7-carbonitrile,
(114) 5-neopentyl-2-{2-oxo-2-[1-oxo-2-(pyridin-4-ylmethyl)-2,7-diazaspira[4.5]dec-7-yl]ethyl}-2,3,4,5-tetrahydro-1H-[1,4]diazepino[5,6-c]isoquinoline-7-carbonitrile,
(115) 2-[2-(2,4-dioxo-1,3,8-triazaspiro[4.5]dec-8-yl)-2-axoethyl]-5-neopentyl-2,3,4,5-tetrahydro-1H-[1,4]diazepino[5,6-c]isoquinoline-7-carbonitrile,
(116) 4-(2-{4-[1-(2-methoxyethyl)piperidin-4-yl]benzoyl}-4,4-dimethylpyrazolidin-1-yl)quinazoline-2-carbonitrile,
(117) 4-{4,4-dimethyl-2-[2-(3-methyl-2,5-dioxo-1,4,9-triazaspiro[5.5]undec-9-yl)-2-oxoethyl]pyrazolidin-1-yl}quinazoline-2-carbonitrile,
(118) 4-{2-[2-(2,4-dioxo-1,3,8-triazaspiro[4,5]dec-8-yl)-2-oxoethyl]-4,4-dimethylpyrazolidin-1-yl}quinazoline-2-carbonitrile,
(119) 2-{4-[1-(2-methoxyethyl)piperidin-4-yl]benzoyl}-1-neopentyl-2,3-dihydro-1H-pyrazolo[3,4-d]pyrimidine-6-carbonitrile,
(120) 2-{4-[(4-methylpiperazin-1-yl)methyl]benzoyl}-1-neopentyl-2,3-dihydro-1H-pyrazolo[3,4-d]pyrimidine-6-carbonitrile,
(121) 3-{[4-(dimethylamino)benzyl]oxy}-1-neopentyl-1H-pyrazolo[3,4-d]pyrimidine-6-carbonitrile,
(122) 2-[2-(3-methyl-2,5-dioxo-1,4,9-triazaspiro[5.5]undec-9-yl)-2-oxoethyl]-1-neopentyl-3-oxo-2,3-dihydro-1H-pyrazolo[3,4-d]pyrimidine-6-carbonitrile,
(123) 2-{2-[2,4-dioxo-3-(pyridin-4-ylmethyl)-1,3,8-triazaspiro[4.5]dec-8-yl]-2-oxoethyl}-1-neopentyl-3-oxo-2,3-dihydro-1H-pyrazolo[3,4-d]pyrimidine-6-carbonitrile,
(124) 2-(2- {4-[(4-methylpiperazin-1-yl)carbonyl]piperidin-1-yl}-2-oxoethyl)-1-neopentyl-3-oxo-2,3-dihydro-1H-pyrazolo[3,4-d]pyrimidine-6-carbonitrile,
(125) 2-[2-(3-methyl-2,5-dioxo-1,4,9-triazaspiro[5.5]undec-9-yl)-2-oxoethyl}-1-neopentyl-1,2-dihydropyrazina[2,3-c]pyridazine-7-carbonitrile,
(126) 2-{4-[1-(2-methoxyethyl)piperidin-4-yl]benzoyl}-1-neopentyl-1,2-dihydropyrazino[2,3-c]pyridazine-7-carbonitrile,
(127) 2-{4-[2-(4-methylpiperazin-1-yl)-1,3-thiazol-4-yl]benzoyl}-1-neopentyl-1,2-dihydropyrazino[2,3-c]pyridazine-7-carbonitrile,
(128) 2-[4-(4-methylpiperazin-1-yl)benzyl]-1-neopentyl-1,2-dihydropyrazino[2,3-c]pyridazine-7-carbanitrile,
(129) N-[(1-cyano-7,7-dimethyl-7,8-dihydro-6H-pyrido[1,2-c]pyrimidin-4-yl)methyl]-4-[(4-methylpiperazin-1-yl)methyl]benzamide,
(130) N-[(6-cyano-3,3-dimethyl-3,4-dihydro-2H-pyrimido[1,6-a]pyrimidin-9-yl)methyl]-4-[(4-methylpiperazin-1-yl)methyl]benzamide,
(131) 3,3-dimethyl-9-[(3-methyl-2,5-dioxo-1,4,9-triazaspiro[5.5]undec-9-yl)carbonyl]-3,4-dihydro-2H-pyrimido[1,6-a]pyrimidine-6-carbonitrile,
(132) 6-{4-[1-(2-methoxyethyl)piperidin-4-yl]benzoyl}-7,7-dimethyl-5,6,7,8-tetrahydropyrimido[5,4-f][1,4]oxazepine-2-carbonitrile,
(133) 6-{4-[1-(2-methoxyethyl)piperidin-4-yl]benzoyl}-7,7-dimethyl-6,7,8,9-tetrahydro-5H-pyrimido[4,5-e][1,4]diazepine-2-carbonitrile,
(134) 6-[(2,4-dioxo-1,3,8-triazaspiro[4,5]dec-8-yl)carbonyl]-7,7,9-timethyl-6,7,8,9-tetrahydro-5H-pyrimido[4,5-e][1,4]diazepine-2-carbonitrile.

In the present invention, isomers are included unless specified. For example, alkyl, alkenyl, alkynyl, alkoxy, alkylthio, alkylene, alkenylene, alkynylene, alkylidene, and alkenylidene include straight chain and branched ones. Furthermore, the present invention includes isomers in double bond, ring, fused ring (E, Z, cis, trans), isomers by the presence of asymmetric carbon *etc.* (R-, S-form, α-, β-configuration, enantiomer, diastereomer), optical isomers having optical rotation (D, L, d, l), polars by chromatography separation (more polar compound, less polar compound), equilibrium compound, rotamer, a compound of arbitrary ratios of those and racemic mixture.

An optically active compound in the present invention includes not only 100% optically pure compound and may include (an)other optical isomer(s) less than 50%.

The salt of the compound of formula (I) includes all of the salt which are pharmaceutically acceptable. With regard to the pharmaceutically acceptable salts, those which are low-toxic and soluble in water are preferred. Examples of appropriate salts are salt with alkaline metal (such as potassium, sodium and lithium), salt with alkaline earth metal (such as calcium and magnesium), ammonium salt (such as tetramethylammonium salt and tetrabutylammonium salt), salt with organic amine (such as triethylamine, methylamine, ethylamine, dimethylamine, cyclopentylamine, benzylamine, phenethylamine, piperidine, monoethanolamine, diethanolamine, tris(hydroxymethyl) methylamine, lysine, arginine and N-methyl-D-glucamine) and acid addition salt [such as inorganic acid salt (e.g., hydrochloride, hydrobromide, hydroiodide, sulfate, phosphate and nitrate, *etc.)* and organic acid salt (e.g., formate, acetate, propionate, trifluoroacetate, lactate, tartrate, oxalate, malonate, succinate, fumarate, malate, maleate, benzoate, citrate, methanesulfonate, ethanesulfonate, benzenesulfonate, toluenesulfonate, isethionate, glucuronate, gluconate, aspartate, and glutamate, *etc.*), *etc.*].

An N-oxide of a compound of formula (I) means a compound of formula (I) which nitrogen is oxidized. An N-oxide of a compound of formula (I) may also be salt of alkaline (earth) metal, ammonium, organic amine, or acid addition salt.

The salt of a compound of formula (I) includes a quaternary ammonium salt thereof. A quaternary ammonium salt means a salt of a compound of formula (I) which nitrogen is quaternarized by proper group, such as alkyl optionally substituted by substituent(s) (C1-8 alkyl optionally substituted by phenyl, *etc.*).

The proper solvate of the compound of formula (I) includes, for example, hydarate, alcoholate (ethanolate, *etc.), etc.* The solvate is preferably non-toxic and water-soluble. The solvate of the compound of formula (I) also includes solvates of the above-mentioned alkaline (earth) metal salt thereof, (quaternary) ammonium salt thereof, organic amine salt thereof, acid addition salt thereof, and N-oxide thereof

The compounds of formula(I) can be converted to salts thereof, N-oxide thereof, or solvate thereof by known methods.

The prodrugs of the compound of formula (I) mean the compounds converted into the compound of formula (I) by the reactions of enzymes, gastric acid and the like in an organism. As the prodrugs of the compound of formula (I), when the compound of formula (I) has amino group, the amino group of the compound is acylated, alkylated, or phosphorylated, (e.g. the amino group of the compound of formula (I) is eicosanoylated, alanylated, pentylaminocarbonylated, (5-methyl-2-oxo-1,3-dioxolene-4-yl)methoxycarbonylated, tetrahydrofuranylated, pyrrolidylmethylated, pivaloyloxymethylated, acetoxymethylated, tert-butylated and the like); when the compound of formula (I) has hydroxy group, the hydroxy group of the compound is acylated, alkylated, phosphorylated, borated (e.g. the hydroxy group of the compound of formula (I) is acetylated, palmitoylated, propanoylated, pivaloylated, succinylated, fumarylated, alanylated, dimethylaminomethylcarbonylated and the like); when the compound of formula (I) has carboxy group, the carboxy group of the compounds are ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolene-4-yl)methylesterified, cyclohexyloxycarbonylethylesterified, methylamidated and the like); and the like. These compounds can be manufactured by the conventional methods per se. In addition, the prodrugs of the compounds of formula (I) may be solvates. The prodrugs of the compound of formula (I) may be those ones which are converted to a compound of formula (I) in physiological conditions as described in Molecular Design, as Vol.7 of Development of pharmaceutical drugs, 1990. 163-198, Hirokawa Publishing.

### Methods for the preparation of the compound of the present invention:

The compound of the present invention represented by formula (I) can be prepared by methods which properly improved and combined known methods, such as methods described in Comprehensive Organic Transformations : A Guide to Functional Group Preparations, 2nd Edition (Richard C. Larock, John Wiley & Sons Inc, 1999), methods described below, or methods described in Examples. In each method described below, a starting material can be used as a salt thereof. An example of the salt includes a pharmaceutically acceptable salt of the compound of formula (I) described above.

### [1] A compound represented by formula (I) can be prepared by cyanation of a compound represented by formula (II)

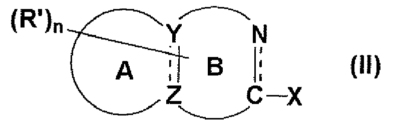

wherein X is a leaving group, for example, halogen, mesyloxy, tosyloxy, *etc.,* R' has the same meaning as R. With proviso that, carboxy group, hydroxy group, amino group or mercapto group in R' may be protected, if necessary. Other symbols have the same meanings as described above,
and if necessary, followed by removal of the protecting group.

The cyanation is well known. For example, it may be carried out by reacting a compound represented by formula (II) with cyanation reagent (e.g. potassium cyanide, sodium cyanide, tetrabutylammonium cyanide, tetraethylammonium cyanide, *etc.)* in an organic solvent (e.g. dimethylsulfoxide, dimethylformamide, dioxane or mixed solvent thereof and water), in the presence of tertiary amine (e.g. 1,4-diazabicyclo[2.2.2]octane (DABCO), trimethylamine, dimethylaminopyridine, *etc.*) at 0 to 150°C.

The reaction for removing the protective group for carboxy, hydroxy, amino or mercapto is known and its examples are as follows.
(1) a deprotection reaction by hydrolyzing reaction with an alkali;
(2) a deprotection reaction under an acidic condition;
(3) a deprotection reaction by hydrogenolysis;
(4) a deprotection reaction of silyl;
(5) a deprotection reaction using a metal; and
(6) a deprotection reaction using metal complex.

Those methods will be specifically illustrated as follows.
(1) A deprotection reaction using an alkali is carried out, for example, at 0 to 40°C using a hydroxide of alkaline metal (e.g. sodium hydroxide, potassium hydroxide and lithium hydroxide, *etc.*), a hydroxide of alkaline earth metal (e.g. barium hydroxide and calcium hydroxide, *etc.),* a carbonate (e.g. sodium carbonate and potassium carbonate, *etc.*), or an aqueous solution thereof or a mixture thereof in an organic solvent (e.g. methanol, tetrahydrofuran and dioxane *etc.*).
(2) A deprotection reaction under an acidic condition is carried out, for example, at 0 to 100°C in an organic acid (e.g. acetic acid, trifluoroacetic acid, methanesulfonic acid or p-toluenesulfonic acid, *etc.),* an inorganic acid (e.g. hydrochloric acid and sulfuric acid, *etc.*) or a mixture thereof (e.g. hydrogen bromide/acetic acid) in an organic solvent (e.g. dichloromethane, chloroform, dioxane, ethyl acetate and anisole *etc.*) in the presence or absence of 2,2,2-trifluoroethanol.
(3) A deprotection reaction by hydrogenolysis is carried out, for example, at 0 to 200°C under a hydrogen atmosphere of ordinary pressure or high pressure or in the presence of ammonium formate in the presence of a catalyst (e.g. palladium-carbon, palladium black, palladium hydroxide-carbon, platinum oxide and Raney nickel, *etc.)* in a solvent [e.g. an ether (e.g. tetrahydrofuran, dioxane, dimethoxyethane and diethyl ether, *etc.),* an alcohol (e.g. methanol and ethanol, *etc.*), a benzene (e.g. benzene and toluene, *etc.*), a ketone (e.g. acetone and methyl ethyl ketone, *etc.*), a nitrile (e.g. acetonitrile, *etc.*), an amide (e.g. dimethylformamide, *etc.*), water, ethyl acetate, acetic acid or a mixed solvent comprising two or more thereof].
(4) A deprotection reaction of silyl is carried out, for example, at 0 to 40°C using tetrabutylammonium fluoride in an organic solvent miscible with water (e.g. tetrahydrofuran and acetonitrile *etc.*).
(5) A deprotection reaction using metal is carried out, for example, at 0 to 40°C with or without ultrasonic wave in the presence of powdery zinc in an acidic solvent (e.g. acetic acid, a buffer of pH 4.2 to 7.2 and a mixed solution of a solution thereof with an organic solvent such as tetrahydrofuran, *etc*.).
(6) A deprotection reaction using a metal complex is carried out, for example, at 0 to 40°C using a metal complex [e.g. tetrakistriphenylphosphine palladium (0), bis(triphenylphosphine) palladium (II) dichloride, palladium (II) acetate and tris(triphenylphosphine) rhodium (I) chloride, *etc*.] in the presence or absence of a phosphine agent (e.g. triphenyl phosphine, *etc.*) in the presence of a trap reagent (e.g. tributyltin hydride, triethylsilane, dimedone, morpholine, diethylamine, pyrrolidine, *etc.*), an organic acid (e.g. acetic acid, formic acid, 2-ethylhexanoic acid, *etc.*) and/or an organic acid salt (e.g. sodium 2-ethylhexanoate, potassium 2-ethylhexanoate, *etc.)* in an organic solvent (e.g. dichloromethane, dimethylformamide, tetrahydrofuran, ethyl acetate, acetonitrile, dioxane, ethanol, *etc.*), water or a mixed solvent thereof

Apart from the above, the deprotection may also be effected, for example, according to the methods described in T.W. Greene, Protective Groups in Organic Synthesis, Wiley, New York, 1999.

The protective group of carboxyl includes, for example, methyl, ethyl, allyl, tert-butyl, trichloroethyl, benzyl (Bn) or phenacyl, p-methoxybenzyl, trityl, 2-chlorotrityl or polymer-supported group bound thereby, *etc.*

The protecting group of hydroxy includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), tertbutyldimethylsilyl (TBDMS), tert-butyldiphenylsilyl (TBDPS), acetyl (Ac), pivaloyl, benzoyl, benzyl (Bn), p-methoxybenzyl, allyloxycarbonyl (Alloc), and 2,2,2-trichloroethoxycarbonyl (Troc), *etc.*

The protecting group of amino includes, for example, benzyloxycarbonyl, tert-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl (Fmoc), benzyl (Bn), p-methoxybenzyl, benzyloxymethyl (BOM) or 2-(trimethylsilyl)ethoxymethyl (SEM), *etc.*

The protective group of mercapto includes, for example, benzyl, methoxybenzyl, methoxymethyl (MOM), 2-tetrahydropyranyl (THP), diphenylmethyl and acetyl (Ac), *etc.*

With regard to the protective group for carboxy, hydroxy, amino and mercapto, there is no particular limitation to the above ones so far as it is a group which is able to be easily and selectively removed. For example, a deprotection reaction may be carried out by a method described in *"*T. W. Greene, Protective Groups in Organic Synthesis, John Wiley & Sons Inc, 1999".

As persons skilled in the art can easily understand that the aimed compound of the present invention is able to be easily produced by using appropriate ones among those deprotection reactions.

### [2] Among a compound represented by formula (I), a compound represented by formula (IA)

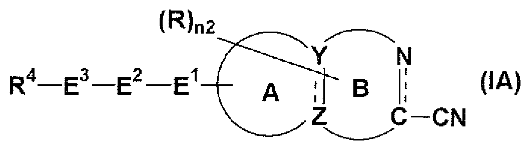

wherein all symbols have the same meanings as described above.
can be also prepared by reaction described hereinafter.
(a) Among a compound represented by formula (IA), a compound wherein E² is -C(=O)NR¹⁰-, -NR¹⁰C(=O)-, -C(=O)O-, or -OC(=O)-, i.e. a compound represented by formula (IA-1) 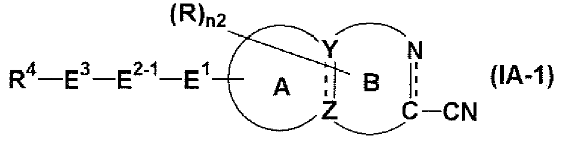
wherein E²⁻¹ is -C(=O)NR¹⁰-, -NR¹⁰C(=O)-, -C(=O)O-, or -OC(=O)-, and other symbols have the same meanings as described above.
can be prepared by a amidation or esterification of a compound represented by formula (III-1) 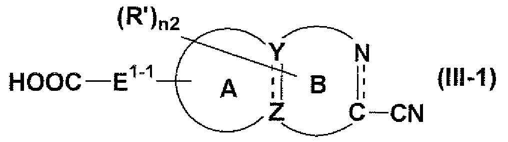 wherein E¹⁻¹ has the same meaning as E¹. With proviso that, carboxy group, hydroxy group, amino group or mercapto group in E¹⁻¹ may be protected, if necessary. Other symbols have the same meanings as described above,
with a compound represented by formula (IV-1)

**R**^{**4-1**}**-E**^{**3-1**}**-R**^{**5**} **(IV-1)**

wherein E³⁻¹ and R⁴⁻¹ have the same meanings as E³ and R⁴ respectively. With proviso that, carboxy group, hydroxy group, amino group or mercapto group in E³⁻¹ and R⁴⁻¹ may be protected, if necessary. R⁵ is -NHR¹⁰, or -OH, and other symbols have the same meanings as described above,
or a compound represented by formula (III-2) 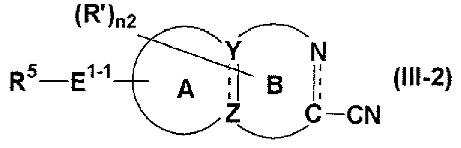 wherein all symbols have the same meanings as described above,
with a compound represented by formula (IV-2)

**R**^{**4-1**}**-E**^{**3-1**}**-COOH** **(IV-2)**

wherein all symbols have the same meanings as described above,
if necessary, followed by removal of the protecting group.

Amidation reaction or esterification reaction is known, for example, (1) a method using acid halide; (2) a method using mixed anhydride; (3) a method using a condensing agent, *etc.* To explain these methods concretely,
(1) The method using acid halide is carried out, for example, by subjecting to a reaction carboxylic acid and acid-halogenating agent (oxalyl chloride, thionyl chloride, *etc.)* in an organic solvent (chloroform, dichloromethane, diethyl ether, tetrahydrofuran, *etc.)* or without a solvent, at between -20°C and refluxing temperature, and then subjecting thus obtained acid halide to a reaction with amine or alcohol in the presence of base (pyridine, triethylamine, dimethylaniline, dimethylaminopyridine, diisopropylethylamine, *etc.)* in an organic solvent (chloroform, dichloromethane, diethyl ether, tetrahydrofuran, *etc*.) at a temperature of 0 to 40°C. Alternatively, it may be carried out by subjecting acid halide to a reaction with amine or alcohol in an organic solvent (dioxane, tetrahydrofuran, *etc*.) using an aqueous alkali solution (an aqueous solution of sodium bicarbonate or sodium hydroxide, *etc*.) at a temperature of 0 to 40°C.
(2) In a method where mixed anhydride is used, for example, carboxylic acid is subjected to a reaction with acid halide (pivaloyl chloride, tosyl chloride, mesylchloride, *etc.)* or acid derivative (ethyl chloroformate, isobutyl chloroformate, *etc*.) in an organic solvent (chloroform, dichloromethane, diethyl ether, tetrahydrofuran, *etc.*) or without a solvent, in the presence of a base (pyridine, triethylamine, dimethylaniline, dimethylaminopyridine, diisopropylethylamine, *etc.*), at a temperature of 0 to 40°C, and then thus obtained mixed anhydride is subjected to a reaction with amine or alcohol in an organic solvent (chloroform, dichloromethane, diethyl ether, tetrahydrofuran, *etc.*) at a temperature of 0 to 40°C.
(3) In a method where a condensing agent is used, for example, carboxylic acid is subjected to a reaction with amine or alcohol derivative in an organic solvent (chloroform, dichloromethane, dimethylformamide, diethyl ether, tetrahydrofuran, *etc*.) or without a solvent, in the presence or absence of a base (pyridine, triethylamine, dimethylaniline, dimethylaminopyridine, *etc.),* using a condensing agent (1,3-dicyclohexylcarbodiimide (DCC), 1-ethyl-3-[3-(dimethyl amino)propyl]carbodiimide (EDC), 1,1'-car-bonyldiimidazole (CDI), 2-chloro-1-methylpyridinium iodide, 1-propanephosphonic acid cyclic anhydride (PPA), *etc.)* in the presence or absence of 1-hydroxybenzotriazole (HOBt) or 1-hydroxy-7-azabenzotriazole (HOAt), at a temperature of 0 to 40°C.

The reactions (1), (2) and (3) are desirably carried out under atmosphere of inert gas (argon, nitrogen, *etc.*) and anhydrous conditions.

The removal of the protecting group may be carried out by the above described method.
(b) Among a compound represented by formula (IA), a compound wherein E² is -SO₂NR¹⁰-, or -NR¹⁰SO₂-, i.e. a compound represented by formula (IA-2) 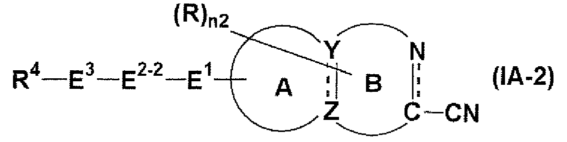
wherein E²⁻² is -SO₂NR¹⁰-, or -NR¹⁰SO₂-, and other symbols have the same meanings as described above,
can be prepared by sulfonamidation of a compound represented by formula (V-1) 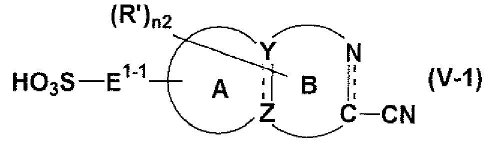 wherein all symbols have the same meanings as described above, 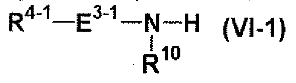 with a compound represented by formula (VI-1)
wherein all symbols have the same meanings as described above, or a compound represented by formula (V-2) 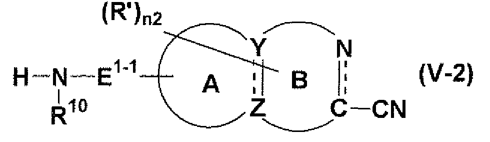 wherein all symbols have the same meanings as described above,
with a compound represented by formula (VI-2)

**R**^{**4-1**}**-E**^{**3-1**}**-SO**_{**3**}**H** **(VI-2)**

wherein all symbols have the same meanings as described above,
if necessary, followed by removal of the protecting group.

Sulfonamidation reaction is known, for example, by subjecting to a reaction sulfonic acid and acid-halogenating agent (oxalyl chloride, thionyl chloride, phosphorus tetrachloride, phosphorus trichloride, *etc*.) in an organic solvent (chloroform, dichloromethane, diethyl ether, tetrahydrofuran, methyl tert-butyl ether, *etc.*) or without a solvent, at between -20°C and refluxing temperature, and then subjecting thus obtained acid halide to a reaction with amine in the presence of base (diisopropylethylamine, pyridine, triethylamine, dimethylaniline, dimethylaminopyridine, *etc.)* in an organic solvent (chloroform, dichloromethane, diethyl ether, tetrahydrofuran, *etc.*) at a temperature of 0 to 40°C.

The removal of the protecting group may be carried out by the above described method.
(c) Among a compound represented by formula (IA), a compound wherein E² is -O-, -NR¹⁰-, or -S-, i.e. a compound represented by formula (IA-3) 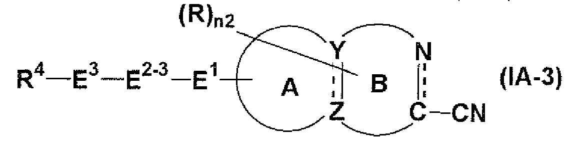
wherein E²⁻³ is -O-, -NR¹⁰-, or -S-, and other symbols have the same meanings as described above,
can be prepared by reaction of a compound represented by formula (VII-1) 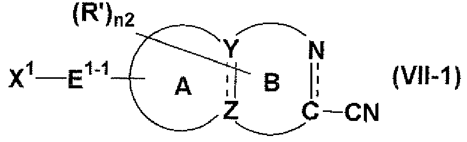 wherein X is a leaving group, for example, halogen, mesyloxy, tosyloxy, *etc.,* and other symbols have the same meanings as described above,
with a compound represented by formula (VIII-1)

**R**^{**4-1**}**-E**^{**3-1**}**-R**^{**6**} **(VIII-1)**

wherein R⁶ is -OH, -SH or -NHR¹⁰, and other symbols have the same meanings as described above,
or a compound represented by formula (VII-2) 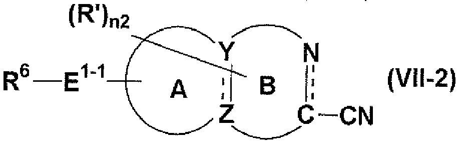 wherein all symbols have the same meanings as described above,
with a compound represented by formula (VIII-2)

**R**^{**4-1**}**-E**^{**3-1**}**-X**^{**1**} **(VIII-2)**

wherein all symbols have the same meanings as described above,
if necessary, followed by removal of the protecting group.

The reaction is well known. For example, it may be carried out with hydroxide of alkaline metal (sodium hydroxide, potassium hydroxide, lithium hydroxide, *etc*.), hydroxide of alkaline earth metal (barium hydroxide, calcium hydroxide, *etc.*) or carbonate (sodium carbonate, potassium carbonate, *etc*.), or aqueous solution thereof, or a mixture thereof at about 0 to 100°C in an organic solvent (dimethylformaimide, dimethylsulfoxide, chloroform, dichloromethane, diethyl ether, tetrahydrofuran, methyl t-butyl ether, *etc.*).

The removal of the protecting group may be carried out by the above described method.

Among a compound represented by formula (IA-3), a compound wherein E²⁻³ is-O-, i.e. a compound represented by formula (IA-3-1) 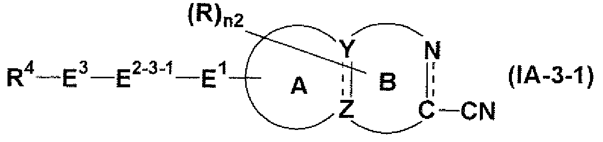 wherein E²⁻³⁻¹ is -O-, and other symbols have the same meanings as described above,
can be prepared by reaction of a compound represented by formula (IX) 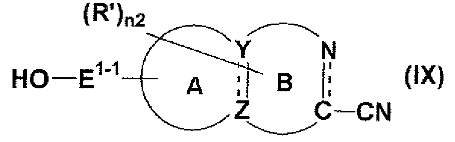 wherein all symbols have the same meanings as described above,
with a compound represented by formula (X)

**R**^{**4-1**}**-E**^{**3-1**}**-OH** **(X)**

wherein all symbols have the same meanings as described above,
if necessary, followed by removal of the protecting group.

The reaction is well known. For example, it may be carried out with corresponding alcohol compound at about 0 to 60°C in an organic solvent (dichloromethane, diethyl ether, tetrahydrofuran, acetonitrile, benzene, toluene, *etc*.) in the presence of azo compound (diethyl azodicarboxylate (DEAD), diisopropyl azodicarboxylate, 1,1'-(azodicarbonyl)dipiperidine, 1,1'-azo-bis(N,N-dimethylformamide), *etc.*) and phosphine compound (triphenylphosphine, tributylphosphine, trimethylphosphine, polymer-supported triphenylphosphine, *etc.*).

The removal of the protecting group may be carried out by the above described method.

The compound represented by formulae (II), (III-1), (III-2), (IV-1), (IV-2), (V-1), (V-2), (VI-1), (VI-2), (VII-1), (VII-2), (VIII-1), (VIII-2), (IX), or (X) used as a starting material are known itself, or can be prepared easily by the methods described in examples or the methods per se such as the methods described in Comprehensive Organic Transformations: A Guide to Functional Group Preparations, 2nd Edition (Richard C. Larock, John Willey & Sons Inc, 1999) or the methods described in Chem. Ber., 96, 1505, 1963; Chem. Ber., 98, 1081, 1965; J. Org. Chem., 53, 4137, 1988; Bioorg. Med. Chem. Lett., 9, 2569-2572, 1999; Synlett, 2000(6), 829-831; Synthesis, 2001, 55-62; J. Chem. Soc., Perkin Trans 1, 2002, 1847, *etc.*

For example, among a compound represented by formula (I), a compound represented by formulae (IB), (IC), (ID-I), (ID-2), (IE), (IF-1), (IF-2), (IG-1), (IG-2), or (IG-3) can also be prepared by reaction schemes 1 to 6 described hereinafter.

In the reaction schemes, R¹⁰¹ represents halogen, R¹⁰² represents a protective group of amino, Ph represents phenyl, TEA represents triethylamine, Me represents methyl, Et represents ethyl, Boc represents tert-butoxycarbonyl, R¹⁰³ represents C1-8 alkyl, and other symbols have the same meanings as described above.

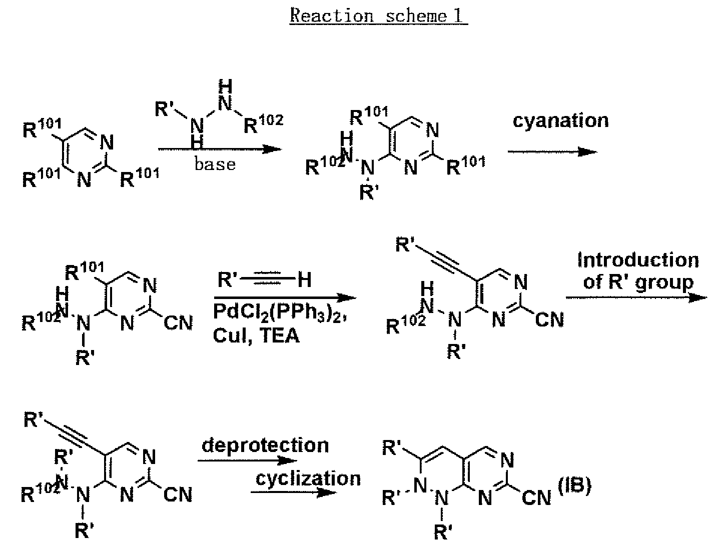

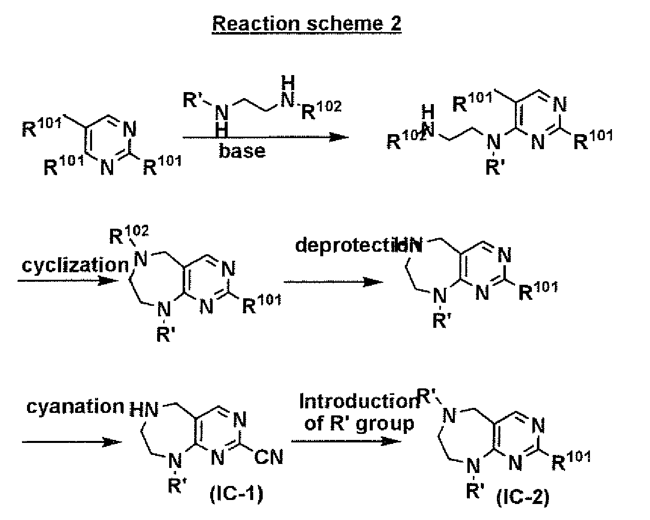

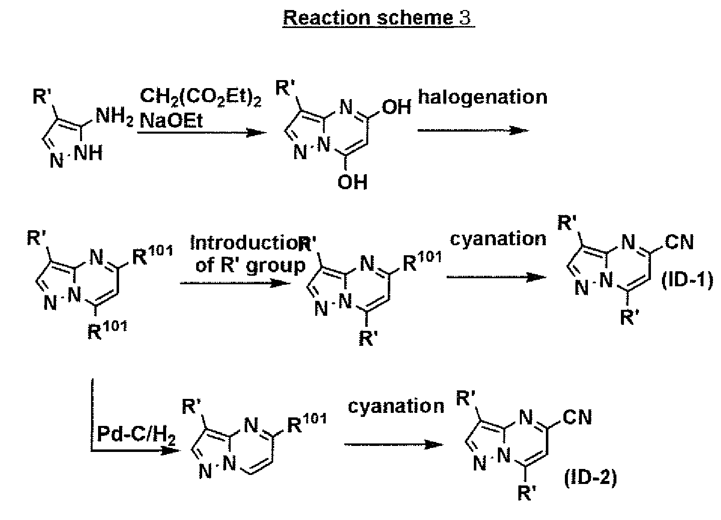

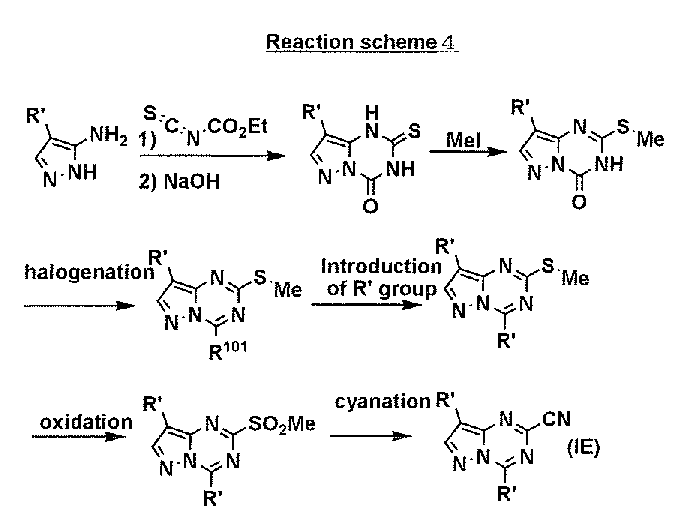

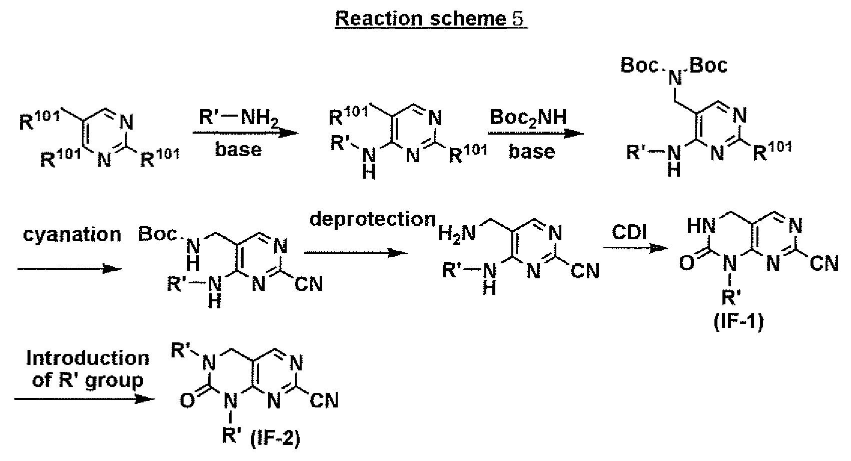

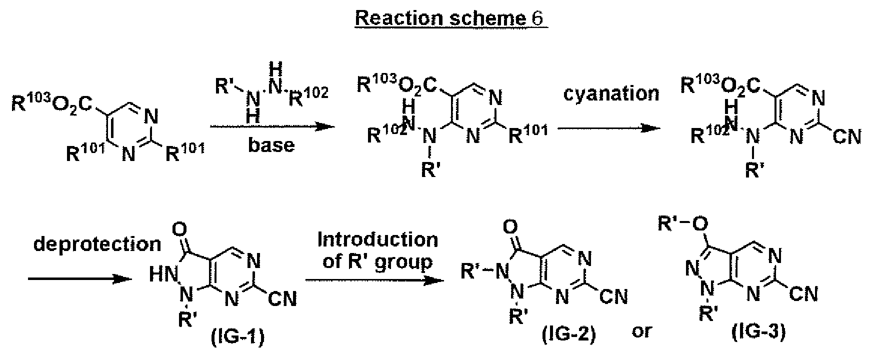

All reactions in each reaction scheme is well known. All starting materials and reagents in the reaction schemes are known compounds, or can be prepared easily by known methods.

In each reaction of the present specification, reaction products may be purified by conventional techniques, for example, distillation under atmospheric or reduced pressure, high performance liquid chromatography, thin layer chromatography or column chromatography using silica gel or magnesium silicate, washing or recrystallization, *etc.* Purification may be carried out after each reaction, or after a series of reactions.

### Pharmacological activities of the compounds of the present invention:

The following methods can be used as pharmacological test besides the methods described in "Biological Examples."

### (1) Measurement of cathepsin B inhibitory activity

Cathepsin B inhibitory activity can be measured in the way prescribed hereinafter.

10 µL of synthesized substrate (carbobenzoxy-L-arginyl-L-arginine-4-methyl-chromanyl-7-amide or carbobenzoxy-L-phenylalanyl-L-arginine-4-methyl-chromanyl-7-amide) solution of several concentrations, 10 µl of cysteine protease inhibitor solution of several concentrations, 70 µl of cathepsin B enzyme reaction buffer (mixture of 400 mmol/L of acetic acid, 4 mmol/L of EDTA, 8 mmol/L of DDT to adjust to pH 5.5) and 10 µl of cathepsin B enzyme solution are mixed and the increase of fluorescence intensity is measured (λex (excitation wavelength)=355 nm, λem (fluorescence wavelength)=460 nm) when reacted at 37°C.

### (2) Measurement of cathepsin S inhibitory activity

Cathepsin S inhibitory activity can be measured in the way prescribed hereinafter.

10 µl of synthesized substrate (carbobenzoxy-L-leucyl-L-leucyl-L-arguinine-4-methyl-chromanyl-7-amide) solution and 5 µl of cysteine protease inhibitor solution of several concentrations, 75 µl of cathepsin S enzyme reaction buffer (100 mmol/L of sodium phosphate, 2 mmol/L of EDTA, 2 mmol/L of DTT are mixed to adjust to pH 6.5) and 10 µl of cathepsin S enzyme solution are mixed and the increase of fluorescence intensity is measured (λex(excitation wavelength) =355 nm, λem (fluorescence wavelength)=460 nm) when reacted at 37°C.

### (3) Measurement of cathepsin L inhibitory activity

Cathepsin L inhibitory activity can be measured in the way prescribed hereinafter.

5 µl of Synthesized substrate (carbobenzoxy-L-phenylalanyl-L-arguine-4-methylchromanyl-7-amide or L-prolyl-L-phenylalanyl-L-arginine-4-methylchromanyl-7-amide) solution and 5 µl of cysteine protease inhibitor solution of several concentrations, 80 µl of cathepsin L enzyme reaction buffer (400 mmol/L of acetic acid, 4 mmol/L of EDTA, 8 mmol/L of DTT are mixed to adjust to pH 5.5) and 10 µl of cathepsin L enzyme solution are mixed and the increase of fluorescence intensity is measured (λex (excitation wavelength)=355 nm, λem (fluorescence wavelength)=460 nm) when reacted at 37°C.

### (4) Measurement ofcalpain inhibitory activity

The activity is measured according to the method described in Calcium-depending protease, Seibutsukagaku-Jikkenhou (Biochemistry Experimental Method) Tanpakubunkaikouso (Protease) I, 57 (1993).

### (5) Measurement of caspase-1 inhibitory activity

Caspase-1 inhibitory activity can be measured in the way prescribed hereinafter.

50 µl of caspase-1 enzyme reaction solution (20 mmol/L of 4-(2-hydroxyethyl)-1-piperazinethanesulfonate-sodium hydroxide buffer (pH 7.4), 10 mmol/L of potassium chloride, 1.5 mmol/L of magnesium chloride, 0.1 mmol/L EDTA, 10% glycerol) and 50 µl of cysteine protease inhibitor solution of several concentrations, 50 µl of caspase-1 enzyme solution and 100 µl of synthesized substrate (acetyl-L-tyrosinyl-L-valinyl-L-alanyl-L-aspartic acid-4-methylchromanyl-7-amide) solution of several concentrations are reacted at 37°C and the fluorescence intensity is measured (λex (excitation wavelength)=355 nm, λem (fluorescence wavelength)=460 nm).

### (6) Investigation in bone resorption inhibitory activity using mouse calvaria cultivation system

Mouse neonatal calvaria is cultured in D-minimum essential medium containing cysteine protease inhibitor (mixture of Penicillin G potassium (final concentration 100 U/ml), streptomycin sulfate (final concentration 0.1 mg/ml), bovine serum albumin (final concentration 0.1%), glutamine (final concentration 0.3 mg/ml) in D-minimal essential medium) with incitant (parathyroid hormone (PTH) or arotinoid) at 37°C and the calcium concentration in the culture medium is measured.

### (7) Bone resorption pit formation test using rabbit osteoclast cells

Osteoclast cells collected from rabbit bones are sowed over slices of bovine cortical bone, dentine or teeth of toothed whale and are cultured at 37°C in α-minimal essential medium containing final concentration 5% of fetal bovine serum and various concentrations of cysteine protease inhibitor. The pits form on the slices by the osteoclast cells are observed and at the same time type-I collagen C-terminal telopeptide (CTx) concentration in culture medium can be measured.

### (8) Investigation of immune reaction inhibitory effect using antigen-sensitized mouse spleen cells

Spleen cells are collected from mice sensitized by ovalbumin (OVA) several times. Inhibitory effect of cysteine protease inhibitors against immune response induced by OVA stimulus can be investigated, using cytokine concentration and immunoglobulin concentration in culture solution as indicators.

### (9) Investigation in inhibitory effect against bone resorption using the rat PTH hypercalcemia model

The effect of cysteine protease inhibitor (compulsory oral administration, intraperitoneal administration) on bone resorption which is promoted by intravenous administration of parathyroid hormone (PTH) solution (30 µg/ml) can be investigated in rats, using calcium concentration in blood as an indicator.

### (10) Studies on bone resorption inhibitory effect using TPTx rat PTHrP-induced hypercalcemia model

The effect of cysteine protease inhibitor (compulsory oral administration, intraperitoneal administration) on bone resorption, promoted by subcutaneous administration of parathyroid hormone related peptide (PTHrP) to a fasting rat (thyroparathyroidectomized; TPTx) can be investigated, using calcium concentration in blood as an indicator.

### (11) Measurement of cathepsin H inhibitory activity

Using Arg-MCA aminomethylcoumarin as synthetic substrate, according to the method described in FEBS Lett. 280(2), 307-310/1991, Methods Enzymol. 80, 535-561/1981, the activity can be measured.

### (12) Measurement of cathepsin C inhibitory activity

Using Gly-Phe-CHN₂ as synthetic substrate, according to the method described in J. Immnol. 150, 4733-4742,1993, the activity can be measured.

### Toxicity:

The toxicity of the compound of formula (I), a salt thereof, a solvate thereof, or an N-oxide thereof, or a prodrug thereof was very low, so the compound is safe enough for pharmaceutical use.

### Application to pharmaceuticals:

The compound represented by formula (I), a salt thereof, a solvate thereof, or an N-oxide thereof, or a prodrug thereof (hereinafter referred to as "the compound of formula (I)") has an inhibitory activity against cysteine proteases (cathepsins such as K, L, S, B, F, H, C, V, O, W, Z, *etc.,* caspases such as caspase-1, calpains such as calpain, *etc.),* and therefore it is useful as an agent for the prophylaxis and/or treatment of inflammatory diseases (periodontitis, arthritis, inflammatory bowel diseases, infectious diseases, pancreatitis, hepatitis, glomerulonephritis, endocarditis, myocarditis, ulcerative colitis, *etc.*)*,* diseases induced by apoptosis (graft versus host diseases, rejection in transplantation, acquired immunodeficiency syndrome (AIDS), AIDS-related complex (ARC), adult T cell leukemia, hairy cells leukemia, spondylopathy, disorders of respiratory apparatus, arthritis, HIV or HTLV-1 related diseases (uveitis *etc.*), virus related diseases (hepatitis C *etc.),* cancer, collagenosis (systemic lupus erythematosus, rheumatoid arthritis, *etc*.), ulcerative colitis, Sjoegren syndrome, primary biliary cirrhosis, spontaneous thrombocytopenic purpura, autoimmune hemolytic anemia, myasthenia gravis, autoimmune diseases (insulin dependent (type I) diabetes, *etc*.), diseases accompanied by thrombocytopenia (myelodysplastic syndrome, cyclic thrombocytopenia, aplastic anemia, spontaneous thrombocytopenia, disseminated intravascular coagulation (DIC), *etc.*), viral hepatitis (A, B, C, F, *etc.*) or hepatitis medicamentosa and cirrhosis, *etc*., dementia such as Alzheimer's disease, Alzheimer-type senile dementia, cerebrovascular injury, neurodegenerative disease, adult acute respiratory distress syndrome, infectious diseases, prostatomegaly, hysteromyoma, bronchial asthma, arteriosclerosis, hyperlipidemia, all kinds of lusus naturae, nephritis , senile cataract, chronic fatigue syndrome, myodystrophy, peripheral nerve injury, *etc.*), diseases induced by immune response disorder (graft versus host diseases, rejection in transplantation, allergic diseases (asthmatic bronchitis, atopic dermatitis, allergic rhinitis, hay fever, diseases by house dust, hypersensitive pneumonia, food allergy, *etc.*), psoriasis, rheumatoid arthritis, *etc*.), autoimmune diseases (insulin dependent (type I) diabetes, systemic lupus erythematosus, Hashimoto's diseases, multiple sclerosis, *etc*.), diseases induced by decomposition of proteins which compose a body (myodystrophy, cataract, periodontitis, hepatocyte injury by bile acid (cholestatic cirrhosis *etc.), etc.,* decomposition of alveolus elastica (emphysema *etc.*), ischemic diseases (brain ischemia, brain disorder by ischemic reperfusion, cardiac infarction, ischemic liver damage, *etc.), etc.),* shock (septic shock, systemic inflammatory responsive syndrome, endotoxin shock, acidosis, *etc.*), circulatory disorder (arteriosclerosis, restenosis after PTCA (percutaneous transluminal coronary angioplasty), *etc.*), disorder of blood coagulation system (thrombocytopenic purpura, hemolytic uremic syndrome, *etc*.), malignant tumor, acquired immune deficiency syndrome (AIDS, AIDS-related complex (ARC), *etc.*), parasitic diseases (malaria *etc*.), neurodegenerative disease (Alzheimer-type senile dementia, Huntington's chorea, Parkinson's disease, multiple sclerosis, traumatic encephalopathy, traumatic spondylopathy, *etc.*), lung disorder (fibroid lungs, *etc.),* bone diseases (osteoporosis, bone fracture, rheumatoid arthritis, arthritis, osteoarthritis, hypercalcaemia, osteometastasis of cancer, periodontitis, bone Paget's disease, *etc.*), endocrinesthenia (hyperthyroidism *etc.*), *etc.* As the compound of formula (I) has an inhibitory activity against cystein protease, it is also useful as a bone resorption inhibitor.

Cysteine protease which the compound of formula (I) inhibits is all preferable, for example, cathepsin K, cathepsin L, cathepsin S, cathepsin B, cathepsin H, cathepsin F, cathepsin Y, cathepsin C, calpain, caspase-1, *etc.* Among them, cathepsin K is most preferred. Of course, cysteine proteases other than them are included in the scope of the present invention and naturally so are those cysteine proteases to be discovered in the future.

The compound of formula (I) may also be administered as a concomitant agent in combination with other agents for
1) supplement and/or reinforcement of preventive and/or treating effect(s) of the compound of formula (I),
2) improvement in kinetics and absorption of the compound of formula (I) and reduction of dose and/or
3) reduction of side effect of the compound of formula (I).

A concomitant agent of the compound of formula (I) with other agents may be administered in a mode of compounded agent in which both components are compounded in a single preparation or in a mode of separate preparations. When administration is conducted using separate preparations, a simultaneous administration and administrations with time difference is included. In the case of administrations with time difference, the compound of formula (I) may be firstly administered and then other drug may be administered, or the other drug may be firstly administered and then the compound of formula (I) may be administered. Each of the methods for the administration may be the same or different.

There is no particular limitation for the diseases for which the above-mentioned concomitant agent achieves the preventive and/or the treating effect but any disease will be acceptable so far as it supplements and/or enforces the preventive and/or treating effect of the compound of formula (I).

For example, examples of the other drug for supplement and/or reinforcing the preventive and/or treating effect of the compound of formula (I) to bone diseases include, for example, bisphosphonate formulations, Vitamin D and its derivatives, Vitamin K and its derivatives, calcitonin formulations, α-calcitonin gene-related peptide formulations, female hormone formulations, selective estrogen receptor modulators (SERM), ipriflavone formulations, calcium formulations, anabolic steroid formulations, parathyroid hormone (PTH) formulations, PTHrP derivatives, caspase-1 inhibitors, farnesoid X receptor agonists, Bone Morphogenetic Protein (BMP) formulations, anti-RANKL (receptor activator of NF-kappa B ligand) antibody, metalloprotease inhibitors, prostaglandin derivatives, strontium formulations, anti TNF-α antibody, anti-IL-6 antibody, HMG-CoA reductase inhibitors, steroidal drugs, and antiinflammatory drugs, *etc.* Nonsteroidal antiinflammatory agent, local anesthetic agent and/or muscle-relaxing drug can also be used together for the purpose of redress of pain accompanied by bone fracture and osteoporosis.

Bisphosphonate formulations include, for example, minodronic acid (1-hydroxy-2-(imidazo[1,2-a]pyridin-3-yl)ethylidenebisphosphonic acid, salt thereof, or hydrate thereof), alendronate (4-amino-1-hydroxybutylidene-1,1-bisphosphonic acid (alendronic acid), or sodium salt thereof, or trihydrate thereof), incadronate (cycloheptylaminomethylene-1,1-diphosphoric acid (incadronic acid), or disodium salt thereof, hydrate thereof), clodronate (1,1-dichloromethylene-1,1-diphosphoric acid (clodronic acid), or disodium salt thereof), tiludronate ((4-chlorophenyl)thiomethylene-1,1-diphosphoric acid (tiludronic acid), or disodium salt thereof), etidronate (1-hydroxy-1,1-diphosphoric acid (etidronic acid), or disodium salt thereof,), ibandronate (1-hydroxy-3-(N-methyl-N-pentylamino)propylidene-1,1-bisphosphonic acid), risedronate (1-hydroxy-2-pyridine-3-ylethylidenediphosphoric acid (risedronic acid), or sodium salt thereof, sester hydrate thereof), piridronate ([2-(2-pyridinyl)ethylidene]-1,1-bisphosphonic acid), pamidronate (3-amino-1-hydroxypropylidene-1,1-bisphasphonic acid (pamidronic acid), or disodium salt thereof, or pentahydrate thereof), zoledronate (1-hydroxy-2-(1H-imidazal-1-yl)ethylidene-1,1-bisphosphanic acid, or hydrate thereof), olpadronate (3-(dimethylamino)-1-hydroxypropylidene-1,1-bisphosphonic acid), neridronate (6-amino-1-hydroxyhexylidene-1,1-bisphosphonic acid), *etc.*

Vitamin D include, for example, Vitamin D₂ (ergacalciferol), Vitamin D₃ (cholecalciferol), *etc.*

Vitamin D derivatives include, for example, alfacalcidol, falecalcitriol, calcitriol, 1α,25-dihydroxycholecalciferol, dihydrataxisterol, ST-630, KDR, ST-630, ED-71, rocaltrol (Ro44-7190), tacalcitol, maxacalcitol, *etc.*

Vitamin K and its derivatives include, for example, vitamin K₁ (phytonadione), vitamin K₂ (menatetrenone), *etc.*

Calcitonin formulations include, for example, calcitonin salmon (STH-32, SMC20-51), calcitonin chicken (MCI-536), secalciferol, elcatonin, TJN-135, *etc.*

Female hormone formulations include, for example, estrogen preparations, progesterone preparations, *etc*. Estrogen preparations include, for example, estrogen, estradiol, estradiol benzoate, estradiol cypionate, estradiol dipropionate, estradiol enannthate, estradiol hexahydrobenzoate, estradiol phenylpropionate, estradiol undecanoate, estradiol valerate, estrone, ethinylestradiol, mestranol, estriol, chlormadinone acetate, norethisterone. Progesterone preparations include, for example, progesterone, hydroxyprogesterone caproate, medroxyprogesterone acetate, trimegestone, *etc*.

Selective estrogen receptor modulators (SERM) include, for example, tamoxifen, lasofoxifene tartrate, raloxifene hydrochloride, bazedoxifene acetate, PSK-3471, *etc.*

Ipriflavone formulations include, for example, ipriflavone, *etc.*

Calcium formulations include, for example, calcium chloride, calcium gluconate, calcium glycerophosphate, calcium lactate, calcium L-aspartate, calcium hydrogen phosphate, *etc.*

Anabolic steroid formulations include, for example, nandrolone decanoate, nandrolone phenylpropionate, nandrolone cyclohexylpropionate, metenolone enanthate, mestanolone, stanozolol, oxymetholone, *etc*.

Parathyroid hormone (PTH) formulations include, for example, PTH, teriparatide acetate, MBRI-93.02, Ostabolin-C, *etc.*

PTHrP derivatives include, for example, RS-66271, hPTHrP, *etc.*

Caspase-1 inhibitors include, for example, pralnacasan, nitroflubiprofen, *etc.*

Farnesoid X receptor agonists include, for example, SR-45023A, *etc.*

Anti-RANKL antibody includes, for example, AMG162, *etc.*

Metalloprotease inhibitors include, for example, minocycline hydrochloride, *etc.*

Prostaglandin derivatives include, for example, EP2 agonist, EP4 agonist, EP4 antagonist, for example, ONO-4819, nitroflurbiprofen, *etc*.

Strontium formulations include, for example, strontium ranelate, *etc.*

Anti TNF-α antibody include, for example, infliximab, etanercept, *etc.*

Anti-IL-6 antibody include, for example, MRA, *etc.*

HMG-CoA reductase inhibitors include, for example, pravastatin, simvastatin, lovastatin, fluvastatin, atorvastatin, pitavastatin, rosuvastatin, *etc.*

Steroidal drugs include, for example, KB-889 (OD14, tibolone), Hipros (TZP-4238), *etc.*

Antiinflammatory drugs include, for example, sasapyrine, sodium salicylate, aspirin, aspirin dialuminate formulation, diflunisal, indomethacin, suprofen, ufenamate, dimethylisopropyl azulen, bufexamac, felbinac, diclofenac, tolmetin sodium, Clinoril, fenbufen, napmetone, proglumetacin, indomethacin farnesil, acemetacin, proglumetacin maleate, amfenac sodium, mofezolac, etodolac, ibuprofen, ibuprofen piconol, naproxen, flurbiprofen, flurbiprofen axethyl, ketoprofen, fenoprofen calcium, tiaprofenen, oxaprozin, pranoprofen, loxoprofen sodium, aluminoprofen, zaltoprofen, mefenamic acid, aluminum mefenamate, tolfenamic acid, floctafenine, ketophenylbutazone, oxyfenbutazone, piroxicam, tenoxicam, anpiroxicam, napageln cream, epirizole, tiaramide hydrochloride, tinoridine hydrochloride, emorfazone, sulpyrine, Migrenin, Saridon, Sedes G, Amipylo N, Sorbon, pyrine system antipyretics, acetaminophen, phenacetin, dimethothiazine mesylate, simetride formulation, or antipyrine system antipyretics, *etc*.

There is no limitation for the ratio by weight of the compound of formula (I) to other agents.

With regard to other agents, two or more agents may be administered in combination.

Such other agents which supplement and/or reinforce the preventive and/or treating effect of the compound of formula (I) include not only those which have been found on the basis of the above-mentioned mechanism but also those which will be found in future.

The pharmaceutical composition comprising the compound of formula (I), a combination of the compound of formula (I) and other drug as an active ingredient is generally administered systemically or topically and orally or parenterally when it is used for the above objects.

The dosages are determined depending on age, body weight, symptom, therapeutic effect, administration route, duration of the treatment and the like. Generally, 1 mg to 1000 mg per adult is orally administered once to several times per day, or 0.1 mg to 100 mg per adult is parenterally administered (preferably by intravenous administration) once to several times per day, or continuously administered from vein for 1 to 24 hours per day.

Since the dose changes depending on various conditions as described above, there are cases in which doses lower than or greater than the above ranges may be used.

The pharmaceutical composition comprising the compound of formula (I), a concomitant agent of the compound of formula (I) and other drug as an active ingredient may be administered in the form of solid compositions, liquid compositions and other compositions for oral administration, and injections, external preparations, suppositories, and the like for parenteral administration.

Solid compositions for oral administration include tablets, pills, capsules, dispersible powders, granules and the like. Capsules include hard capsules and soft capsules.

In such solid compositions, one or more of the active compound(s) may be admixed with vehicles (such as lactose, mannitol, glucose, microcrystalline cellulose or starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid compositions for oral administration include pharmaceutically acceptable solutions, suspensions, emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration in the present invention include solutions, suspensions and emulsions, and also solid injections which are to be dissolved or suspended in solvents upon use. Such an injection is prepared by dissolving, suspending or emulsifying one or more active substances in a solvent and then put to use. Examples of the solvent include distilled water for injection, physiological saline, plant oil, alcohols such as propylene glycol, polyethylene glycol and ethanol, and combinations thereof Further, the injection may contain a stabilizer, a solubilizing auxiliary agent such as glutamic acid, aspartic acid and POLYSORBATE 80 (registered trade mark) *etc.) ,* suspending agent, emulsifying agent, soothing agent, buffering agent, preservative agent and the like. The injection may be sterilized in the final step of the preparation process or the whole preparation process may be operated under sterile conditions. Alternatively, the sterile product, for example a sterile freeze-dried product may be prepared, and upon use, the product may be dissolved in sterilized or aseptic distilled water for injection or other sterilized or aseptic solvents.

Other compositions for parenteral administration include liquids for external use, ointments, endemic liniments, inhalants, spray compositions, suppositories for intrarectal administration, and pessaries for intravaginal administration and the like containing one or more active compound(s) which can be prepared by known methods.

Spray compositions may contain stabilizing agents such as sodium hydrogen sulfate, buffering agents to give isotonicity, isotonic solutions such as sodium chloride, sodium citrate or citric acid, in addition to inert diluents. For preparation of such spray compositions, for example, the method described in the United States Patent Nos 2,868,691 and 3.095.355.

### Effect of the invention:

The compounds of the present invention have the inhibitory activity against cathepsin K, so they are useful for preventing and/or treating cathepsin K-related diseases.

### BEST MODE FOR CARRYING OUT THE INVENTION

The following Examples are intended to illustrate the present invention, but the present invention is not limited thereto.

In chromatographic separations and TLC, the solvents in parenthesis show the eluting and developing solvents and the ratios of the solvents used are by volume. The solvents in the parentheses in NMR show the solvents for measurement.

All the compounds described in the present specification were named by using ACD/Name (registered trademark, Advanced Chemistry Development Inc.) or ACD/Name Batch (registered trademark, Advanced Chemistry Development Inc.), which is a computer software that generally names the compound according to IUPAC nomenclature system, or directly named according to IUPAC nomenclature system.

### Example 1

### 2-chloro-5-(chloromethyl)-N-(2,2-dimethylpropyl)-4-pyrimidineamine:

Under atmosphere of argon, 2,4-dichloro-5-(chloromethyl)pyrimidine (3.95 g) was dissolved to tetrahydrofuran (34 mL), and the mixture was cooled to -5°C. To the reaction mixture was added triethylamine (3.37 mL), and neopentylamine (2.36 mL) was dropped thereto, then the mixture was stirred for 3.5 hours with temperature rising little by little. The reaction mixture was diluted by ethyl acetate, added by brine and water, then separated. The water layer was extracted with ethyl acetate. The combined organic layer was washed with a small amount of saturated aqueous solution of sodium hydrogen carbonate and brine, dried over anhydrous sodium sulfate, then concentrated. To the residue was added tert-butyl methyl ether, and an insoluble powder was removed by filtration to give the title compound (872 mg; white powder). The filtrate was concentrated. The obtained residue was purified by column chromatography on silica gel (hexane : ethyl acetate = 5 : 1 → 4 : 1) to give the title compound (1.19 g) having the following physical data.
TLC : Rf 0.44 (hexane : ethyl acetate = 2 : 1).

### Example 2

### di-tert-butyl ({2-chloro-4-[(2,2-dimethylpropyl)amino]-5-pyrimidinyl}methyl)imidodicarbonate:

To a solution of di-tert-butyl imidodicarbonate (798 mg) in dimethylformamide (7 mL) on ice bath were added sodium hydride (147 mg; 60%) and dimethylformamide (3 mL), then the mixture was stirred for 20 minutes. To the reaction mixture was added a solution of the compound prepared in Example 1 (870 mg) in dimethylformamide (4 mL), then the mixture was stirred for 30 minutes at 0°C. The reaction mixture was added by iced water, extracted with ethyl acetate. The organic layer was washed with brine, dried over anhydrous sodium sulfate, concentrated The obtained residue was purified by column chromatography on silica gel (hexane : ethyl acetate = 5 : 1) to give the title compound (429 mg) having the following physical data.
TLC : Rf 0.40 (hexane : ethyl acetate =4:1).

### Example 3

### tert-butyl ({2-cyano-4-[(2,2-dimethylpropyl)amino]-5-pyrimidinyl}methyl)carbamate:

To a solution of the compound prepared in Example 2 (200 mg) in dimethylsulfoxide (2 mL) were added potassium cyanide (61 mg), 1,4-diazabicyclo[2.2.2]octane (16 mg) and water (0.3 mL), then the mixture was stirred for 2 hours at 80°C, 4 hours at 100°C, refluxed for 19 hours at 110°C. The reaction mixture was cooled to room temperature, poured into water, extracted with ethyl acetate. The extract was washed with brine, dried over anhydrous sodium sulfate, concentrated. The obtained residue was purified by column chromatography on silica gel (hexane : ethyl acetate = 5 : 2) to give the title compound (75 mg; yellow crystals) having the following physical data.
TLC : Rf 0.39 (hexane : ethyl acetate = 2 : 1).

### Example 4

### 5-(aminomethyl)-4-[(2,2-dimethylpropyl)amino]-2-pyrimidinecarbonitrile:

To a solution of the compound prepared in Example 3 (67 mg) in dichloromethane (I mL) on ice bath was added trifluoroacetic acid (0.1 mL). After the reaction mixture was stirred for 30 minutes, trifluoroacetic acid (0.1 mL) was added thereto, then the mixture was stirred for 1 hour at room temperature. To the reaction mixture was added trifluoroacetic acid (0.05 mL), then the mixture was stirred for 1 hour at room temperature. The reaction mixture was concentrated, then the obtained residue was dissolved to ethyl acetate. The solution was poured into saturated aqueous solution of sodium hydrogen carbonate, extracted with ethyl acetate. The extract was washed with a small amount of brine, dried over anhydrous sodium sulfate, concentrated to give the title compound (45 mg) having the following physical data.
TLC : Rf 0.51 (dichloromethane : methanol = 10 : 1).

### Example 5

### 8-(2,2-dimethylpropyl)-7-oxo-5,6,7,8-tetrahydropyrimido[4,5-d]pyrimidine-2-carbonitrile:

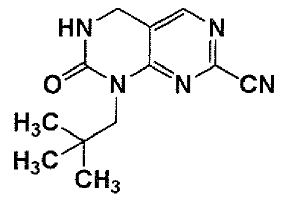

To a solution of carbodiimidazole (31 mg; CDI) in dioxane (0.5 mL) was dropped a solution of the compound prepared in Example 4 (42 mg) in dioxane (0.5 mL), the mixture was stirred for 30 minutes at room temperature. CDI (10 mg) was added thereto, then the mixture was stirred for I hour at room temperature, refluxed for 6.5 hours at 100°C. The reaction mixture was cooled to room temperature, then concentrated. To the residue was added ethyl acetate, the solution was washed with brine, dried over anhydrous sodium sulfate, concentrated. The obtained residue was purified by column chromatography on silica gel (hexane : ethyl acetate = 1 : 1) to give the title compound (35 mg) having the following physical data.
TLC : Rf 0.57 (dichloromethane : methanol = 10 : 1);
¹H NMR (CDCl₃) : δ 0.95 (s, 9 H), 4.07 (s, 2 H), 4.51 (s, 2 H), 5.52 - 5.66 (m, 1 H), 8.31 (t, J = 1.01 Hz, 1 H).

### Example 6

### 7-[(2,2-dimcthylpropyl)amino]-6-methylpyrazolo[1,5-a]pyrimidine-5-carbonitrile:

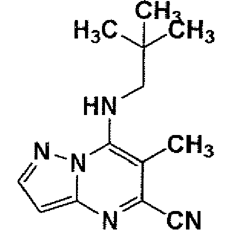

By the same procedures as described in Example 1 → Example 3, using 5,7-dichloro-6-methylpyrazolo[1,5-a]pyrimidine instead of 2,4-dichloro-5-(chloromethyl)pyrimidine in the step corresponding to Example 1, the title compound having the following physical data was obtained.
TLC : Rf0.39(hexane : ethyl acetate = 4 : 1);
¹H NMR (CDCl₃): δ 1.08 (s, 9 H), 2.67 (s, 3 H), 3.59 (d, J = 5.86 Hz, 2 H), 6.60 (d, J = 2.38 Hz, 1 H), 6.75 - 6.90 (m, 1 H), 8.06 (d, J = 2.38 Hz, 1 H).

### Example 7

### 1-(2,2-dimethylpropyl)-3-oxo-2,3-dihydro-1H-pyrazolo[3,4-d]pyrimidine-6-carbonitrile:

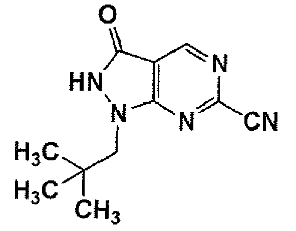

By the same procedures as described in Example 1 → Example 3 → Example 4, using methyl 2,4-dichloropyrimidine-5-carboxylate instead of 2,4-dichloro-5-(chloromethyl)pyrimidine and tert-butyl 2-(2,2-dimethylpropyl)hydrazinecarboxylate instead of neopentylaznine in the step corresponding to Example 1, the title compound having the following physical data was obtained.
TLC : Rf 0.47 (dichloromethane : methanol = 10 : 1);
¹H NMR (CDCl₃) : δ 1.05 (s, 9 H), 4.16 (s, 2 H), 9.20 (s, 1 H).

### Example 8

### 3-(4-biphenylylmethoxy)-1-(2,2-dimethylpropyl)-1H-pyrazolo[3,4-d]pyrimidine-6-carbonitrile (compound 8A), and 2-(4-biphenylylmethyl)-1-(2,2-dimethylpropyl)-3-oxo-2,3-dihydro-1H-pyrazolo[3,4-d]pyrimidine-6-carbonitrile (compound 8B):

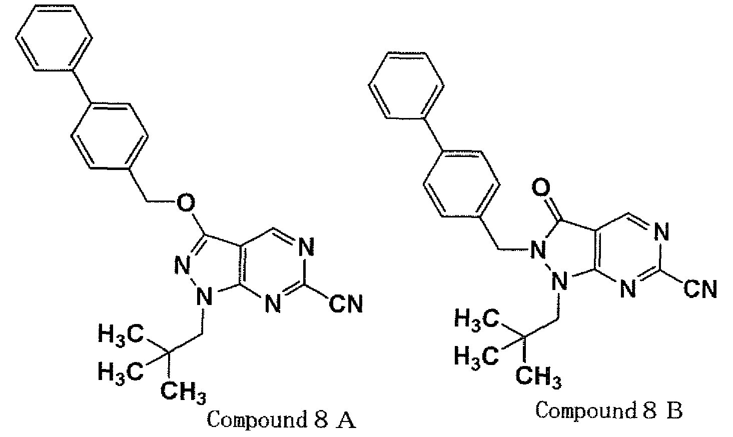

Under atmosphere of argon, to a solution of the compound prepared in Example 7 (60 mg) in tetrahydrofuran (1 mL) were added triphenylphosphine (68 mg) and 4-biphenylylmethanol (48 mg), diethyl azodicarboxylate (2.2M toluene solution, 118 µL) was dropped thereinto, the reaction mixture was stirred overnight at room temperature. The reaction mixture was concentrated. The obtained residue was purified by column chromatography on silica gel (hexane : ethyl acetate = 9 : 1 → 4 : 1) to give the title compound 8A (60 mg) and compound 8B (6 mg) having the following physical data.
Compound 8A :
TLC : Rf 0.63 (hexane : ethyl acetate = 4 : 1);
¹H NMR (CDCl₃) : δ 1.01 (s, 9 H), 4.15 (s, 2 H), 5.48 (s, 2 H), 7.30 - 7.41 (m, 1 H), 7.40 - 7.51 (m, 2 H), 7.51 - 7.72 (m, 6 H), 9.07 (s, 1 H).
Compound 8B :
TLC : Rf 0.43 (hexane : ethyl acetate = 2 : 1);
¹H NMR (CDCl₃) : δ 1.00 (s, 9 H), 3.94 (s, 2 H), 5.32 (s, 2 H), 7.16 (d, J = 8.23 Hz, 2 H), 7.29 - 7.48 (m, 3 H), 7.47 - 7.58 (m, 4 H), 9.13 (s, 1 H).

### Example 8(1) - Example 8(5)

By the same procedures as described in Example 8, using corresponding alcohol compound instead of 4-biphenylylmethanol, the following compounds were obtained. In Example 8(2), using 2-thienylmethanol instead of 4-biphenylylmethanol, the compound 8(2A) and the compound 8(2B) were obtained.

### Example 8(1)

1-(2,2-dimethylpropyl)-3-(3-pyridinylmcthoxy)-1H-pyrazolo[3,4-d]pyrimidine-6-carbonitrile:
TLC : Rf 0.36 (hexane : ethyl acetate = 1 : 1);
¹H NMR (CDCl₃) : δ 1.00 (s, 9 H), 4.14 (s, 2 H), 5.47 (s, 2 H), 7.30 - 7.43 (m, 1 H), 7.79 - 7.92 (m, 1 H), 8.63 (dd, J = 4.76, 1.70 Hz, 1 H), 8.78 (d, J = 1.70 Hz, 1 H), 9.07 (s, 1 H).

### Example 8(2)

1-(2,2-dimethylpropyl)-3-(2-thienylmethoxy)-1H-pyrazola[3,4-d]pyrimidine-6-carbonitrile (compound 8(2A)), and 1-(2,2-dimethylpropyl)-3-oxo-2-(2-thienylmethyl)-2,3-dihydro-1H-pyrazolo[3,4-d]pyrimidine-6-carbonitrile (compound 8(2B)):
Compound 8(2A):
TLC : Rf 0.67 (hexane : ethyl acetate = 4 : 1);
¹H NMR (CDCl₃) : δ 1.03 (s, 9 H), 4.15 (s, 2 H), 5.61 (s, 2 H), 7.03 (dd, J = 5.10, 3.48 Hz, 1 H), 7.20 - 7.25 (m, 1 H), 7.38 (dd, J = 5.10, 1.19 Hz, 1 H), 9.04 (s, 1 H).
Compound 8(2B):
TLC : Rf 0.20 (hexane : ethyl acetate = 4 : 1);
¹H NMR (CDCl₃) : δ 0.99 (s, 9 H), 4.03 (s, 2 H), 5.37 (d, J = 0.80 Hz, 2 H), 6.92 (dd, J = 5.12, 3.48 Hz, 1 H), 6.96 - 7.04 (m, 1 H), 7.22 (dd, J = 5.12, 1.28 Hz, 1 H), 9.06 (s, 1 H).

### Example 8(3)

1-(2,2-dimethylpropyl)-3-(2-phenylethoxy)-1H-pyrazolo[3,4-d]pyrimidine-6-carbonitrile:
TLC : Rf 0.36 (hexane : ethyl acetate = 9 : 1);
¹H NMR (CDCl₃) : δ 1.00 (s, 9 H), 3.19 (t, J = 7.14 Hz, 2 H), 4.12 (s, 2 H), 4.62 (t, J = 7.14 Hz, 2 H), 7.21 - 7.40 (m, 5 H), 9.02 (s, 1 H).

### Example 8(4)

1-(2,2-dimethylpropyl)-3-(2-phenoxyethoxy)-1H-pyrazolo[3,4-d]pyrimidine-6-carbonitrile:
TLC : Rf 0.49 (hexane : ethyl acetate = 4 : 1);
¹H NMR (CDCl₃) : δ 1.01 (s, 9 H), 4.14 (s, 2 H), 4.35 - 4.47 (m, 2 H), 4.70 - 4.84 (m, 2 H), 6.88 - 7.05 (m, 3 H), 7.27 - 7.39 (m, 2 H), 9.08 (s, 1 H).

### Example 8(5)

1-(2,2-dimethylpropyl)-3-[2-(4-morpholinyl)ethoxy]-1H-pyrazolo[3,4-d]pyrimidine-6-carbonitrile:
TLC : Rf0.33 (hexane : ethyl acetate = 1 : 3);
¹H NMR (CDCl₃) : δ 1.00 (s, 9 H), 2.50 - 2.68 (m, 4 H), 2.88 (t, J = 5.76 Hz, 2 H), 3.66 - 3.83 (m, 4 H), 4.12 (s, 2 H), 4.55 (t, J = 5.76 Hz, 2 H), 9.06 (s, 1 H).

### Example 9

8-(2,2-dimethylpropyl)-2-(methylthio)pyrazolo[1,5-a][1,3,5]trlazin-4(1H)-one:

### Step (9-A):

To a suspension of molecular sieves 4A powder (MS4A, 2.0 g) in toluene (40 mL) were added piperidine (1.19 mL) and acetic acid (0.69 mL), then the mixture was stirred for 10 minutes, methyl cyanoacetate (3.53 mL) and pivalaldehyde (4.77 mL) were added thereto, the mixture was stirred for 5 hours at 80°C. The reaction mixture was cooled to room temperature, added by ethyl acetate and water. After MS4A was separated by filtration, the filtrate was extracted by ethyl acetate. The organic layer was washed with brine, dried over anhydrous sodium sulfate, concentrated. The obtained residue was purified by column chromatography on silica gel (ethyl acetate : hexane = 1 : 20 → 1 : 10) to give methyl 2-cyano-4,4-dimethyl-2-pentenoate (4.72 g).

### Step (9-B):

Under atmosphere of argon, to a solution of the compound prepared in step (9-A) (3.79 g) in ethyl acetate (76 mL) was added 5% palladium on carbon (192 mg). Under atmosphere of hydrogen, the reaction mixture was stirred for 40 minutes. The atmosphere of reaction mixture was replaced by argon, the mixture was filtrated by cerite (trademark), the filtrate was concentrated to give methyl 2-cyano-4,4-dimethylpentanoate (3.69 g).

### Step (9-C):

Under atmosphere of argon, to a solution of the compound prepared in step (9-B) (1.68 g) in tetrahydrofuran (20 mL) was dropped diisobutylaluminium hydride (1.0 M toluene solution, 10.0 mL) at -74°C, then the mixture was stirred for 2.5 hours at -75°C. The temperature of reaction mixture was raised to -42°C little by little, the mixture was added by methanol (1.5 mL) and 1N hydrochloric acid, then extracted with ethyl acetate. The organic layer was washed with brine, dried over anhydrous sodium sulfate, concentrated to give crude 2-formyl-4,4-dimethylpentanenitrile (1.61 g).

### Step (9-D):

To a solution of the compound prepared in step (9-C) (1.42 g) in ethanol (28 mL) was added hydrazine monohydrate (0.428 mL), then the mixture was refluxed for 1 day. The reaction mixture was cooled to room temperature, concentrated. To the residue were added water, ethyl acetate and 1N hydrochloric acid, then separated. The water layer was washed with ethyl acetate, neutralized by saturated aqueous solution of sodium hydrogen carbonate, then extracted with ethyl acetate. The organic layer was washed with brine, dried over anhydrous sodium sulfate, concentrated to give 4-(2,2-dimethylpropyl)-1H-pyrazol-5-amine (530 mg).

### Step (9-E):

To a solution of the compound prepared in step (9-D) (210 mg) in a mixed solvent of ethyl acetate (4 mL) and benzene (15 mL) on ice bath was dropped a solution of ethyl isothiocyanatoformate (180 mg) in benzene (5 mL), then the mixture was stirred for 1 hour on ice bath, 2 hours at room temperature. The reaction mixture was concentrated. The residue was washed with a mixed solvent of ethyl acetate and hexane to give ethyl ({[4-(2,2-dimethylpropyl)-1H-pyrazol-5-yl]amino}carbonothioyl)carbamate (207 mg).

### Step (9-F):

The compound prepared in step (9-E) (200 mg) was dissolved to 2N aqueous solution of sodium hydroxide (1.5 mL), then stirred for 1 hour at room temperature. The reaction mixture was added by 2N aqueous solution of sulfuric acid to make pH of solution 1, then stirred for 1 hour. The precipitates was collected, then dried to give 8-(2,2-dimethylpropyl)-2-thioxo-2,3-dihydropyrazolo[1,5-a][1,3,5]triazin-4(1H)-one (135 mg).

### Step(9-G):

To a solution the compound prepared in step (9-F) (115 mg) in ethanol (2 mL) was added 2N aqueous solution of sodium hydroxide (0.48 mL), then methyl iodide (0.03 mL) was added thereto at room temperature, the mixture was stirred for 20 minutes. The reaction mixture was concentrated. After the obtained solid was washed with diisopropyl ether, it was dissolved to water (23 mL), added by 2N aqueous solution of sulfuric acid (0.5 mL). The obtained solid was collected, dried to give the title compound (102 mg) having the following physical data.
TLC : Rf 0.47 (dichloromethane : methanol = 9 : 1).

### Example 10

### 4-chloro-8-(2,2-dimethylpropyl)-2-(methylthio)pyrazolo[1,5-a][1,3,5]triazine:

To phosphorus oxychloride (1.9 g) were added the compound prepared in Example 9 (94.5 mg) and N,N-dimethylaniline (3 drops), the mixture was stirred for 3 hours at 120°C. The reaction mixture was poured into iced water, extracted with ethyl acetate. The organic layer was washed with water and brine, dried over anhydrous sodium sulfate, concentrated to give the title compound (109 mg) having the following physical data.
TLC : Rf 0.39 (hexane : ethyl acetate =9:1).

### Example 11

### N,8-bis(2,2-dimethylpropyl)-2-(methylthio)pyrazolo[1,5-a][1,3,8]triazine-4-amine:

To a solution of the compound prepared in Example 10 (98 mg) in tetrahydrofuran (2 mL) were added triethylamine (0.056 mL) and (2,2-dimethylpropyl)amine (0.047 mL), the mixture was stirred overnight at room temperature. The reaction mixture was poured into water, extracted with ethyl acetate. The organic layer was washed with water, dried over anhydrous sodium sulfate, concentrated. The obtained residue was purified by column chromatography on silica gel (ethyl acetate : hexane = 5 : 95 → 15 : 85 → 100 : 0) to give the title compound (68 mg) having the following physical data.
TLC : Rf0.39 (hexane : ethyl acetate = 9 : 1).

### Example 12

### N,8-bis(2,2-dimethylpropyl)-2-(methylsulfonyl)pyrazolo[1,5-a][1,3,5]triazine-4-amine:

To a solution of the compound prepared in Example 11 (65 mg) in dichloromethane (3 mL) on ice bath was added m-chloroperbenzoic acid (128 mg), and the mixture was stirred for 1 hour on ice bath, 1.5 hours at room temperature. The reaction mixture was poured into saturated aqueous solution of sodium hydrogen carbonate, extracted with ethyl acetate. The organic layer was washed with water, dried over anhydrous sodium sulfate, concentrated. The residue was purified by column chromatography on silica gel (parallel preparative purifier system). The obtained solid was dissolved to ethyl acetate, the solution was washed with saturated aqueous solution of sodium sulfite and brine sequentially, dried over anhydrous sodium sulfate, concentrated to give the title compound (65 mg) having the following physical data.
TLC : Rf0.33 (hexane : ethyl acetate = 3 : 1).

### Example 13

### 8-(2,2-dimethylpropyl)-4-[(2,2-dimethylpropyl)amino]pyrazolo[1,5-a][1,3,5]triazine-2-carbonitrile:

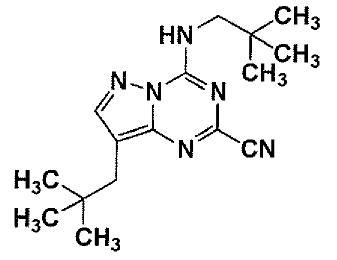

To a solution of the compound prepared in Example 12 (58 mg) in dimethylsulfoxide (1 mL) were added 1,4-diazabicyclo[2.2,2]octane (18 mg), potassium cyanide (30 mg) and water (0.1 mL), then the mixture was stirred for 4 hours at 50°C. The reaction mixture was poured into water, extracted with ethyl acetate. The organic layer was washed with water, dried over anhydrous sodium sulfate, concentrated. The obtained residue was purified by column chromatography on silica gel (parallel preparative purifier system) to give the title compound (44.4 mg) having the following physical data.
TLC : Rf 0.50 (hexane : ethyl acetate = 4 : 1);
¹H NMR (CDCl₃) : δ 0.94 (s, 9 H), 1.05 (s, 9 H), 2.61 (s, 2 H), 3.52 (d, J = 6.59 Hz, 2 H), 6.69 - 6.82 (m, 1 H), 7.91 (s, 1 H).

### Example 14

### 4-[(2,2-dimethylpropyl)amino]pyrazolo[1,5-a][1,3,5]triazine-2-carbonitrile:

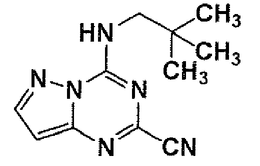

By the same procedures as described in the step (9-G) in Example 9 → Example 10 → Example 11 → Example 12 → Example 13, using 2-thioxo-2,3-dihydropyrazolo[1,5-a][1,3,5]triazin-4(1H)-one instead of the compound prepared in the step (9-F) in the corresponding step of the step (9-G) in Example 9, the title compound having the following physical data was obtained.
TLC : Rf 0.48 (hexane : ethyl acetate = 2 : 1);
¹H NMR (CDCl₃) : δ 1.05 (s, 9 H), 3.53 (d, J = 6.59 Hz, 2 H), 6.62 (d, J = 2.20 Hz, 1 H), 6.76 - 6.93 (m, 1 H), 8.08 (d, J = 2.20 Hz, 1 H).

### Example 15

### ethyl 4-[2-(tert-butoxycarbonyl)-1-(2,2-dimethylpropyl)hydrazino]-2-cyano-5-pyrimidinecarboxylate:

By the same procedures as described in Example 1 → Example 12 → Example 13, using ethyl 4-chloro-2-(methylthio)-5-pyrimidinecarboxylate instead of 2,4-dichlaro-5-(chloromethyl)pyrimidine, and tert-butyl 2-(2,2-dimethylpropyl)hydrazinecarboxylate instead of neopentylamine in the step corresponding to Example 1, the title compound having the following physical data was obtained.
TLC : Rf0.64 (hexane : ethyl acetate =2:1);
¹H NMR (CDCl₃) : δ 1.00 (s, 9 H), 1.16 - 1.53 (m, 12 H), 2.57 - 3,03 (m, 1 H), 4.24 - 4.54 (m, 2 H), 4.58 - 4.99 (m, 1 H), 6.18 - 7.12 (m, 1 H), 8.59 (s, 1 H).

### Example 16

### ethyl 4-{2-(tert-butoxycarbonyl)-1-(2,2-dimethylpropyl)-2-[2-(4-morphalinyl)ethyl]hydrazino}-2-cyano-5-pyrimidinecarboxylate:

To a solution of the compound prepared in Example 15 (189 mg) and 4-(2-chloroethyl) morpholine hydrochloride (186 mg) in dimethylformamide (1 mL) was added potassium carbonate (207 mg), then the mixture was stirred for 14 hours at 90°C. The reaction mixture was added by ethyl acetate and water, extracted with ethyl acetate. The organic layer was washed with water and brine sequentially, dried over anhydrous sodium sulfate, concentrated. The obtained residue was purified by column chromatography on silica gel (hexane : ethyl acetate = 70 : 30 → 60 : 40) to give the title compound (230 mg) having the following physical data.
TLC : Rf0.25 (hexane : ethyl acetate = 6 : 4);
¹H NMR (CDCl₃) : δ 1.05 (s, 9 H), 1.26 (t, J = 7.14 Hz, 3 H), 1.33 (s, 9 H), 2.43 - 2.59 (m, 4 H), 2.60 - 2.81 (m, 2 H), 3.22 - 3.38 (m, 1 H), 3.45 (d, J = 14 Hz, 1 H), 3.71 (t, J = 4.67 Hz, 4 H), 3.73 - 3.86 (m, 1 H), 4.02 (d, J = 14 Hz, 1 H), 4.23 - 4.49 (m, 2 H), 8.33 (s, 1 H).

### Example 17

### 1-(2,2-dimethylpropyl)-2-[2-(4-morpholinyl)ethyl]-3-oxo-2,3-dihydro-1H-pyrazolo[3,4-d]pyrimidine-6-carbonitrile 4-methylbenzenesulfonate:

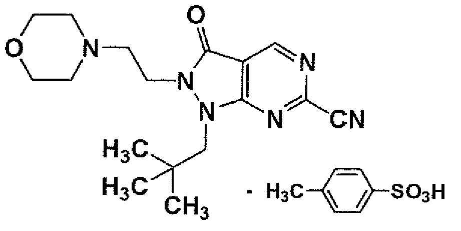

To a solution of the compound prepared in Example 16 (203 mg) in isopropylamine (2 mL) was added p-toluenesulfonic acid monohydrate (157 mg), then the mixture was stirred for 3.5 hours at 90°C. The reaction mixture was cooled to room temperature, the precipitated solid was collected. The solid was washed with isopropylamine (1 mL), dried to give the title compound (169 mg) having the following physical data.
TLC : Rf 0.15 (hexane : ethyl acetate = 1 : 1);
¹H NMR (DMSO-d₆) : δ 0.90 (s, 9 H), 2.28 (s, 3 H), 2.95 - 3.77 (m, 8 H), 3.87 - 4.05 (m, 2 H), 4.08 (s, 2 H), 4.32 - 4.50 (m, 2 H), 7.11 (d, J = 7.68 Hz, 2 H), 7.46 (d, J = 8.05 Hz, 2 H), 9.25 (s, 1 H), 9.58 (s, 1 H).

### Example 18(1) - Example 18(2)

By the same procedures as described in Example 16 → Example 17, using corresponding halide compound instead of 4-(2-chloroethyl)morpholine hydrochloride in the step corresponding to Example 16, the title compound having the following physical data was obtained.

### Example 18(1)

### 1-(2,2-dimethylpropyl)-2-(4-methoxybenzyl)-3-oxo-2,3-dihydro-1H-pyrazolo[3,4-d]pyrimidine-6-carbonitrile:

TLC : Rf 0.51 (hexane : ethyl acetate = 1 : 1);
¹H NMR (CDCl₃) : δ 0.97 (s, 9 H), 3.76 (s, 3 H), 3.91 (s, 2 H), 5.21 (s, 2 H), 6.81 (d, J = 8.70 Hz, 2 H), 7.04 (d, J = 8.70 Hz, 2 H), 9.09 (s, 1 H).

### Example 18(2)

### [6-cyano-1-(2,2-dimethylpropyl)-3-oxo-1,3-dihydro-2H-pyrazolo[3,4-d]pyrimidin-2-yl]acetic acid:

TLC : Rf 0.43 (dichloromethane : methanol: acetic acid = 20 : 2 : 1);
¹H NMR (CDCl₃) : δ 0.98 (s, 9 H), 3.94 (s, 2 H), 4.82 (s, 2 H), 7.98 (s, 1 H), 9.09 (s, 1 H).

### Example 19

### 1-(2,2-dimethylpropyl)-3-ethoxy-1H-pyrazolo[3,4-d]pyrimidine-6-carbonitrile;

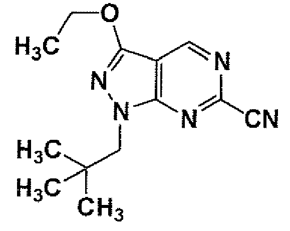

By the same procedures as described in Example 17, using the compound prepared in Example 15 instead of the compound prepared in Example 16, the title compound having the following physical data was obtained.
TLC : Rf0.69 (hexane : ethyl acetate =4:1);
¹H NMR (CDCl₃) : δ 1.00 (s, 9 H), 1.50 (t, J = 7.10 Hz, 2 H), 4.12 (s, 2 H), 4.47 (q, J = 7.10 Hz, 2 H), 9.04 (s, 1H).

### Example 20

### 5-bromo-4-[1-(2,2-dimethylpropyl)hydrazino]-2-pyrimidinecarbonitrile:

By the same procedures as described in Example 1 → Example 3 → Example 17, using 5-bromo-2,4-dichloropyrimidine instead of 2,4-dichloro-5-(chloromethyl)pyrimidine and tert-butyl 2-(2,2-dimethylpropyl)hydrazinecarboxylate instead of neopentylamine in the step corresponding to Example 1, the title compound having the following physical data was obtained.
TLC : Rf 0.57 (hexane : ethyl acetate = 2 : 1);
¹H NMR (DMSO-d₆) : δ 0.94 (s, 9 H), 3.68 (s, 2 H), 5.07 (s, 2 H), 8.45 (s, 1H).

### Example 21

### 1-(2,2-dimethylpropyl)-3-{4-[(4-methyl-1-piperazinyl)methyl]phenyl}-1H-pyrimido[4,5-e][1,3,4]oxadiazine-7-carbonitrile:

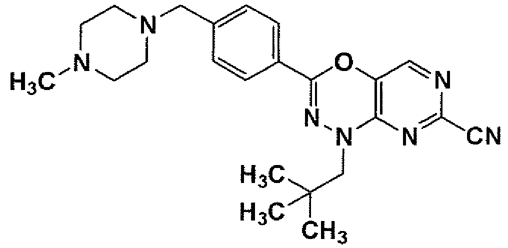

To a solution of the compound prepared in Example 20 (50 mg) and 4-[(4-methyl-1-piperazinyl)methyl]benzoic acid dihydrochloride (135 mg) in dimethylformamide (1.8 mL) were added triethylamine (184 µL), 1-hydroxy-7-azabenzotriazole (HOAt, 60 mg) and 1-ethyl-3-(3-dimethylaminopropyl)carbodiimide hydrochloride (84 mg), then the mixture was stirred for 19 hours at 40°C, 7 hours at 50°C. The reaction mixture was cooled to room temperature, then added by water, extracted with ethyl acetate. The organic layer was washed with brine, dried over anhydrous sodium sulfate, concentrated. The obtained residue was purified by column chromatography on silica gel (ethyl acetate → ethyl acetate : methanol = 10 : 1 → dichloromethane : methanol = 10 : 1), then by preparative thin-layer chromatography (dichloromethane : methanol = 9 : 1) to give the title compound (7 mg) having the following physical data.
TLC : Rf 0.37 (dichloromethane : methanol =10:1);
¹H NMR (CDCl₃) : δ 1.05 (s, 9 H), 2.30 (s, 3 H), 2.34 - 2.73 (m, 8 H), 3.46 (s, 2 H), 3.54 (s, 2 H), 7.3 7 (d, J = 8.42 Hz, 2 H), 7.51 (s, 1 H), 7.67 (d, J = 8.42 Hz, 2 H).

### Example 21(1) - Example 21(2)

By the same procedures as described in Example 21, using corresponding carboxylic acid compound instead of 4-[(4-methyl-1-piperazinyl)methyl]benzoic acid dihydrochlorde, the title compound was obtained.

### Example 21(1)

### 1-(2,2-dimethylpropyl)-3-{4-[1-(2-methoxyethyl)-4-piperidinyl]phenyl}-1H-pyrimido[4,5-e][1,3,4]oxadiazine-7-carbonitrile:

TLC : Rf0.51 (dichloromethane : methanol = 10 : 1);
¹H NMR (CDCl₃) : δ 1.04 (s, 9 H), 1.71 - 1.96 (m, 4 H), 1.98 - 2.23 (m, 2 H), 2.46 - 2.59 (m, 1 H), 2.62 (t, J = 5.58 Hz, 2 H), 3.00 - 3.20 (m, 2 H), 3.37 (s, 3 H), 3.45 (s, 2 H), 3.55 (t, J = 5.58 Hz, 2 H), 7.27 (d, J = 8.30 Hz, 2 H), 7.50 (s, 1 H), 7.66 (d, J = 8.30 Hz, 2 H).

### Example 21(2)

### 1-(2,2-dimethylpropyl)-3-vinyl-1H-pyrimido[4,5-e][1,3,4]oxadiazine-7-carbonitrile:

TLC : Rf0.54 (hexane : ethyl acetate =4:1);
¹H NMR (CDCl₃) : δ 0.99 (s, 9 H), 3.38 (s, 2 H), 5.61 (dd, J = 9.60, 2.10 Hz, 1 H), 5.88 (dd, J = 17.40, 2.10 Hz, 1 H), 5.97 (dd, J = 17.40, 9.60 Hz, 1 H), 7.47 (s, 1 H).

### Example 22

### 2-chloro-N-(2,2-dimethylpropyl)-5-{[(4-methoxybenzyl)amino]methyl}-4-pyrimidineamine:

To a solution of (4-methoxybenzyl)amine(137 mg) in tetrahydrofuran (3 mL) on ice bath was added diisopropylethylamine (192 µL), then the mixture was dropped by a solution of the compound prepared in Example 1 (248 mg) in tetrahydrofuran (2 mL), stirred for 30 minutes at room temperature, 2.5 hours at 50°C, then 4 hours at 60°C. The reaction mixture was added by (4-methoxybenzyl)amine (137 mg) and diisopropylethylamine (192 µL), stirred for 9 hours at 65°C. The reaction mixture was concentrated. The obtained residue was diluted with ethyl acetate, washed with water, saturated aqueous solution of sodium hydrogen carbonate and brine sequentially, dried over anhydrous sodium sulfate, concentrated. The obtained residue was purified by column chromatography on silica gel (hexane : ethyl acetate = 3 : 1) to give the title compound (300 mg) having the following physical data.
TLC : Rf 0.29 (hexane : ethyl acetate = 2 : 1);
¹H NMR (CDCl₃) : δ 0.96 (s, 9 H), 3.31 (d, J = 5.85 Hz, 2 H), 3.64 - 3.72 (m,, 4 H), 3.80 (s, 3 H), 6.87 (d, J = 8.70 Hz, 2 H), 7.18 (d, J = 8.70 Hz, 2 H), 7.69 (s, 1H), 7.73 - 7.89 (m, 1 H).

### Example 23

### 4-[(2,2-dimethylpropyl)amino]-5-{[(4-methoxybenzyl)amino]methyl}-2-pyrimidinecarbonitrile:

By the same procedures as described in Example 3, using the compound prepared in Example 22 instead of the compound prepared in Example 2, the title compound having the following physical data was obtained.
TLC : Rf 0.29 (hexane : ethyl acetate = 2 : 1);
¹H NMR (CDCl₃) : δ 0.96 (s, 9 H), 3.31 (d, J = 6.04 Hz, 2 H), 3.68 (s, 2 H), 3.74 (s, 2 H), 3.81 (s, 3 H), 6.87 (d, J = 8.70 Hz, 2 H), 7.15 (d, J = 8.70 Hz, 2 H), 7.85 (s, 1 H), 7.90 - 8.08 (m, 1 H).

### Example 24

### 9-(2,2-dimethylpropyl)-6-(4-methoxybenzyl)-7-oxo-6,7,8,9-tetrahydro-5H-pyrimido[4,5-e][1,4]diazepine-2-carbonitrile:

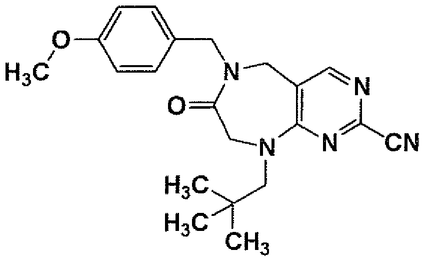

To a solution of the compound prepared in Example 23 (34 mg) in tetrahydrofuran (0.5 mL) on ice bath was added diisopropylethylamine (35 µL), the mixture was added by a solution of chloroacetyl chloride (12 mg) in tetrahydrofuran (0.5 mL), stirred for 1 hour at room temperature. To the reaction mixture were added potassium carbonate (28 mg), potassium iodide (33 mg) and dimethylformamide (0.2 mL), then the mixture was stirred for 2 hours at 55°C, 5 hours at 80°C. The reaction mixture was cooled to room temperature, poured into water, extracted with ethyl acetate. The organic layer was washed with water and brine sequentially, dried over anhydrous sodium sulfate, concentrated. The obtained residue was purified by column chromatography on silica gel (hexane : ethyl acetate = 1 : 2) to give the title compound (19 mg) having the following physical data.
TLC : Rf 0.53 (hexane : ethyl acetate = 1 : 3);
¹H NMR (CDCl₃) : δ 1.02 (s, 9 H), 3.68 (s, 2 H), 3.79 (s, 3 H), 4.35 (s, 2 H), 4.47 (s, 2 H), 4.61 (s, 2 H), 6.81 (d, J = 8.70 Hz, 2 H), 7.12 (d, J = 8.70 Hz, 2 H), 7. 54 (s, 1 H).

### Example 25

### 8-(2,2-dimethylpropyl)-6-(4-methoxybenzyl)-7-oxo-5,6,7,8-tetrahydropyrimido[4,5-d]pyrimidine-2-carbonitrile:

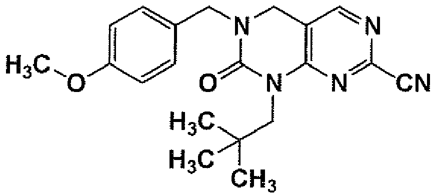

To a solution of tetrahydrofuran (0.5 mL) in triethylamine (42 µL) on ice bath was dropped a solution of triphosgene (10 mg) in tetrahydrofuran (0.3 mL). To the reaction mixture was dropped a solution of the compound prepared in Example 23 (34 mg) in tetrahydrofuran (0.4 mL), the reaction mixture was stirred for 1 hour at room temperature. To the reaction mixture were added triethylamine (42 µL) and triphosgene (8 mg), the mixture was stirred for 5 minutes at room temperature, 30 minutes at 50°C, 21 hours at 70°C. The reaction mixture was cooled to room temperature, poured into water, extracted with ethyl acetate. The organic layer was washed with brine, dried over anhydrous sodium sulfate, concentrated. The obtained residue was purified by column chromatography on silica gel (hexane : ethyl acetate = 4 : 1 → 2 : 1) to give the title compound (20 mg) having the following physical data.
TLC : Rf 0.34 (hexane : ethyl acetate = 2 : 1);
¹H NMR (CDCl₃) : δ 0.94 (s, 9 H), 3.81 (s, 3 H), 4.14 (s, 2 H), 4.25 (s, 2 H), 4.43 - 4.80 (m, 2 H), 6.79 - 6.94 (m, 2 H), 7.20 - 7.27 (m, 2 H), 8.19 (s, 1 H).

### Example 26

### N-[(5Z)-4-chloro-5H-1,2,3-dithiazol-5-ylidene]-2-(2,2-dimethylpropoxy)aniline:

To a solution of [2-(2,2-dimethylpropoxy)phenyl]amine (529 mg) in dichloromethane (20 mL) was added 4,5-dichloro-1,2,3-dithiazol-1-ium chloride (679 mg, this compound is described in Chem Ber., 118, 1632-1643 (1985)) at room temperature, then the mixture was stirred overnight at room temperature. The reaction mixture was added by water and ethyl acetate, extracted with ethyl acetate. The organic layer was washed with water and brine, dried over anhydrous sodium sulfate, concentrated. The obtained residue was purified by column chromatography on silica gel (hexane : ethyl acetate = 1 : 20) to give the title compound (868 mg) having the following physical data.
TLC : Rf 0.74 (hexane : ethyl acetate = 4 : 1).

### Example 27

### 4-(2,2-dimethylpropoxy)-1,3-benzothiazol-2-carbonitrile;

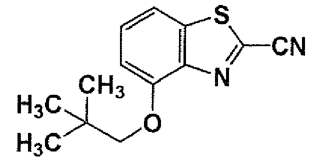

The compound prepared in Example 26 (199 mg) was dissolved to N-methylmorpholine (2 mL), then the mixture was microwaved for 1 minutes (max temperature : 150°C, 90W). The reaction mixture was cooled to room temperature, added by water and ethyl acetate, extracted with ethyl acetate. The organic layer was washed with water and brine sequentially, dried over anhydrous sodium sulfate, concentrated. The obtained residue was purified by column chromatography on silica gel (hexane : ethyl acetate = 1 : 20), then by preparative thin-layer chromatography (hexane : ethyl acetate = 1 : 9) to give the title compound (24.8 mg) having the following physical data.
TLC : Rf 0.42 (hexane : ethyl acetate = 9 : 1);
¹H NMR (CDCl₃) : δ 1.14 (s, 9 H), 3.88 (s, 2 H), 7.00 (dd, J = 7.32, 1.65 Hz, 1 H), 7.46 - 7.56 (m, 2 H).

### Example 28(1) - Example 28(4)

By the same procedures as described in Example 1 → Example 3, using 5,7-dichloropyrazolo[1,5-a]pyrimidine or 2,4-dichloropyrido[2,3-d]pyrimidine instead of 2,4-dichloro-5-(chloromethyl)pyrimidine, and (4-methoxybenzyl)amine instead of neopentylamine in the step corresponding to Example 1, the following compounds are obtained.

### Example 28(1)

### 7-[(4-methoxybenzyl)amino]pyrazolo[1,5-a]pyrimidine-5-carbonitrile:

TLC : Rf 0.38 (hexane : ethyl acetate = 2 : 1);
¹H NMR (CDCl₃) : δ 3.82 (s, 3 H), 4.57 (d, J = 5.67 Hz, 2 H), 6.25 (s, 1 H), 6.66 (d, J = 2.20 Hz, 1 H), 6.86 - 6.97 (m, 1 H), 6.92 (d, J = 8.78 Hz, 2 H), 7.28 (d, J = 8.78 Hz, 2 H), 8.09 (d, J = 2.20 Hz, 1 H).

### Example 28(2)

### 7-[(4-methoxybenzyl)amino]-2-methylpyrazolo[1,5-a]pyrimidine-5-carbonitrile:

TLC : Rf0.43 (hexane : ethyl acetate = 2:1);
¹H NMR (CDCl₃) : δ 2.49 (s, 3 H), 3.83 (s, 3 H), 4.55 (d, J = 5.68 Hz, 2 H), 6.19 (s, 1 H), 6.44 (s, 1 H), 6.77 - 6.87 (m, 1 H), 6.93 (d, J = 8.60 Hz, 2 H), 7.29 (d, J = 8.60 Hz, 2 H).

### Example 28(3)

### 7-[(2,2-dimethylpropyl)amino]pyrazolo[1,5-a]pyrimidine-5-carbonitrile:

TLC : Rf 0.44 (hexane : ethyl acetate = 3 : 1);
¹H NMR (CDCl₃) : δ 1.09 (s, 9 H), 3.22 (d, J = 6.59 Hz, 2 H), 6.26 (s, 1 H), 6.66 (d, J = 2.38 Hz, 1 H), 6.64 - 6.78 (m, 1 H), 8.10 (d, J = 2.38 Hz, 1H).

### Example 28(4)

### 4-[(2,2-dimethylpropyl)amino]pyridn[2,3-d]pyrimidine-2-carbonitrile:

TLC : Rf 0.49 (hexane : ethyl acetate = 1 : 4);
¹H NMR (CDCl₃) : δ 1.06 (s, 9 H), 3.60 (d, J = 6.22 Hz, 2 H), 5.99 - 6.23 (m, 1 H), 7.56 (dd, J = 8.32, 4.30 Hz, 1 H), 8.16 (dd, J = 8.42, 1.83 Hz, 1 H), 9.16 (dd, J = 4.39, 1.83 Hz, 1 H).

### Example 29

### 1-(2,2-dimethylpropyl)-3-[(4-methoxybenzyl)oxy]-1H-pyrazolo[3,4-d]pyrimidine-6-carbonitrile:

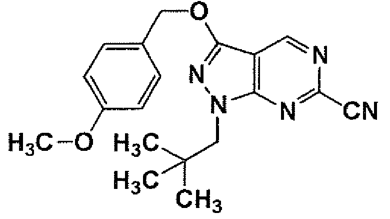

To a solution of the compound prepared in Example 7 (10 mg) in dimethylformamide (0.2 mL) was added potassium carbonate (6.6 mg), then the mixture was stirred for 10 minutes at room temperature. To the reaction mixture was added a solution of p-methoxybenzyl chloride (6.8 mg) in dimethylformamide (0.2 mL), the mixture was stirred for 2 hours at 60°C. The reaction mixture was cooled to room temperature, added by water and 1N hydrochloric acid, extracted with tert-butyl methyl ether. The organic layer was washed with water and brine sequentially, dried over anhydrous sodium sulfate, concentrated. The obtained residue was purified by column chromatography on silica gel (hexane : ethyl acetate = 9 : 1) to give the title compound (14 mg) having the following physical data.
TLC : Rf 0.52 (hexane : ethyl acetate = 4 : 1);
¹H NMR (CDCl₃) : δ 1.01 (s, 9 H), 3.83 (s, 3 H), 4.14 (s, 2 H), 5,37 (s, 2 H), 6.93 (d, J = 8.60 Hz, 2 H), 7.44 (d, J = 8.60 Hz, 2 H), 9.02 (s, 1 H).

### Example 30

### 2-[6-cyano-1-(2,2-dimethylpropyl)-3-oxo-1,3-dihydro-2H-pyrazolo[3,4-d]pyrimidin-2-yl]-N-[2-(dimethylamino)ethyl]acetamide:

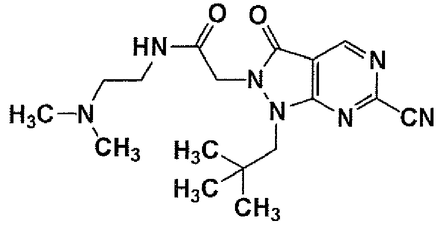

To a solution of the compound prepared in Example 18(2) (58 mg) and N,N-dimethyl-1,2-ethanediamine (21 mg) in dimethylformamide (1 mL) were added 1-hydroxybenzotriazole (HOBt, 30 mg) and 1-ethyl-3-(3-dimethylaminopropyl)carbodiimide hydrochloride (46 mg), then the mixture was stirred for 17 hours at room temperature. The reaction mixture was added by ethyl acetate, water and saturated aqueous solution of sodium hydrogen carbonate, extracted with ethyl acetate. The organic layer was washed with brine, dried over anhydrous sodium sulfate, concentrated. The residue was washed with tert-butyl methyl ether, dried to give the title compound (41.9 mg) having the following physical data.
TLC : Rf 0.50 (dichloromethane : methanol : acetic acid = 10 : 2 : 1);
¹H NMR (CDCl₃) : δ 0.98 (s, 9 H), 2.16 (s, 6 H), 2.31 - 2.44 (m, 2 H), 3.23 - 3.30 (m, 2 H), 4.01 (s, 2 H), 4.68 (s, 2 H), 6.32 - 6.48 (m, 1 H), 9.08 (s, 1H).

### Example 30(1) - Example 30(7)

By the same procedures as described in Example 30, using corresponding amine compounds or alcohol compounds instead of N,N-dimethyl-1,2-ethanediamine, the following compounds were obtained.

### Example 30(1)

### isopropyl [6-cyano-1-(2,2-dimethylpropyl)-3-oxo-1,3-dihydro-2H-pyrazolo[3,4-d]pyrimidin-2-yl]acetate:

TLC : Rf 0.50 (hexane : ethyl acetate = 6 : 4);
¹H NMR (CDCl₃) : δ 0.98 (s, 9 H), 1.22 (d, J = 6.22 Hz, 6 H), 3.76 - 3.94 (m, 2 H), 4.65 - 4.82 (m, 2 H), 4.91 - 5.12 (m, 1 H), 8.90 - 9.33 (m, 1 H).

### Example 30(2)

### tert-butyl [6-cyano-1-(2,2-dimethylpropyl)-3-oxo-1,3-dihydro-2H-pyrazolo[3,4-d]pyrimidin-2-yl]acetate:

TLC : Rf 0.37 (hexane : ethyl acetate = 7 : 3);
¹H NMR (CDCl₃) : δ 0.99 (s, 9 H), 1.40 (s, 9 H), 3.87 (s, 2 H), 4.65 (s, 2 H), 9.07 (s, 1 H).

### Example 30(3)

### ethyl [6-cyano-1-(2,2-dimethylpropyl)-3-oxo-1,3-dihydro-2H-pyrazolo[3,4-d]pyrimidin-2-yl]acetate:

TLC : Rf 0.20 (hexane : ethyl acetate =7:3);
¹H NMR (CDCl₃) : δ 0.98 (s, 9 H), 1.25 (t, J = 7.14 Hz, 3 H), 3.89 (s, 2 H), 4.19 (q, J = 7.14 Hz, 2 H), 4.75 (s, 2 H), 9.08 (s, 1 H).

### Example 30(4)

### 2-[6-cyano-1-(2,2-dimethylpropyl)-3-oxo-1,3-dihydro-2H-pyrazolo[3,4-d]pyrimidin-2-yl]-N-[5-(dimethylamino)pentyl]acetamide:

TLC : Rf 0.50 (dichloromethane : methanol: acetic acid = 10 : 2 : 1);
¹H NMR (CDCl₃) : δ 0.99 (s, 9 H), 1.23 - 1.38 (m, 2 H), 1.38 - 1.57 (m, 4 H), 2.20 (s, 6 H), 2.21 - 2.28 (m, 2 H), 3.12 - 3.29 (m, 2 H), 4.05 (s, 2 H), 4.69 (s, 2 H), 6.55 - 6.69 (m, 1 H), 9.08 (s, 1 H).

### Example 30(5)

### 2-[6-cyano-1-(2,2-dimethylpropyl)-3-oxo-1,3-dihydro-2H-pyrazolo[3,4-d]pyrimidin-2-yl]-N-[4-(dimethylamino)butyl]acetamide:

TLC : Rf 0.50 (dichloromethane : methanol : acetic acid = 10 : 2 : 1);
¹H NMR (CDCl₃): δ 0.99 (s, 9 H), 1.48 - 1.62 (m, 4 H), 2.26 (s, 6 H), 2.27 - 2.34 (m, 2 H), 3.13 - 3.29 (m, 2 H), 4.03 (s, 2 H), 4.66 (s, 2 H), 8.06 - 8.20 (m, 1 H), 9.06 (s, 1 H).

### Example 30(6)

### 2-[6-cyano-1-(2,2-dimethylpropyl)-3-oxo-1,3-dihydro-2H-pyrazolo[3,4-d]pyrimidin-2-yl]-N-[3-(dimethylamino)propyl]acetamide:

TLC : Rf 0.50 (dichloromethane : methanol : acetic acid = 10 : 2 : 1);
¹H NMR (CDCl₃) : δ 0.99 (s, 9 H), 1.49 - 1.77 (m, 2 H), 2.13 (s, 6 H), 2.30 - 2.50 (m, 2 H), 3.27 - 3.37 (m, 2 H), 4.01 (s, 2 H), 4.66 (s, 2 H), 7.85 - 8.08 (m, 1 H), 9.07 (s, 1 H).

### Example 30(7)

### 2-[6-cyano-1-(2,2-dimethylpropyl)-3-oxo-1,3-dihydro-2H-pyrazolo[3,4-d]pyrimidin-2-yl]-N-(4-fluorophenyl)acetamide:

TLC : Rf 0.20 (dichloromethane : ethyl acetate = 1 : 1);
¹H NMR (CDCl₃) : δ 0.99 (s, 9H), 4.02 (s, 2H), 4.90 (s, 2H), 7.10-7.20 (m, 2H), 7.48-7.58 (m, 2H), 9.23 (s, 1H), 10.41 (s, 1H).

### Example 31

### ethyl 4-[(2-aminoethyl)(2,2-dimethylpropyl)amino]-2-(methylthio)-5-pyrimidinecarboxylate 4-methylbenzenesulfonate:

By the same procedures as described in Example 1 → Example 17, using ethyl 4-chloro-2-(methylthio)-5-pyrimidinecarboxylate instead of 2,4-dichloro-5-(chloromethyl)pyrimidine, and tert-butyl {2-[(2,2-dimethylpropyl)amino]ethyl}carbamate instead of neopentylamine in the step corresponding to Example 1, the title compound having the following physical data was obtained.
TLC : Rf 0.46 (ethyl acetate : methanol = 9 : 1);
¹H NMR (CDCl₃) : δ 0.98 (s, 9H), 1.33 (t, J = 6.9 Hz, 3H), 2.35 (s, 3H), 2.58 (s, 3H), 2.70-2.80 (m, 2H), 3.31-3.46 (m, 2H), 4.12-4.25 (m, 2H), 4.32 (q, J = 6.9 Hz, 2H), 7.16 (d, J = 8.4 Hz, 2H), 7.73 (d, J = 8.4 Hz, 2H), 8.41-8.59 (m, 2H), 8.64 (s, 1H), 9.10-9.30 (m, 1H).

### Example 32

### Disodium 4-[(2-aminoethyl)(2,2-dimethylpropyl)amino]-2-(methylthio)-5-pyrimidinecarboxylate 4-methylbenzenesulfonate:

To a solution of the compound prepared in Example 31 (226 mg) in dioxane (4 mL) were added 1N aqueous solution of sodium hydroxide (1.2 mL) and ethanol (1 mL), then the mixture was stirred for 3 hours at 80°C. The reaction mixture was concentrated. The obtained residue was azeotroped with ethanol to give the title compound (282 mg) having the following physical data.
TLC : Rf 0.20 (ethyl acetate : methanol : acetic acid = 8 : 1 : 1);
¹H NMR (DMSO-d₆) : δ 0.84 (s, 9H), 2.28 (s, 3H), 2.40 (s, 3H), 2.62-2.74 (m, 2H), 3.20-3.50 (m, 4H), 7.10 (d, J = 8.1 Hz, 2H), 7.46 (d, J = 8.1 Hz, 2H), 8.29 (s, 1H).

### Example 33

### 9-(2,2-dimethylpropyl)-2-(methylthio)-6,7,8,9-tetrahydro-5H-pyrimido[4,5-e][1,4]diazepin-5-one:

To a solution of the compound prepared in Example 32 (275 mg) and 1-hydroxybenzotriazole (88.8 mg) in dimethylformamide (7 mL) on ice bath was added 1-ethyl-3-(3-dimethylaminopropyl)carbodiimide hydrochloride (116 mg), then the reaction mixture was stirred for overnight with rising to room temperature. The reaction mixture was added by ethyl acetate and saturated aqueous solution of sodium hydrogen carbonate, extracted with ethyl acetate. The organic layer was washed with saturated aqueous solution of sodium hydrogen carbonate and brine sequentially, dried over anhydrous sodium sulfate, concentrated. The obtained residue was purified by column chromatography on silica gel (ethyl acetate : hexane = 1 : 2 → 1 : 1) to give the title compound (94 mg) having the following physical data.
TLC : Rf 0.28 (ethyl acetate : hexane = 1 : 1);
¹H NMR (CDCl₃) : δ 0.99 (s, 9 H), 2.51 (s, 3 H), 3.38 (s, 2 H), 3.58 - 3.71 (m, 4 H), 5.75 - 5.83 (m, 1 H), 8.82 - 8.84 (m, J = 0.73 Hz, 1 H).

### Example 34

### 9-(2,2-dimethylpropyl)-5-oxo-6,7,8,9-tetrahydro-5H-pyrimido[4,5-e][1,4]diazepine-2-carbonitrile:

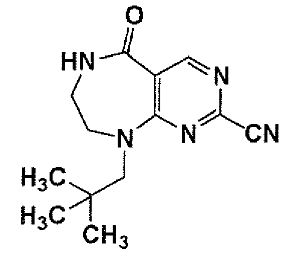

By the same procedures as described in Example 12 → Example 13, using the compound prepared in Example 33 instead of the compound prepared in Example 11 in the step corresponding to Example 12, the title compound having the following physical data was obtained.
TLC : Rf0.60 (hexane : ethyl acetate = 1 : 2);
¹H NMR (CDCl₃) : δ 1.00 (s, 9 H), 3.41 (s, 2 H), 3.66 - 3.73 (m, 4 H), 6.05 - 6.14 (m, 1 H), 8.98 - 9.00 (m, 1 H).

### Example 35

### ethyl 7-chloro-5-methylimidazo[1,2-a]pyrimidin-2-carboxylate:

To a solution of 4-chloro-6-methyl-2-pyrimidineamine (2.87 g) in tetrahydrofuran (50 mL) was added ethyl 3-bromo-2-oxopropanoate (2.51 mL), then the mixture was stirred for 3 hours at room temperature, then refluxed for 6 hours. The reaction mixture was added by ethyl acetate and saturated aqueous solution of sodium hydrogen carbonate, extracted with ethyl acetate. The organic layer was washed with water and brine sequentially, dried over anhydrous sodium sulfate, concentrated. The obtained residue was purified by column chromatography on silica gel (ethyl acetate : hexane = 1 : 1 → 2 : 1 → 3 : 1). The obtained compound was washed with a mixed solvent of ethyl acetate and hexane to give the title compound (1.65 g) having the following physical data.
TLC : Rf 0.29 (ethyl acetate : hexane = 2 : 1);
¹H NMR (CDCl₃) : δ 1.44 (t, J = 7.14 Hz, 3 H), 2.68 (s, 3 H), 4.47 (t, J = 7.14 Hz, 2 H), 6.83 (s, 1 H), 8.06 (s, 1 H).

### Example 36

### Sodium 7-[(2,2-dimethylpropyl)amino]-5-methylimidazo[1,2-a]pyrimidin-2-carboxylate:

By the same procedures as described in Example 1 → Example 32, using the compound prepared in Example 35 instead of 2,4-dichloro-5-(chloromethyl)pyrimidine in the step corresponding to Example 1, the title compound having the following physical data was obtained.
TLC : Rf 0.07 (dichloromethane : methanol = 9 : 1);
¹H NMR (CD₃OD) : δ 0.88 - 1.00 (m, 9 H), 2.50 (s, 3 H), 3.16 - 3.29 (m, 2 H), 6.23 - 6.30 (m, 1 H), 7.59 - 7.67 (m, 1 H).

### Example 37

### 7-[(2,2-dimethylpropyl)amino]-5-methylimidazo[1,2-a]pyrimidin-2-carboxamide:

To a solution of the compound prepared in Example 36 (297 mg) in dimethylformamide (1 mL) on ice bath were added 1-hydroxybenzoiriazole (157 mg) and 1-ethyl-3-(3-dimethylaminopropyl)carbodiimide hydrochloride (210 mg), the mixture was stirred for 5 hours with rising to room temperature. The reaction mixture was added by 28% aqueous solution of ammonia (10 mL), stirred for a while, added by ethyl acetate and water, then extracted with ethyl acetate. The organic layer was washed with saturated aqueous solution of sodium hydrogen carbonate and brine sequentially, dried over anhydrous sodium sulfate, concentrated. The residue was washed with a mixed solvent of ethyl acetate and hexane to give the title compound (83 mg) having the following physical data.
TLC : Rf0.36 (dichloromethane : methanol =9:1);
¹H NMR (CDCl₃) : δ 0.98 (s, 9 H), 2.48 (s, 3 H), 3.34 - 3.44 (m, 2 H), 4.76 - 4.93 (m, 1 H), 5.41 - 5.55 (m, 1H), 5.99 - 6.02 (m, 1H), 7.21 - 7.38 (m, 1 H), 7.76 (s, 1H).

### Example 38

### 7-[(2,2-dimethylpropyl)amino]-5-methylimidazo[1,2-a]pyrimidine-2-carbonitrile:

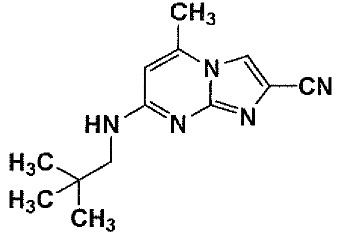

To a solution of the compound prepared in Example 37 (68 mg) in dichloromethane (8 mL) on ice bath were added pyridine (63 µL) and trifluoroacetic anhydride (74 µL), then the mixture was stirred for 3 hours at room temperature. The reaction mixture was added by saturated aqueous solution of sodium hydrogen carbonate, extracted with ethyl acetate. The organic layer was washed with saturated aqueous solution of sodium hydrogen carbonate and brine, dried over anhydrous sodium sulfate, concentrated. The obtained residue was purified by column chromatography on silica gel (ethyl acetate : hexane = 1 : 1 → 2 : 1), then the obtained compound was dissolved to methanol, then concentrated to give the title compound (52.6 mg) having the following physical data.
TLC : Rf0.42 (hexane : ethyl acetate = 1 : 2);
¹H NMR (CDCl₃) : δ 0.97 (s, 9 H), 2.49 (d, J = 0.91 Hz, 3 H), 3.27 - 3.47 (m, 2 H), 4.80 - 5.14 (m, 1 H), 6.03 - 6.11 (m, 1H), 7.59 (s, 1H).

### Example 39

### methyl 3-amino-4-(2,2-dimethylpropyl)-1H-pyrrole-2-carboxylate:

Under atmosphere of argon, methyl 2-cyano-4,4-dimethylpentanoate (1.61 g) was dissolved to dry tetrahydrofuran (20 mL), the mixture was cooled to -70°C, diisobutylaluminium hydride (1.01M toluene solution, 9.42 mL) was slowly dropped thereto, the mixture was stirred for 2 hours at -70°C, then stirred for 1.5 hours with rising to -34°C. The reaction mixture was added by methanol (0.77 mL), and 1N hydrochloric acid, extracted with ethyl acetate. The organic layer was washed with 1N hydrochloric acid and brine sequentially, dried over anhydrous sodium sulfate, concentrated to give crude 2-formyl-4,4-dimethylpentanenitrile (1.58 g).

Under atmosphere of argon, to a solution of 2-formyl-4,4-dimethylpentanenitrile (1.58 g) in methanol (50 mL) were added dimethyl aminomalonate hydrochloride (2.62 g) and a solution of sodium acetate (1.17 g) in water (10 mL) at room temperature, then the mixture was stirred overnight at room temperature. The reaction mixture was concentrated, added by ethyl acetate and water, extracted with ethyl acetate. The organic layer was washed with water and brine sequentially, dried over anhydrous sodium sulfate, concentrated to give crude dimethyl {[(1Z)-2-cyano-4,4-dimethyl-1-penten-1-yl]amino}malonate (2.09 g). To a solution of dimethyl {[(1Z)-2-cyano-4,4-dimethyl-1-penten-1-yl]amino}malonate (2.08 g) in methanol (50 mL) was added sodium methoxide (28% methanol solution, 916 mg), the mixture was stirred for 2 hours at room temperature, then refluxed for 30 minutes. The reaction mixture was cooled to room temperature, concentrated. The obtained residue was added by diisopropylether and water, extracted with diisopropylether. The organic layer was washed with water and brine sequentially, dried over anhydrous sodium sulfate, concentrated. The obtained residue was purified by column chromatography on silica gel (ethyl acetate : hexane = 1 : 4) to give the title compound (518 mg) having the following physical data.
TLC : Rf 0.47 (ethyl acetate : hexane = 1 : 2);
¹H NMR (CDCl₃) : δ 0.92 (s, 9H), 2.21 (s, 2H), 3.83 (s, 3H), 4.00-4.55 (m, 2H), 6.42-6.64 (m, 1H), 7.75-8.32 (m, 1H).

### Example 40

### methyl 3-[(tert-butoxycarbonyl)amino]-4-(2,2-dimethylpropyl)-1H-pyrrole-2-carboxylate:

Under atmosphere of argon, to the compound prepared in Example 39 (373 mg) was added a solution of di-tert-butyl dicarbonate (635 mg) in dioxane (4.5 mL), the mixture was stirred for 11.5 hours at 90°C. The reaction mixture was cooled to room temperature, then concentrated. The obtained residue was purified by column chromatography on silica gel (ethyl acetate : hexane = 1 : 4 → 1 : 3) to give the title compound (401 mg) having the following physical data.
TLC : Rf 0.18 (ethyl acetate : hexane = 1 : 4);
¹H NMR (CDCl₃) : δ 0.86 (s, 9 H), 1.47 - 1.52 (m, 9 H), 2.47 (s, 2 H), 3.84 (s, 3 H), 6.44 - 6.86 (m, 1H), 6.63 (d, J = 3.11 Hz, 1 H), 8.52 - 8.78 (m, 1H).

### Example 41

### methyl 3-[(tert-butoxycarbonyl)amino]-4-(2,2-dimethylpropyl)-1-methyl-1H-pyrrole-2-carboxylate:

To a solution of the compound prepared in Example 40 (299 mg) in dimethylformamide (2 mL) were added potassium carbonate (173 mg) and methyl iodide (78 µL), then the mixture was stirred overnight at room temperature. The reaction mixture was concentrated. The obtained residue was added by ethyl acetate and water, extracted with ethyl acetate. The organic layer was washed with water and brine sequentially, dried over anhydrous sodium sulfate, concentrated. The obtained residue was purified by column chromatography on silica gel (ethyl acetate : hexane = 1 : 4 → 1 : 3) to give the title compound (246 mg) having the following physical data.
TLC : Rf 0.36 (ethyl acetate : hexane = 1 : 4);
¹H NMR (CDCl₃) : δ 0.86 (s, 9 H), 1.48 (s, 9 H), 2.41 (s, 2 H), 3.81 (s, 3 H), 3.83 (s, 3 H), 6.47 (s, 1 H), 6.55 - 6.95 (m, 1 H).

### Example 42

### tert-butyl [2-(aminocarbonyl)-4-(2,2-dimethylpropyl)-1-methyl-1H-pyrral-3-yl]carbamate:

By the same procedures as described in Example 32 → Example 37, using the compound prepared in Example 41 instead of the compound prepared in Example 31 in the step corresponding to Example 32, the title compound having the following physical data was obtained.
TLC : Rf0.38 (ethyl acetate : hexane = 1 : 1);
¹H NMR (DMSO-d₆) : δ 0.81 (s, 9H), 1.40 (s, 9H), 2.13 (s, 2H), 3.68 (s, 3H), 6.58 (s, 1H), 6.70-7.30 (m, 2H), 8.12-8.26 (m, 1H).

### Example 43

### 3-amino-4-(2,2-dimethylpropyl)-1-methyl-1H-pyrrole-2-carboxamide:

To a solution of the compound prepared in Example 42 (13.4 mg) in dichloromethane (0.3 mL) was added trifluoroacetic acid (0.1 mL), then the mixture was stirred for 1.5 hours at room temperature. The reaction mixture was concentrated. The obtained residue was added by ethyl acetate and saturated aqueous solution of sodium hydrogen carbonate, extracted with ethyl acetate. The organic layer was washed with brine, dried over anhydrous sodium sulfate, concentrated to give the title compound (13.4 mg) having the following physical data.
TLC : Rf 0.56 (ethyl acetate : methanol = 9 : 1);
¹H NMR (CDCl₃) : δ 0.91 (s, 9H), 2.22 (s, 2H), 2.60-3.60 (m, 2H), 3.86 (s, 3H), 6.37 (s, 1H), 6.40-6.66 (m, 2H).

### Example 44

### 7-(2,2-dimethylpropyl)-5-methyl-2-thioxo-1,2,3,5-tetrahydro-4H-pyrrolo[3,2-d]pyrimidin-4-one:

To a solution of the compound prepared in Example 43 (8.7 mg) in dimethylformamide (0.3 mL) were added carbon disulfide (2.5 µL) and sodium hydroxide (1.9 mg), then the mixture was stirred for 1 day at room temperature. The reaction mixture was added by diluted hydrochloric acid and brine, extracted with ethyl acetate. The organic layer was washed with brine, dried over anhydrous sodium sulfate, concentrated to give the title compound (11.0 mg) having the following physical data.
TLC : Rf 0.61 (ethyl acetate : hexane =1:1);
¹H NMR (CDCl₃) : δ 0.93 (s, 9 H), 2.30 (s, 2 H), 3.97 (s, 3 H), 6.73 (s, 1 H), 8.78 - 9.07 (m, 2H).

### Example 45

### 7-(2,2-dimethylpropyl)-4-methoxy-5-methyl-2-(methylthio)-5H-pyrrolo[3,2-d]pyrimidine:

To a solution of the compound prepared in Example 44 (11.0 mg) in dimethylformamide (0.3 mL) were added potassium carbonate (13.2 mg) and methyl iodide (5.7 µL), then the mixture was stirred for 3 hours at room temperature. The reaction mixture was added by 1N hydrochloric acid and brine, extracted with ethyl acetate. The organic layer was washed with brine, dried over anhydrous sodium sulfate, concentrated to give the crude title compound having the following physical data.
TLC : Rf 0.61 (ethyl acetate : hexane = 1 : 3);
¹H NMR (CDCl₃) : δ 0.91 (s, 9 H), 2.51 (s, 2 H), 2.60 (s, 3 H), 3.55 (s, 3 H), 4.03 (s, 3 H), 6.75 (s, 1 H).

### Example 46

### 7-(2,2-dimethylpropyl)-4-methoxy-5-methyl-5H-pyrrolo[3,2-d]pyrimidine-2-carbonitrile:

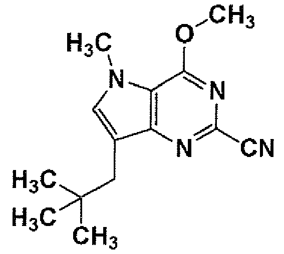

By the same procedures as described in Example 12 → Example 13, using the compound prepared in Example 45 instead of the compound prepared in Example 11 in the step corresponding Example 12, the title compound having the following physical data was obtained.
TLC : Rf 0.32 (hexane : ethyl acetate = 4 : 1);
¹H NMR (CDCl₃) : δ 0.91 (s, 9 H), 2.56 (s, 2 H), 3.78 (s, 3 H), 4.10 (s, 3 H), 6.91 (s, 1 H).

### Example 47

### 7-(2,2-dimethylpropyl)-4-[(2,2-dimethylpropyl)amino]-5-methyl-5H-pyrrolo[3,2-d]pyrimidine-2-carbonitrile:

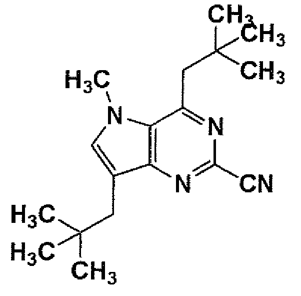

By the same procedures as described in step (9-G) of Example 9 → Example 10 → Example 11 → Example 12 → Example 13, using the compound prepared in Example 44 instead of the compound prepared in step (9-F) in the step corresponding to the step (9-G) of Example 9, the title compound having the following physical data was obtained.
TLC : Rf 0.48 (hexane : ethyl acetate = 2 : 1);
¹H NMR (CDCl₃) δ 0.91 (s, 9 H), 1.02 (s, 9 H), 2.61 (s, 2 H), 3.48 (d, J = 5.85 Hz, 2 H), 4.08 (s, 3 H), 5.01 - 5.17 (m, 1 H), 6.90 (s, 1 H).

### Example 48

### 2-chloro-N-(2,2-dimethylpropyl)-1H-purine-6-amine:

To a suspension of 2,6-dichloropurine (700 mg) in butanol (10 mL) was added neopentylaznine (0.96 mL), then the mixture was stirred for 1 hour at 100°C. The reaction mixture was cooled to room temperature, the precipitate was collected. The obtained crystals were washed with tert-butyl methyl ether to give the title compound (565 mg) having the following physical data.
TLC : Rf 0.66 (chloroform : methanol = 5 : 1).

### Example 49

### 2-chloro-N-(2,2-dimethylpropyl)-9-trityl-9H-purine-6-amine;

The compound prepared in Example 48 (300 mg) and potassium carbonate (190 mg) were suspended to dimethylformamide (4 mL), the mixture was stirred for 30 minutes at room temperature. To the reaction mixture was added trityl chloride (384 mg), then the mixture was stirred for 4 hours at room temperature. The reaction mixture was added by trityl chloride (77 mg), stirred for 30 minutes at 50°C. The reaction mixture was cooled to room temperature, poured into water, extracted with chloroform. The organic layer was washed with brine, dried over anhydrous sodium sulfate, concentrated. The residue was azeotroped with toluene, washed with tert-butyl methyl ether to give the title compound (558 mg) having the following physical data.
TLC : Rf 0.65 (hexane : ethyl acetate = 1 : 1).

### Example 50

### 6-[(2,2-dimethylpropyl)amino]-1H-purine-2-carbonitrile:

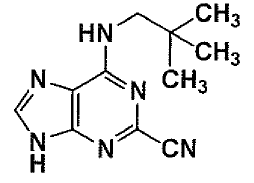

To a suspension of the compound prepared in Example 49 (200 mg) in dimethylsulfoxide (1.5 mL) were added potassium cyanide (54 mg), 1,4-diazabicyclo[2.2.2]octane (14 mg) and water, then the mixture was stirred for 3.5 hours at 150°C. The reaction mixture was poured into water, extracted with ethyl acetate. The organic layer was washed with brine, dried over anhydrous sodium sulfate, concentrated. The residue was washed with tert-butyl methyl ether to give the title compound (45 mg) having the following physical data.
TLC : Rf 0.66 (chloroform : methanol = 5 : 1).

### Example 51

### 6-[(2,2-dimethylpropyl)amino]-9-trityl-9H-purine-2-carbonitrile:

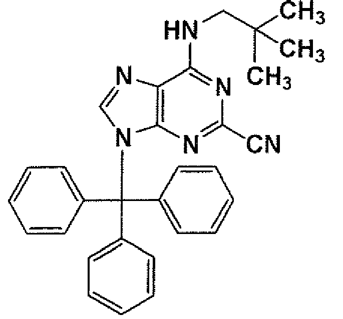

By the same procedures as described in Example 49, using the compound prepared in Example 50 instead of the compound prepared in Example 48, the title compound having the following physical data was obtained.
TLC : Rf 0.29 (hexane : ethyl acetate : methanol = 6 : 2 : 0.1).

### Example 52

### 6-[(2,2-dimethylpropyl)amino]-9-methyl-9H-purine-2-carbonitrile:

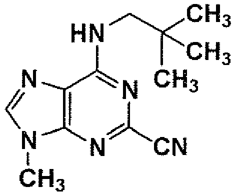

By the same procedures as described in Example 49 → Example 50, using methyl iodide instead of trityl chloride in the step corresponding to Example 49, the title compound having the following physical data was obtained.
TLC : Rf 0.59 (chloroform : methanol = 9 : 1).

### Example 53

### tert-butyl [7-cyano-1-(2,2-dimethylpropyl)-2-oxo-1,4-dihydropyrimido[4,5-d]pyrimidin-3(2H)-yl]acetate:

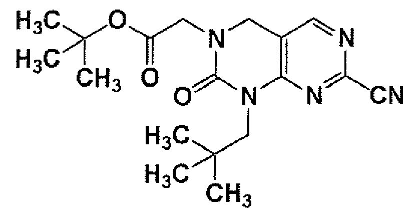

Under atmosphere of argon, to a solution of the compound prepared in Example 5 (53.8 mg) in dimethylformamide (4.5 mL) were added potassium carbonate (207 mg) and tert-butyl bromoacetate (38 µL), then the mixture was stirred overnight at room temperature. The reaction mixture was added by ethyl acetate and water, extracted with ethyl acetate. The organic layer was washed with water and brine, dried over anhydrous sodium sulfate, concentrated. The obtained residue was purified by column chromatography on silica gel (ethyl acetate : hexane = 1 : 4 → 1 : 3 → 1 : 2) to give the title compound (74.3 mg) having the following physical data.
TLC : Rf 0.57 (ethyl acetate : hexane = 1 : 2);
¹H NMR (CDCl₃) : δ 0.93 (s, 9 H), 1.48 (s, 9 H), 3.95 - 4.22 (m, 4 H), 4.43 - 4.68 (m, 2 H), 8.28 - 8.30 (m, 1 H).

### Example 53(1)

### 6-(4-biphenylylmethyl)-8-(2,2-dimethylpropyl)-7-oxo-5,6,7,8-tetrahydropyrimido[4,5-d]pyrimidine-2-carbanitrile:

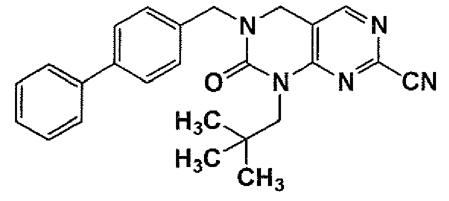

By the same procedures as described in Example 53, using biphenylylmethyl bromide instead of tert-butyl bromoacetate, the title compound having the following physical data was obtained.
TLC : Rf 0.24 (ethyl acetate : hexane = 1 : 3);
¹H NMR (CDCl₃) : δ 0.96 (s, 9 H), 4.13 - 4.20 (m, 2 H), 4.30 - 4.37 (m, 2 H), 4.68 - 4.79 (m, 2 H), 7.29 - 7.49 (m, 5 H), 7.52 - 7.64 (m, 4 H), 8.23 (s, 1 H).

### Example 54

### [7-cyano-1-(2,2-dimethylpropyl)-2-oxo-1,4-dihydropyrimido[4,5-d]pyrimidin-3(2H)-yl]acetic acid:

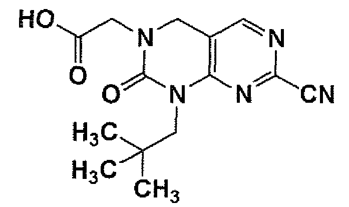

To a solution of the compound prepared in Example 53 (58.0 mg) in tert-butanol (1 mL) was added p-toluenesulfonicacid monohydrate (46 mg), then the mixture was stirred for 2 days at 80°C. The reaction mixture was cooled to room temperature, concentrated. The obtained residue was washed with diisopropyl ether to give the crude title compound (63.4 mg). The obtained residue (51 mg) was purified by column chromatography on silica gel (ethyl acetate : methanol : acetic acid = 50 : 1 : 1) to give the title compound (34.7 mg) having the following physical data.
TLC : Rf0.38 (ethyl acetate : methanol : acetic acid =18:1:1);
¹H NMR (CDCl₃) : δ 0.92 (s, 9 H), 3.98 - 4.37 (m, 4 H), 4.41 - 4.70 (m, 2 H), 8.31 (s, 1 H).

### Example 55(1) - Example 55(16)

By the same procedures as described in Example 30, using the compound prepared in Example 54 instead of the compound prepared in Example 18(2), N,N-dimethyl-1,2-ethanediamine or corresponding amine compound instead of it, the following compounds were obtained.

### Example 55(1)

### 2-[7-cyano-1-(2,2-dimethylpropyl)-2-oxo-1,4-dihydropyrimido[4,5-d]pyrimidin-3 (2H)-yl]-N-[2-(dimethylamino)ethyl]acetamide:

TLC : Rf 0.31 (dichloromethane : methanol : acetic acid = 8 : 1 : 1);
¹H NMR (CDCl₃) : δ 0.93 (s, 9 H), 2.23 (s, 6 H), 2.36 - 2.52 (m, 2 H), 3.27 - 3.42 (m, 2 H), 3.97 - 4.22 (m, 4 H), 4.50 - 4.68 (m, 2 H), 6.46 - 6.61 (m, 1 H), 8.29 (s, 1 H).

### Example 55(2)

### N-benzyl-2-[7-cyano-1-(2,2-dimethylpropyl)-2-oxo-1,4-dihydropyrimido[4,5-d]pyrimidin-3(2H)-yl]acetamide:

TLC : Rf 0.16 (ethyl acetate : hexane=1:1);
¹H NMR (CDCl₃) : δ 0.89 (s, 9 H), 3.95 - 4.20 (m, 4 H), 4.44 (d, J = 5.85 Hz, 2 H), 4.53 - 4.71 (m, 2 H), 6.23 - 6.35 (m, 1 H), 7.20 - 7.39 (m, 5 H), 8.28 (s, 1 H).

### Example 55(3)

### 8-(2,2-dimethylpropyl)-7-oxo-6-[2-oxo-2-(1-pyrrolidinyl)ethyl]-5,6,7,8-tetrahydropyrimido[4,5-d]pyrimidine-2-carbonitrile:

TLC : Rf0.26 (ethyl acetate);
¹H NMR (CDCl₃) : δ 0.93 (s, 9 H), 1.76 - 2.12 (m, 4 H), 3.38 - 3.56 (m, 4 H), 3.90 - 4.32 (m, 4 H), 4.41 - 4.75 (m, 2 H), 8.25 (s, 1 H).

### Example 55(4)

### 2-[7-eyano-1-(2,2-dimethylpropyl)-2-oxo-1,4-dihydropyrimido[4,5-d]pyrimidin-3(2H)-yl]-N-phenylacetamide:

TLC : Rf 0.19 (ethyl acetate : hexane = 1 : 1);
¹H NMR (CDCl₃) : δ 0.94 (s, 9 H), 3.96 - 4.36 (m, 4 H), 4.59 - 4.75 (m, 2 H), 7.07 - 7.17 (m, 1 H), 7.28 - 7.38 (m, 2H), 7.42 - 7.53 (m, 2 H), 8.01 - 8.16 (m, 1 H), 8.33 (s, 1 H).

### Example 55(5)

### 8-(2,2-dimethylpropyl)-6-[2-(4-morpholinyl)-2-oxoethyl]-7-oxo-5,6,7,8-tetrahydropyrimido[4,5-d]pyrimidine-2-carbonitrile:

TLC : Rf 0.15 (ethyl acetate);
¹H NMR (CDCl₃) : δ 0.93 (s, 9 H), 3.43 - 3.52 (m, 2 H), 3.58 - 3.66 (m, 2 H), 3.67 - 3.76 (m, 4 H), 4.03 - 4.14 (m, 2 H), 4.14 - 4.37 (m, 2 H), 4.48 - 4.69 (m, 2 H), 8.27 (s, 1 H).

### Example 55(6)

### 2-[7-cyano-1-(2,2-dimethylpropyl)-2-oxo-1,4-dihydropyrimido[4,5-d]pyrimidin-3(2H)-yl]-N-[3-(dimethylamino)propyl]acetamide:

TLC : Rf 0.35 (dichloromethane : methanol : acetic acid = 8 : 1 : 1);
¹H NMR (CDCl₃) : δ 0.93 (s, 9 H), 1.57 - 1.75 (m, 2 H), 2.14 (s, 6 H), 2.32 - 2.43 (m, 2 H), 3.29 - 3.44 (m, 2 H), 3.98 - 4.18 (m, 4 H), 4.48 - 4.68 (m, 2 H), 7.54 - 7.66 (m, 1 H), 8.29 (s, 1 H).

### Example 55(7)

### 2-[7-cyano-1-(2,2-dimethylpropyl)-2-oxo-1,4-dihydropyrimido[4,5-d]pyrimidin-3(2H)-yl]-N-[4-(dimethylamino)butyl]acetamide:

TLC : Rf 0.26 (dichloromethane : methanol : acetic acid = 8 : 1 : 1);
¹H NMR (CDCl₃) : δ 0.92 (s, 9 H), 1.46 - 1.63 (m, 4 H), 2.24 (s, 6 H), 2.26 - 2.38 (m, 2 H), 3.17 - 3.32 (m, 2 H), 3.88 - 4.21 (m, 4 H), 4.46 - 4.73 (m, 2 H), 7.29 - 7.46 (m, 1H), 8.28 (s, 1H).

### Example 55(8)

### 2-[7-cyano-1-(2,2-dimethylpropyl)-2-oxo-1,4-dihydropyrimido[4,5-d]pyrimidin-3(2H)-yl]-N-[5-(dimethylamino)pentyl]acetamide:

TLC : Rf 0.40 (dichloromethane : methanol : acetic acid = 8 : 1 : 1);
¹H NMR (CDCl₃) : δ 0.92 (s, 9 H), 1.20 - 1.61 (m, 6 H), 2.15 - 2.35 (m, 8 H), 3.18 - 3.33 (m, 2 H), 3.86 - 4.25 (m, 4 H), 4.46 - 4.73 (m, 2 H), 6.07 - 6.24 (m, 1 H), 8.29 (s, 1 H).

### Example 55(9)

### 2-[7-cyano-1-(2,2-dimethylpropyl)-2-oxo-1,4-dihydropyrimido[4,5-d]pyrimidin-3(2H)-yl]acetamide:

TLC : Rf0.10 (ethyl acetate);
¹H NMR (CDCl₃) δ 0.93 (s, 9 H), 3.98 - 4.20 (m, 4 H), 4.48 - 4.69 (m, 2 H), 5.27 - 5.52 (m, 1 H), 5.77 - 6.03 (m, 1 H), 8.30 (s, 1H).

### Example 55(10)

### 2-[7-cyano-1-(2,2-dimethylpropyl)-2-oxo-1,4-dihydropyrimido[4,5-d]pyrimidin-3(2H)-yl]-N-methylacetamide:

TLC : Rf 0.12 (ethyl acetate);
¹H NMR (CDCl₃) : δ 0.90 - 0.98 (m, 9 H), 2.83 (d, J = 4.94 Hz, 3 H), 3.90 - 4.23 (m, 4 H), 4.48 - 4.73 (m, 2 H), 5.89 - 6.06 (m, 1 H), 8.29 (s, 1 H).

### Example 55(11)

### 2-[7-cyano-1-(2,2-dimethylpropyl)-2-oxo-1,4-dihydropyrimido[4,5-d]pyrimidin-3(2H)-yl]-N,N-dimethylacetamide:

TLC : Rf0.14 (ethyl acetate);
¹H NMR (CDCl₃) : δ 0.93 (s, 9 H), 2.98 (s, 3 H), 3.03 (s, 3 H), 3.99 - 4.36 (m, 4 H), 4.42 - 4.70 (m, 2 H), 8.25 (s, 1 H).

### Example 55(12)

### 2-[7-cyano-1-(2,2-dirnethylpropyl)-2-oxo-1,4-dihydropyrimido[4,5-d]pyrimidin-3(2H)-yl]-N-(2-phenylethyl)acetamide:

TLC : Rf0.26 (ethyl acetate : hexane = 2 : 1);
¹H NMR (CDCl₃) : δ 0.91 (s, 9 H), 2.74 - 2.90 (m, 2 H), 3.46 - 3.63 (m, 2 H), 3.88 - 4.17 (m, 4 H), 4.35 - 4.59 (m, 2 H), 5.83 - 6.02 (m, 1 H), 7.07 - 7.38 (m, 5 H), 8.23 (s, 1 H).

### Example 55(13)

### 2-[7-cyano-1-(2,2-dimethylpropyl)-2-oxo-1,4-dihydropyrimido[4,5-d]pyrimidin-3(2H)-yl]-N-(3-phenylpropyl)acetamide:

TLC : Rf0.23 (ethyl acetate : hexane = 2 : 1);
¹H NMR (CDCl₃) : δ 0.92 (s, 9 H), 1.74 - 1.94 (m, 2 H), 2.56 - 2.72 (m, 2 H), 3.21 - 3.38 (m, 2 H), 3.88 - 4.20 (m, 4 H), 4.48 - 4.66 (m, 2 H), 5.90 - 6.03 (m, 1 H), 7.09 - 7.35 (m, 5 H), 8.28 (s, 1 H).

### Example 55(14)

### 8-(2,2-dimethylpropyl)-6-[2-(4-methyl-1-piperazinyl)-2-oxoethyl]-7-oxo-5,6,7,8-tetrahydropyrimido[4,5-d]pyrimidine-2-carbonitrile:

TLC : Rf 0.22 (dichloromethane : methanol: acetic acid =8:1:1);
¹H NMR (CDCl₃) : δ 0.93 (s, 9 H), 2.32 (s, 3 H), 2.35 - 2.50 (m, 4 H), 3.43-3.51 (m, 2 H), 3.59 - 3.67 (m, 2 H), 3.96 - 4.36 (m, 4 H), 4.42 - 4.71 (m, 2 H), 8.25 (s, 1 H).

### Example 55(15)

### 6-[2-(4-benzyl-1-piperazinyl)-2-oxoethyl]-8-(2,2-dimethylpropyl)-7-oxo-5,6,7,8-tetrahydropyrimido[4,5-d]pyrimidine-2-carbonitrile:

TLC : Rf 0.66 (dichloromethane : methanol : acetic acid = 8 : 1 : 1);
¹H NMR (CDCl₃) : δ 0.93 (s, 9 H), 2.34 - 2.57 (m, 4 H), 3.38 - 3.49 (m, 2 H), 3.53 (s, 2 H), 3.57 - 3.68 (m, 2 H), 3.97 - 4.42 (m, 4 H), 4.40 - 4.74 (m, 2 H), 7.19 - 7.38 (m, 5 H), 8.25 (s, 1 H).

### Example 55(16)

### 2-[7-cyano-1-(2,2-dimethylpropyl)-2-oxo-1,4-dihydropyrimido[4,5-d]pyrimidin-3(2H)-yl]-N-(2,2-dimethylpropyl)acetamide:

TLC : Rf 0.29 (ethyl acetate : hexane = 2 : 1);
¹H NMR (CDCl₃) : δ 0.90 (s, 9 H), 0.92 (s, 9 H), 3.08 (d, J = 6.40 Hz, 2 H), 3.93 - 4.22 (m, 4 H), 4.53 - 4.71 (m, 2 H), 5.99 - 6.16 (m, 1 H), 8.31 (s, 1 H).

### Example 56

### 1-[(2-methyl-2-propen-1-yl)oxy]-2-nitrobenzene:

Under atmosphere of argon, to a solution of 2-nitrophenol (2.78 g) in dimethylformamide (10 mL) were added 3-bromo-2-methyl-1-propene (2.12 mL) and potassium carbonate (3.04 g), then the mixture was stirred overnight at room temperature. The reaction mixture was added by ethyl acetate and water, extracted with ethyl acetate. The organic layer was washed with water and brine sequentially, dried over anhydrous sodium sulfate, concentrated to give the title compound (3.94 g) having the following physical data.
TLC : Rf 0.36 (ethyl acetate : hexane = 1 : 9);
¹H NMR (CDCl₃) : δ 1.84 - 1.86 (m, 3 H), 4.57 (s, 2 H), 5.01 - 5.05 (m, 1 H), 5.14 - 5.17 (m, 1 H), 6.98 - 7.09 (m, 2 H), 7.46 - 7.54 (m, 1 H), 7.81 - 7.87 (m, 1 H).

### Example 57

### 2-(2-methyl-2-propen-1-yl)-6-nitrophcnol:

Under atmosphere of argon, the compound prepared in Example 56 (3.09 g) was warmed on oil bath (190°C) without solvent, then stirred for 6 hours. The reaction mixture was cooled to room temperature. The obtained residue was purified by column chromatography on silica gel (ethyl acetate : hexane = 1 : 30 → 1 : 10) to give the title compound (1.64 g) having the following physical data.
TLC : Rf 0.69 (ethyl acetate : hexane =1:9);
¹H NMR (CDCl₃) : δ 1.74 - 1.77 (m, 3 H), 3.44 (s, 2 H), 4.67 - 4.70 (m, 1 H), 4.85 - 4.89 (m, 1 H), 6.93 (dd, J = 8.60, 7.32 Hz, 1 H), 7.44 - 7.49 (m, 1 H), 8.01 (dd, J = 8.60, 1.74 Hz, 1H), 10.95(s, 1H).

### Example 58

### 2-amino-6-isobutylphenol:

Under atmosphere of argon, the compound prepared in Example 57 (1.35 g) was dissolved to methanol (13.5 mL), the mixture was degassed, then 10% palladium on carbon (50%wet, 138 mg) was added thereto. Under atmosphere of hydrogen, the mixture was stirred for 4 hours at room temperature. After the hydrogen was replaced to argon, the reaction mixture was filtrated with cerite (trademark), filtrate was concentrated to give the title compound (1.15 g) having the following physical data.
TLC : Rf 0.63 (ethyl acetate : hexane = 1 : 1);
¹H NMR (CDCl₃) : δ 0.94 (d, J = 6.59 Hz, 5 H), 1.82 - 1.98 (m, 1 H), 2.44 (d, J = 7.14 Hz, 2 H), 3.02 - 4.45 (m, 2 H), 6.53 - 6.79 (m, 3 H).

### Example 59

### 7-isobutyl-1,3-benzoxazole-2-carbonitrile;

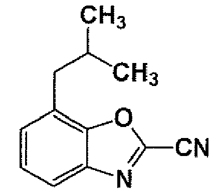

By the same procedures as described in Example 26 → Example 27, using the compound prepared in Example 58 instead of [2-(2,2-dimethylpropoxy)phenyl]amine in the step corresponding to Example 26, the title compound having the following physical data was obtained.
TLC : Rf 0.18 (dichloromethane : hexane =1:9);
¹H NMR (CDCl₃) : δ 0.95 (d, J = 6.59 Hz, 6 H), 1.99 - 2.16 (m, 1 H), 2.79 (d, J = 7.32 Hz, 2 H), 7.33 - 7.37 (m, 1 H), 7.43 (dd, J = 7.78 Hz, 1 H), 7.68 - 7.74 (m, 1 H).

### Example 60

### 2-(ethylthio)-5,7-dihydrokro[3,4-d]pyrimidin-4-ol:

To a solution of ethyl imidothiocarbamate hydrobromide (2,59 g) in water (25 mL) were added sodium carbonate (1.48 g) and methyl 4-oxotetrahydro-3-furancarboxylate (1.44 g), then the mixture was stirred for 3.5 hours at room temperature, 18 hours at 70°C, then 4 hours at 80°C. The reaction mixture was cooled to room temperature, the precipitate was collected, washed with water and diisopropyl ether sequentially to give the title compound (744 mg) having the following physical data. The former water layer was washed with ethyl acetate, extracted with dichloromethane. The organic layer was washed with brine, dried over anhydrous sodium sulfate, concentrated. The obtained residue was added by diisopropyl ether, the precipitated solid was collected to give the title compound (139 mg) having the following physical data.
TLC : Rf0.65 (ethyl acetate);
¹H NMR (CDCl₃): δ 1.38 (t, J = 7.36 Hz, 3 H), 3.20 (q, J = 7.36 Hz, 2 H), 4.80 - 4.97 (m, 2 H), 4.98 - 5.15 (m, 2 H), 11.62 - 12.25 (m, 1 H).

### Example 61

### 4-chloro-2-(ethylthio)-5,7-dihydrofuro[3,4-d]pyrimidine:

To a suspension of the compound prepared in Example 60 (50 mg) in thionyl chloride (148 µL) was added dimethylformamide (20 µL), then the mixture was stirred for 5 minutes at 45°C. The reaction mixture was cooled to room temperature, poured into iced water, extracted with ethyl acetate. The organic layer was washed with brine, dried over anhydrous sodium sulfate, concentrated to give the title compound (49 mg) having the following physical data.
TLC : Rf 0.68 (hexane : ethyl acetate = 4 : 1);
¹H NMR (CDCl₃) : δ 1.40 (t, J = 7.35 Hz, 3 H), 3.17 (q, J = 7.35 Hz, 2 H), 5.00 (m, 2 H), 5.10 (m, 2 H).

### Example 62

### 4-[(2,2-dimethylpropyl)amino]-5,7-dihydrokro[3,4-d]pyrimidine-2-carbonitrile:

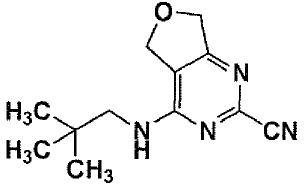

By the same procedures as described in Example 1 → Example 12 → Example 13, using the compound prepared in Example 61 instead of 2,4-dichloro-5-(chloromethyl)pyrimidine in the step corresponding to Example 1, the title compound having the following physical data was obtained.
TLC : Rf0.34 (hexane : ethyl acetate = 3 : 2);
¹H NMR (CDCl₃) : δ 0.97 (s, 9 H), 3.14 - 3.55 (m, 2H), 4.40 - 4.82 (m, 1 H), 4.96 (t, J = 2.74 Hz, 2 H), 5.00 - 5.15 (m, 2 H).

### Example 63

### 4-[(2,2-dimethylpropyl)amino]-6,7-dihydro-5H-cydopenta[d]pyrimidine-2-carbonitrile:

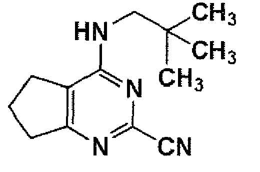

By the same procedures as described in Example 10 → Example 1 → Example 3, using 6,7-dihydro-1H-cyclopenta[d]pynmidin-2,4(3H,5H)-dione instead of the compound prepared in Example 9 in the step corresponding to Example 10, the title compound having the following physical data was obtained.
TLC : Rf0.36 (hexane : ethyl acetate =2:1);
¹H NMR (CDCl₃) : δ 0.96 (s, 9 H), 2.09 - 2.27 (m, 2 H), 2.71 (t, J = 7.50 Hz, 2 H), 2.94 (t, J = 7.50 Hz, 2 H), 3.38 (d, J = 6.40 Hz, 2 H), 4.51 - 4.74 (m, 1 H).

### Biological Examples

### Pharmacological activities of the compounds of the present invention:

It was confirmed by the following experiments that the compound of formula (I) of the present invention has an inhibitory activity against cystein protease.

### (i) Measurement of cathepsin K inhibitory activity

65 µL of cathepsin K enzyme reaction buffer (50 mmol/L of 2-(N-morpholino)ethanesulfonate, 2 mmol/L of ethylenediaminetetraacetate (EDTA) and 4 mmol/L of dithiothreitol (DTT) were mixed to adjust to pH 5.5), 5 µL of cysteine protease inhibitor solution of several concentrations, 20 µL of synthesized substrate (t-butyloxyearbonyl-L-alanyl-glycyl-L-prolyl-L-arginine-4-methylchro many l-7-amide) solution of several concentrations and 10 µL of cathepsin K enzyme solution were mixed and the increase of fluorescence intensity when reacted at 37°C was measured (λex (excitation wavelength) = 355 nm, λem (fluorescence wavelength) = 460 nm). As to the substrate and the compound of the present invention, enzyme reactions were carried out in combination of several appropriate concentrations and Dixon plotting was prepared to define the absolute value of X-coordinate of the intersection point of the graph as Ki value.

It was confirmed that the compound of formula (I) of the present invention showed strong inhibitory activity, i.e. the IC₅₀ value is under 10 µM. For example, the IC₅₀ value of the compound 8(2B) prepared in example 8 was 2.9 nM, that of the compound prepared in example 55(14) was 12 nM.

### Formulation Example 1

The following components were admixed in a conventional method and punched out to give 10,000 tablets each containing 10 mg of the active ingredient.
8-(2,2-Dimethylpropyl)-7-oxo-5,6,7,8-tetrahydropyrimido[4,5-d]pyrimidine-2-carbonitrile (100 g),
Carboxymethylcellulose calcium (disintegrating agent) (20.0 g),
Magnesium stearate (lubricant) (10.0 g),
Microcrystalline cellulose (870 g).

### Formulation Example 2

The following components were admixed in a conventional method, and the solution was filtered through a dustproofing filter, and then 5 ml aliquots were charged into ampoules, which were autoclaved to give 10,000 ampoules each containing 20 mg of the active ingredient.
8-(2,2-Dimethylpropyl)-7-oxo-5,6,7,8-tetrahydropyrimido[4,5-d]pyrimidine-2-carbonitrile (200 g),
Mannitol (2 kg),
Distilled water (50 L).

### INDUSTRIAL APPLICABILITY

A compound of formula (I) in the present invention has an inhibitory activity against cysteine proteases, especially cathepsin K, and therefore it is useful as an agent for the prophylaxis and/or treatment of inflammatory diseases (periodontitis, arthritis, inflammatory bowel diseases, infectious diseases, pancreatitis, hepatitis, glomerulonephritis, endocarditis, myocarditis, ulcerative colitis, *etc*.), diseases induced by apoptosis (graft versus host diseases, rejection in transplantation, acquired immunodeficiency syndrome (AIDS), AIDS-related complex (ARC), adult T cell leukemia, hairy cells leukemia, spondylopathy, disorders of respiratory apparatus, arthritis, HIV or HTLV-1 related diseases (uveitis *etc.*), virus related diseases (hepatitis C *etc.*), cancer, collagenosis (systemic lupus erythematosus, rheumatoid arthritis, *etc.*), ulcerative colitis, Sjoegren syndrome, primary biliary cirrhosis, spontaneous thrombocytopenic purpura, autoimmune hemolytic anemia, myasthenia gravis, autoimmune diseases (insulin dependent (type I) diabetes, *etc.),* diseases accompanied by thrombocytopenia (myelodysplastic syndrome, cyclic thrombocytopenia, aplastic anemia, spontaneous thrombocytopenia, disseminated intravascular coagulation (DIC), *etc.*), viral hepatitis (A, B, C, F, *etc.*) or hepatitis medicamentosa and cirrhosis, *etc.,* dementia such as Alzheimer's disease, Alzheimer-type senile dementia, cerebrovascular injury, neurodegenerative disease, adult acute respiratory distress syndrome, infectious diseases, prostatomegaly, hysteromyoma, bronchial asthma, arteriosclerosis, hyperlipidemia, all kinds of lusus naturae, nephritis , senile cataract, chronic fatigue syndrome, myodystrophy, peripheral nerve injury, *etc*.), diseases induced by immune response disorder (graft versus host diseases, rejection in transplantation, allergic diseases (asthmatic bronchitis, atopic dermatitis, allergic rhinitis, hay fever, diseases by house dust, hypersensitive pneumonia, food allergy, *etc.*), psoriasis, rheumatoid arthritis, *etc*.), autoimmune diseases (insulin dependent (type I) diabetes, systemic lupus erythematosus, Hashimoto's diseases, multiple sclerosis, *etc.*), diseases induced by decomposition of proteins which compose a body (myodystrophy, cataract, periodontitis, hepatocyte injury by bile acid (cholestatic cirrhosis *etc.*), *etc.,* decomposition of alveolus elastica (emphysema *etc.*), ischemic diseases (brain ischemia, brain disorder by ischemic reperfusion, cardiac infarction, ischemic liver damage, *etc*.), *etc.*), shock (septic shock, systemic inflammatory responsive syndrome, endotoxin shock, acidosis, *etc.*), circulatory disorder (arteriosclerosis, restenosis after PTCA (percutaneous transluminal coronary angioplasty), *etc*.), disorder of blood coagulation system (thrombocytopenic purpura, hemolytic uremic syndrome, *etc.*), malignant tumor, acquired immune deficiency syndrome (AIDS, AIDS-related complex (ARC), *etc.),* parasitic diseases (malaria *etc.*), neurodegenerative disease (Alzheimer-type senile dementia, Huntington's chorea, Parkinson's disease, multiple sclerosis, traumatic encephalopathy, traumatic spondylopathy, *etc.*), lung disorder (fibroid lungs, *etc.*), bone diseases (osteoporosis, bone fracture, rheumatoid arthritis, arthritis, osteoarthritis, hypercalcaemia, osteometastasis of cancer, periodontitis, bone Paget's disease, *etc.),* endocrinesthenia (hyperthyroidism *etc.*), *etc.*

## Claims

1. A compound of formula (I) 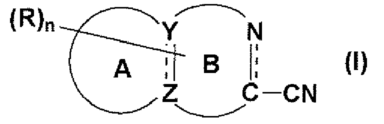 wherein ring A is a carbocyclic group or a heterocyclic group; ring B is a heterocyclic group containing at least one nitrogen atom; 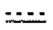 is a single bond or a double bond; Y and Z each is independently a carbon atom or a nitrogen atom; n is 0, or an integer of from 1 to 10; R is a hydrogen atom or a substituent, if R is plural, each of R are the same or different, in which two of R may form a ring which may have a substituent(s) together with an atom to which they bind,
a salt thereof, a solvate thereof, or an N-oxide thereof, or a prodrug thereof

2. The compound according to claim 1, wherein ring A is a C5-7 monocyclic carbocyclic group, or a five- to seven-membered monocyclic heterocyclic group; and ring B is a five- to seven-membered monocyclic heterocyclic group which contains one nitrogen atom and contains 1 to 2 hetero atom(s) selected from an oxygen atom(s), a nitrogen atom(s) and a sulfur atom(s) which may be oxidized.

3. The compound according to claim 1, wherein ring A is a ring selected from benzene, cyclopentene, cyclohexene, cycloheptene, pyridine, pyrimidine, pyrazine, tetrahydropyrimidine, dihydropyridazine, pyridazine, dihydropyrimidine, dihydropyrazine, dihydrotriazine, pyrazole, dihydropyrazole, pyrrole, imidazole, triazole, thiophene, furan, dihydrofuran, oxadiazine, tetrahydrodiazepine and diazepane.

4. The compound according to claim 1, wherein ring B is a ring selected from pyrrole, imidazole, pyrazole, thiazole, oxazole, pyridine, pyrimidine, dihydrotriazine, pyrazine and dihydropyrimidine.

5. The compound according to claim 1, wherein the compound of formula (I) is 5,6,7,8-tetrahydropyrimido[4,5-d]pyrimidine-2-carbonitrile, pyrazolo[1,5-a]pyrimidine-5-carbonitrile, 2,3-dihydro-1H-pyrazolo[3,4-d]pyrimidine-6-carbonitrile, pyrazolo[1,5-a][1,3,5]triazine-2-carbonitrile, 1H-pyrimido[4,5-e][1,3,4]oxadiazine-7-carbonitrile, 1H-pyrazolo[3,4-d]pyrimidine-6-carbonitrile, imidazo[1,2-a]pyrimidine-2-carbonitrile, 1,3-benzothiazol-2-carbonitrile, 6,7,8,9-tetrahydro-5H-pyrimido[4,5-e][1,4]diazepine-2-carbonitrile, 5H-pyrrolo[3,2-d]pyrimidine-2-carbonitrile, pyrido[2,3-d]pyrimidine-2-carbonitrile, 5,7-dihydrofuro[3,4-d]pyrimidine-2-carbonitrile, 6,7-dihydro-5H-cyclopenta[d]pyrimidine-2-carbonitrile, 9H-purine-2-carbonitrile, or 1,3-benzoxazole-2-carbonitrile.

6. The compound according to claim 1, which is a compound of formula (I-A) 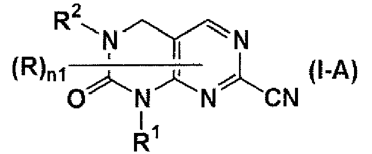 or formula(I-B) 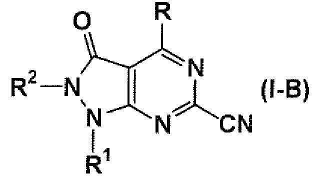 wherein R¹ is a hydrogen atom or a substituent; n1 is 0, or an integer of from 1 to 3; and R² is 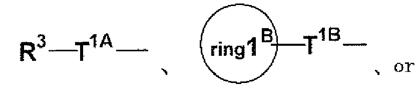 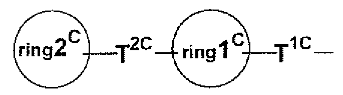 wherein T^{1A}, T^{1B}, T^{1C} and T^{2c} each is independently a bond or a spacer having from 1 to 10 atoms of the principle chain; ring 1^{B}, ring1^{C} and ring2^{C} each is independently a cyclic group which may have a substituent(s); and R³ is a hydrogen atom or a substituent,
and other symbols have the same meanings as described in claim 1.

7. The compound according to claim 6, wherein R¹ is a branched C1-8 alkyl.

8. The compound according to claim 6, wherein -T^{1A}- is -E³-E²-E¹- in which E¹ and E³ each is independently a bond or a divalent C1-5 hydrocarbon group which may have a substituent(s), and E² is -O-, -NR¹⁰-, -S-, -C(=O)-, -C(=O)NR¹⁰-, - NR¹⁰C(=O)-, -SO₂NR¹⁰-, -NR¹⁰SO₂-, -C(=O)O-, -OC(=O)-, or -NR¹⁰C(=O)NR¹¹- in which R¹⁰ and R¹¹ each is independently a hydrogen atom or a hydrocarbon group which may have a substituent(s); and R³ is a hydrogen atom or a substituent containing a nitrogen atom which is basic.

9. The compound according to claim 6, wherein -T^{1B}- is -E³-E²-E¹- in which all symbols have the same meanings as described in claim 8; and ring 1^{B} is (1) C3-10 mono- or bicyclic carbocyclic group which may have a substituent(s), or (2) three- to ten-membered monocyclic or bicyclic heterocyclic group which may have a substituent(s).

10. The compound according to claim 6, wherein -T^{1C}- is -E³-E²-E¹- in which all symbols have the same meanings as described in claim 8; -T^{2C}- is a bond or a divalent C1-5 hydrocarbon group which may have a substituent(s): and ring 1^{C} and/or ring 2^{C} is (1) C3-10 mono- or bicyclic carbocyclic group which may have a substituent(s), or (2) three- to ten-membered monocyclic or bicyclic heterocyclic group which may have a substituent(s).

11. The compound according to claim 1, which is selected from the group consisting of:
(1) 8-(2,2-dimethylpropyl)-7-oxo-5,6,7,8-tetrahydropyrimido[4,5-d]pyrimidine-2-carbonitrile,
(2) 1-(2,2-dimethylpropyl)-2-(4-methoxybenzyl)-3-oxo-2,3-dihydro-1H-pyrazolo[3,4-d]pyrimidine-6-carbonitrile,
(3) 1-(2,2-dimethylpropyl)-3-[(4-methoxybenzyl)oxy]-1H-pyrazolo[3,4-d]pyrimidine-6-carbonitrile,
(4) 4-(2,2-dimethylpropoxy)-1,3-benzothiazol-2-carbonitrile,
(5) 3-(4-biphenylylmethoxy)-1-(2,2-dimethylpropyl)-1H-pyrazolo[3,4-d]pyrimidine-6-carbonitrile,
(6) 2-(4-biphenylylmethyl)-1-(2,2-dimethylpropyl)-3-oxo-2,3-dihydro-1H-pyrazolo[3,4-d]pyrimidine-6-carbonitrile,
(7) 1-(2,2-dimethylpropyl)-3-oxo-2-(2-thienylmethyl)-2,3-dihydro-1H-pyrazolo[3,4-d]pyrimidine-6-carbonitrile,
(8) 1-(2,2-dimethylpropyl)-3-[2-(4-morpholinyl)ethoxy]-1H-pyrazolo[3,4-d]pyrimidine-6-carbonitrile,
(9) 9-(2,2-dimethylpropyl)-6-(4-methoxybenzyl)-7-oxo-6,7,8,9-tetrahydro-5H-pyrimido[4,5-e][1,4]diazepine-2-carbonitrile,
(10) 8-(2,2-dimethylpropyl)-6-(4-methoxybenzyl)-7-oxo-5,6,7,8-tetrahydropyrimido[4,5-d]pyrimidine-2-carbonitrile,
(11) 4-[(2,2-dimethylpropyl)amino]pyrido[2,3-d]pyrimidine-2-carbonitrile,
(12) 1-(2,2-dimethylpropyl)-3-{4-[(4-methyl-1-piperazinyl)methyl]phenyl}-1H-pyrimido[4,5-e][1,3,4]oxadiazine-7-carbonitrile,
(13) 2-[6-cyano-1-(2,2-dimethylpropyl)-3-oxo-1,3-dihydro-2H-pyrazolo[3,4-d]pyrimidin-2-yl]-N-[2-(dimethylamino)ethyl]acetamide,
(14) 4-[(2,2-dimethylpropyl)amino]-5,7-dihydrofuro[3,4-d]pyrimidine-2-carbonitrile, and
(15) 4-[(2,2-dimethylpropyl)amino]-6,7-dihydro-5H-cyclopenta[d]pyrimidine-2-carbonitrile.

12. A pharmaceutical composition comprising the compound of formula (I) which is described in claim 1, a salt thereof, a solvate thereof, or an N-oxide thereof, or a prodrug thereof as an active ingredient.

13. The pharmaceutical composition according to claim 12, which is an agent for prevention and/or treatment for a cysteine protease-related disease.

14. The pharmaceutical composition according to claim 13, wherein a cysteine protease-related disease is a cathepsin K-related disease.

15. The pharmaceutical composition according to claim 14, wherein a cathepsin K-related disease is a bone disease.

16. The pharmaceutical composition according to claim 15, wherein a bone disease is at least one selected from osteoporosis, bone fracture, arthritis, rheumatoid arthritis, osteoarthritis, hypercalcaemia, osteometastasis of cancer, osteosarcoma, periodontitis, and bone Paget's disease.

17. The pharmaceutical composition according to claim 12, which is a bone resorption inhibitor.

18. A drug comprising the compound of formula (I) described in claim 1, a salt thereof, a solvate thereof, or an N-oxide thereof, or a prodrug thereof, combined with at least one selected from bisphosphonate formulations, Vitamin D and its derivatives, Vitamin K and its derivatives, calcitonin formulations, α-calcitonin gene-related peptide formulations, female hormone formulations, selective estrogen receptor modulators, ipriflavone formulations, calcium formulations, anabolic steroid formulations, parathyroid hormone formulations, PTHrP derivatives, caspase-1 inhibitors, farnesoid X receptor agonists, Bone Morphogenetic Protein formulations, anti-RANKL antibody, metalloprotease inhibitors, prostaglandin derivatives, strontium formulations, anti TNF-α, antibody, anti-IL-6 antibody, HMG-CoA reductase inhibitors, steroidal drugs, and antiinflammatory drugs.

19. A method for the prophylaxis and/or treatment of a cysteine protease-related disease in a mammal, which comprises administering to a mammal an effective amount of the compound of formula (I) described in claim 1, a salt thereof, a solvate thereof, or an N-oxide thereof, or a prodrug thereof

20. Use of the compound of formula (I) described in claim 1, a salt thereof, a solvate thereof, or an N-oxide thereof, or a prodrug thereof, for the manufacture of a pharmaceutical preparation for the prophylaxis and/or treatment of a cysteine protease-related disease.
